# EUROPEAN PATENT SPECIFICATION

(11) **EP 4 267 550 B1**
(45) Date of publication and mention of the grant of the patent: **11.11.2026**
(21) Application number: 21840972.0
(22) Date of filing: 23.12.2021
(51) Int. Cl.: C07C 271/20, C07C 323/12, C07D 211/14, C07D 295/13, A61K 9/127, A61K 47/16, A61K 47/20, A61K 47/22, A61P 35/00

(54) **IONIZABLE LIPIDS**
IONISIERBARE LIPIDE
LIPIDES IONISABLES

(30) Priority: 23.12.2020 EP 20216879
(43) Date of publication of application: 01.11.2023
(73) Proprietor: eTheRNA Immunotherapies NV, 2845 Niel (BE); Universiteit Gent, 9000 Gent (BE)
(72) Inventor: DE KOKER, Stefaan, 9551 Ressegem (BE); DE GEEST, Bruno, 9845 De Pinte (BE); YONG, Chen, 9000 Gent (BE)
(74) Representative: Arnold & Siedsma
(86) International application number: PCT/EP2021/087492
(87) International publication number: WO 2022/136641

(56) References cited:
- EP-A1- 2 966 068
- WO-A1-2013/185116
- WO-A1-2019/141814

## Description

### FIELD OF THE INVENTION

The present invention generally relates to the field of ionizable (also termed cationic) lipids, and in particular provides a novel type of such lipids as represented by formula (I). The present invention further provides methods for making such lipids as well as uses thereof, in particular in the preparation of nanoparticle compositions, more in particular nanoparticle compositions comprising nucleic acids. It further provides vaccine formulations comprising nanoparticle compositions based on the ionizable lipids disclosed herein.

### BACKGROUND TO THE INVENTION

Nucleic acid-based drugs are being explored in a growing number of therapeutic areas. Nonetheless, due to their negative charge, size and instability, the targeted delivery of nucleic acids such as plasmid DNA, messenger RNA, short interfering RNA, single guide RNA and micro-RNAs to tissues and cells poses a major challenge. A plethora of nanoparticulate carrier systems has been explored to encapsulate and deliver nucleic acids. These nanoparticles need to combine efficient and stable encapsulation of the nucleic acid upon storage and in the extracellular environment, with maximum cellular uptake and efficient release of their payload from endosomes into the cytosol.

Lipid based nanoparticles are clinically used to deliver small interfering RNA and mRNA vaccines and represent the most advanced class of RNA delivery vehicles. Lipid based nanoparticles are typically composed of a cationic or ionizable lipid that can be protonated at acid pH, a helper phospholipid, a PEGylated lipid and a sterol. Each component has specialized functions in LNP stability and activity. The sterol and the PEGylated lipid are vital for LNP structure and stability, whereas the phospholipid can contribute to stability and endosomal escape. The cationic or ionizable lipid in turn is considered the main driver of activity and tolerability by governing mRNA encapsulation, cellular uptake and endosomal escape. Although effective nucleic acid delivery vehicles, LNPs can induce dose limiting toxicities, such as Complement Activation Related Pseudo-allergy, inflammatory cytokine release and cellular toxicities by accumulation of non-degradable ionizable lipids into cellular membranes. Further improvements in cationic or ionizable lipid chemistries are hence needed to improve efficacy and safety of LNP delivered nucleic acid drugs.

EP2966068 discloses ionizable lipids having a carbamate group and an S-link; however, the molecules disclosed herein differ from those of the present invention in that the carbamate group and the -S- group are linked in a different manner.

Accordingly, the present invention relates to a new class of ionizable lipids as defined by the present set of claims, which have improved characteristics over the currently available classes of ionizable lipids.

### SUMMARY OF THE INVENTION

In a first aspect, the present invention provides a lipid, in particular an ionizable lipid represented by formula (I) 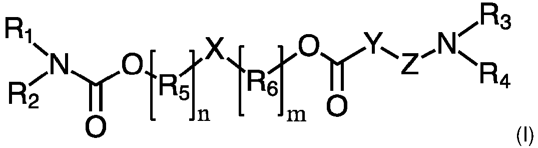 wherein
R₁ and R₂ are each independently selected from -H, -C₁₋₂₀alkyl, -C₂₋₂₀alkenyl, and -C₂₋₂₀alkynyl; wherein each of said -C₁₋₂₀alkyl, -C₂₋₂₀alkenyl, and -C₂₋₂₀alkynyl may optionally be substituted with from 1 to 3 -O-(C=O)-R₇, -(C=O)-O-R₇, -C₁₋₂₀alkyl, -C₂₋₂₀alkenyl, and -C₂₋₂₀alkynyl; and wherein the total number of C atoms in R₁ and R₂ together is at least 8;
R₃ and R₄ are each independently a -C₁₋₆alkyl; or R₃ and R₄ taken together with the N atom to which they are attached form a 5-10 membered aromatic or non-aromatic heterocycle; said heterocycle may further optionally comprise one or more additional N atoms, and/or may optionally be substituted with from 1-3 substituents selected from: -C₁₋₆alkyl; and
each R₅ and R₆ is independently-CH₂-;
each R₇ is independently selected from -C₁₋₂₀alkyl, -C₂₋₂₀alkenyl, -C₂₋₂₀alkynyl; wherein each of said -C₁₋₂₀alkyl, -C₂₋₂₀alkenyl, -C₂₋₂₀alkynyl may optionally be substituted with from 1 to 3 -O-(C=O)-R₇, -(C=O)-O-R₇, -C₁₋₂₀alkyl, -C₂₋₂₀alkenyl, -C₂₋₂₀alkynyl;
m and n are each independently an integer selected from 1, 2, 3 and 4;
X is selected from -O-, -S-, -S-S-, -O-(C=O)-, -O-(C=O)-O-, -(C=N-NH₂)-, -O-CR₈R₉-O-, and -S-CR₈R₉-S-;
each R₈ and R₉ is independently selected from -H, -C₁₋₆alkyl, and -C₃₋₆cycloalkyl;
Y is selected from -NH- and -O-; and
Z is -C₁₋₆alkylene-.

In a specific embodiment, the present invention provides a lipid, in particular an ionizable lipid as defined herein and being represented by formula (II) 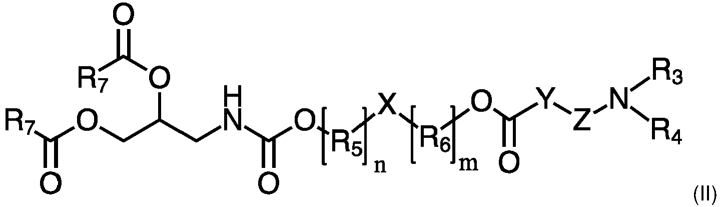 wherein
R₃ and R₄ are each independently a -C₁₋₆alkyl; or R₃ and R₄ taken together with the N atom to which they are attached form a 5-10 membered aromatic or non-aromatic heterocycle; said heterocycle may further optionally comprise one or more additional N atoms, and/or may optionally be substituted with from 1-3 substituents selected from: -C₁₋₆alkyl; and
each R₅ and R₆ is independently-CH₂-;
each R₇ is independently selected from -C₁₋₂₀alkyl, -C₂₋₂₀alkenyl, -C₂₋₂₀alkynyl; wherein each of said -C₁₋₂₀alkyl, -C₂₋₂₀alkenyl, -C₂₋₂₀alkynyl may optionally be substituted with from 1 to 3 -O-(C=O)-R₇, -(C=O)-O-R₇, -C₁₋₂₀alkyl, -C₂₋₂₀alkenyl, -C₂₋₂₀alkynyl; and the total number of C atoms in both R₇ moieties together is at least 5;
m and n are each independently an integer selected from 1, 2, 3 and 4;
X is selected from -O-, -S-, -S-S-, -O-(C=O)-, -O-(C=O)-O-, -(C=N-NH₂)-, -O-CR₈R₉-O-, and -S-CR₈R₉-S-;
each R₈ and R₉ is independently selected from -H, -C₁₋₆alkyl, and -C₃₋₆cycloalkyl;
Y is selected from -NH- and -O-; and
Z is -C₁₋₆alkylene-.

In yet a further embodiment, the present invention provides a lipid, in particular an ionizable lipid as defined herein and being represented by any one of formula (IIIa), (IIIb) or (IIIc) 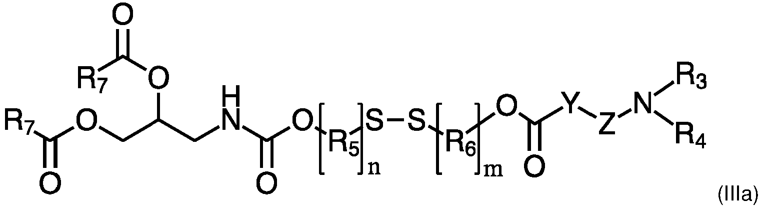 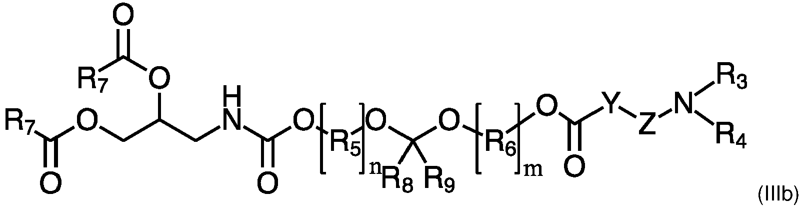 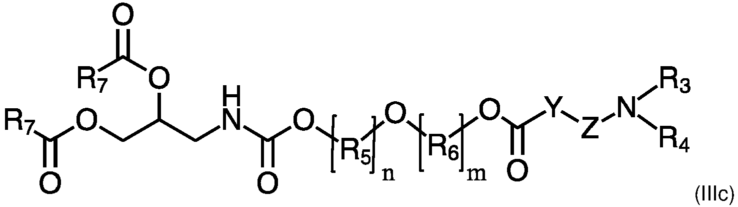 wherein
R₃ and R₄ are each independently a -C₁₋₆alkyl; or R₃ and R₄ taken together with the N atom to which they are attached form a 5-10 membered aromatic or non-aromatic heterocycle; said heterocycle may further optionally comprise one or more additional N atoms, and/or may optionally be substituted with from 1-3 substituents selected from: -C₁₋₆alkyl; and
each R₅ and R₆ is independently-CH₂-;
each R₇ is independently selected from -C₁₋₂₀alkyl, -C₂₋₂₀alkenyl, -C₂₋₂₀alkynyl; wherein each of said -C₁₋₂₀alkyl, -C₂₋₂₀alkenyl, -C₂₋₂₀alkynyl may optionally be substituted with from 1 to 3 -O-(C=O)-R₇, -(C=O)-O-R₇, -C₁₋₂₀alkyl, -C₂₋₂₀alkenyl, -C₂₋₂₀alkynyl; and the total number of C atoms in both R₇ moieties together is at least 5;
m and n are each independently an integer selected from 1, 2, 3 and 4;
each R₈ and R₉ is independently selected from -H, -C₁₋₆alkyl, and -C₃₋₆cycloalkyl;
Y is selected from -NH- and -O-; and
Z is -C₁₋₆alkylene-.

The present invention further provides a lipid, in particular an ionizable lipid as defined herein and being selected from the list comprising:

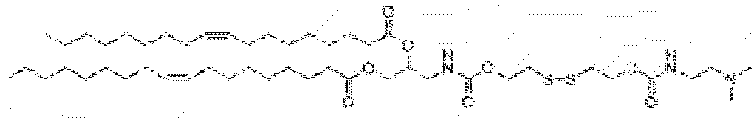

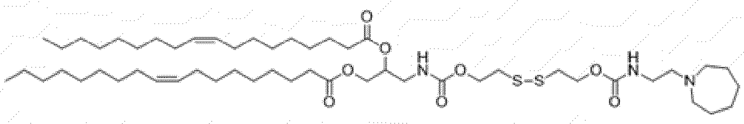

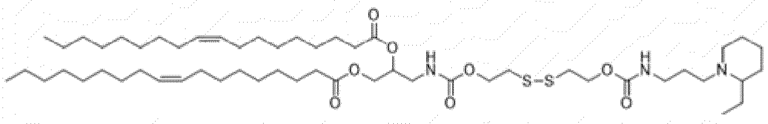

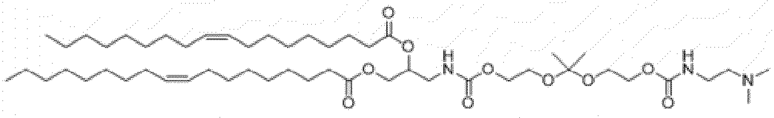

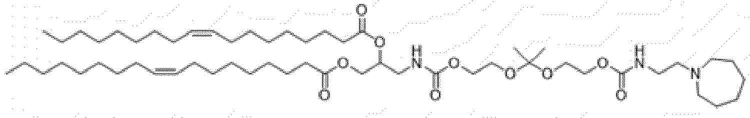

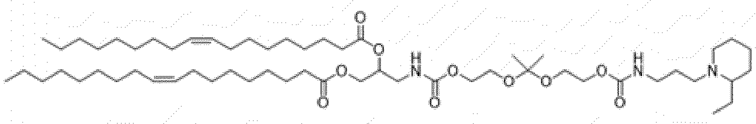

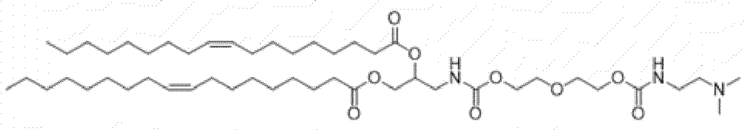

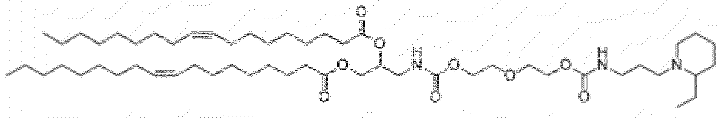

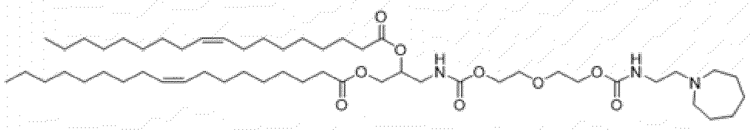

In yet a further embodiment, the present invention provides a lipid, in particular an ionizable lipid as defined herein and being represented by any one of formula (IVa), (IVb) and (IVc) 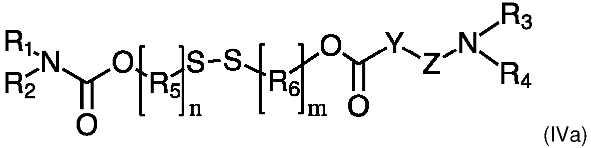 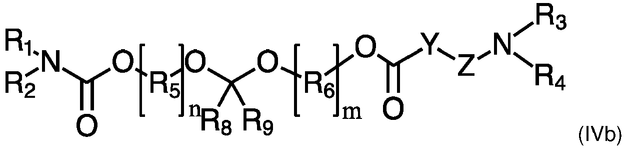 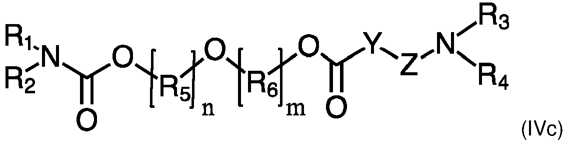 wherein
R₁ and R₂ are each independently selected from -H, -C₁₋₂₀alkyl, -C₂₋₂₀alkenyl, and -C₂₋₂₀alkynyl; wherein each of said -C₁₋₂₀alkyl, -C₂₋₂₀alkenyl, and -C₂₋₂₀alkynyl may optionally be substituted with from 1 to 3 -O-(C=O)-R₇, -(C=O)-O-R₇, -C₁₋₂₀alkyl, -C₂₋₂₀alkenyl, and -C₂₋₂₀alkynyl; and wherein the total number of C atoms in R₁ and R₂ together is at least 8;
R₃ and R₄ are each independently a -C₁₋₆alkyl; or R₃ and R₄ taken together with the N atom to which they are attached form a 5-10 membered aromatic or non-aromatic heterocycle; said heterocycle may further optionally comprise one or more additional N atoms, and/or may optionally be substituted with from 1-3 substituents selected from: -C₁₋₆alkyl; and
each R₅ and R₆ is independently selected from -CH₂-, and -O-CH₂-;
each R₇ is independently selected from -C₁₋₂₀alkyl, -C₂₋₂₀alkenyl, -C₂₋₂₀alkynyl; wherein each of said -C₁₋₂₀alkyl, -C₂₋₂₀alkenyl, -C₂₋₂₀alkynyl may optionally be substituted with from 1 to 3 - O-(C=O)-R₇, -(C=O)-O-R₇, -C₁₋₂₀alkyl, -C₂₋₂₀alkenyl, -C₂₋₂₀alkynyl;
m and n are each independently an integer selected from 1, 2, 3 and 4;
each R₈ and R₉ is independently selected from -H, -C₁₋₆alkyl, and -C₃₋₆cycloalkyl;
Y is selected from -NH- and -O-; and
Z is -C₁₋₆alkylene-.

In a further embodiment, the present invention provides a lipid, in particular an ionizable lipid as defined herein and being selected from the list comprising:

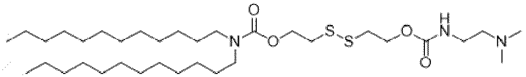

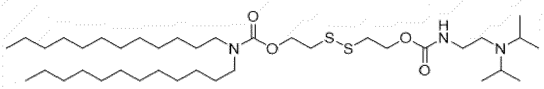

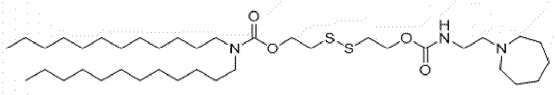

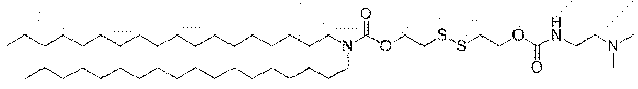

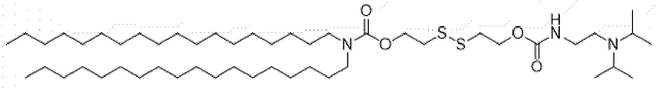

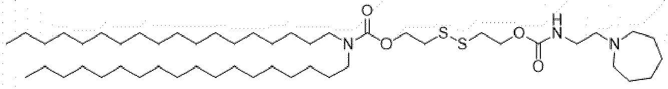

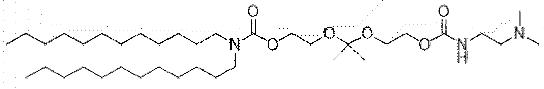

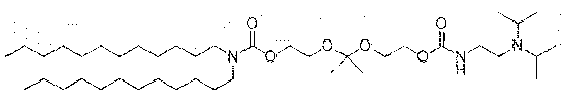

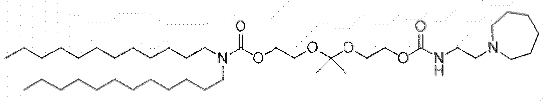

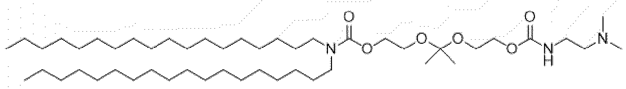

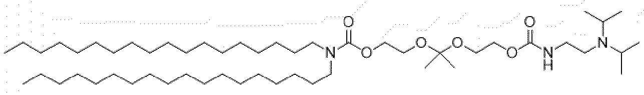

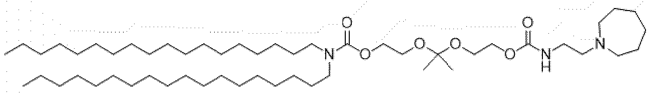

In yet a further embodiment, the present invention provides a lipid, in particular an ionizable lipid as defined herein, wherein the total number of C atoms in R₁ and R₂ together is at least 14.

The present invention further provides a lipid, in particular an ionizable lipid as defined herein; wherein each R₅ and R₆ is -CH₂-.

In a further embodiment, the present invention provides a lipid, in particular an ionizable lipid as defined herein; wherein m and n are the same, being an integer selected from 1, 2, 3 and 4,preferably 2.

In yet a further embodiment, the present invention provides a lipid, in particular an ionizable lipid as defined herein, wherein Y is -NH-.

In a further aspect, the present invention provides a lipid nanoparticle or lipid nanoparticle composition comprising a lipid, in particular an ionizable lipid as defined herein. Said nanoparticle composition may further comprise a phospholipid, a sterol and a PEG lipid.

In yet a further embodiment of the present invention, the lipid nanoparticle or lipid nanoparticle composition as defined herein further comprises an active agent, in particular a nucleic acid, preferably mRNA.

In a further aspect, the present invention provides the use of a lipid, in particular an ionizable lipid as defined herein in the manufacture of a lipid nanoparticle or lipid nanoparticle composition.

In a final aspect, the present invention provides a pharmaceutical composition comprising a lipid nanoparticle or lipid nanoparticle composition as defined herein and a pharmaceutically acceptable agent.

The invention also provides the pharmaceutical compositions as defined herein for use in human and/or veterinary medicine.

In yet a further embodiment, the present invention provides a lipid, in particular an ionizable lipid as defined herein and being represented by formula (V) 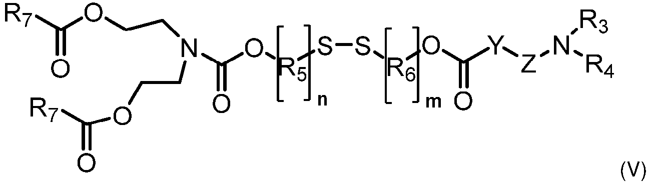 wherein
R₃ and R₄ are each independently a -C₁₋₆alkyl; or R₃ and R₄ taken together with the N atom to which they are attached form a 5-10 membered aromatic or non-aromatic heterocycle; said heterocycle may further optionally comprise one or more additional N atoms, and/or may optionally be substituted with from 1-3 substituents selected from: -C₁₋₆alkyl; and
each R₅ and R₆ is independently selected from -CH₂-, and -O-CH₂-;
each R₇ is independently selected from -C₁₋₂₀alkyl, -C₂₋₂₀alkenyl, -C₂₋₂₀alkynyl; wherein each of said -C₁₋₂₀alkyl, -C₂₋₂₀alkenyl, -C₂₋₂₀alkynyl may optionally be substituted with from 1 to 3 -O-(C=O)-R₇, -C₁₋₂₀alkyl, -C₂₋₂₀alkenyl, -C₂₋₂₀alkynyl; and the total number of C atoms in both R₇ moieties together is at least 5;
m and n are each independently an integer selected from 1, 2, 3 and 4;
Y is selected from -NH- and -O-; and
Z is -C₁₋₆alkylene-.

The present invention further provides a lipid, in particular an ionizable lipid as defined herein and being selected from the list comprising:

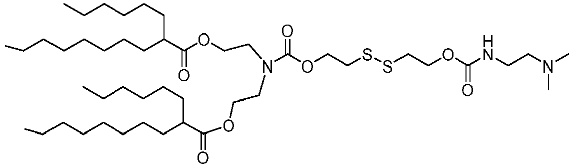

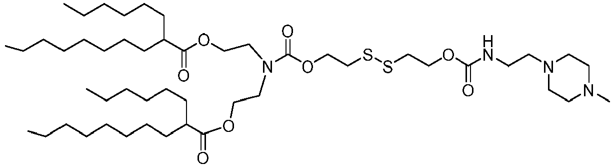

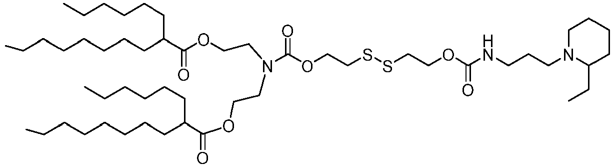

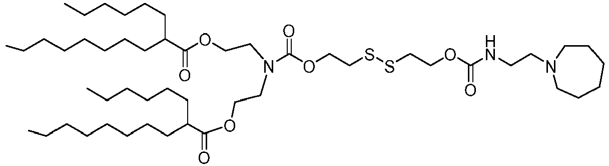

In yet a further embodiment, the present invention provides a lipid, in particular an ionizable lipid as defined herein and being represented by any one of formula (Vla) or (VIb) 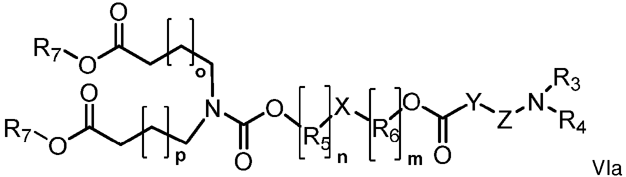 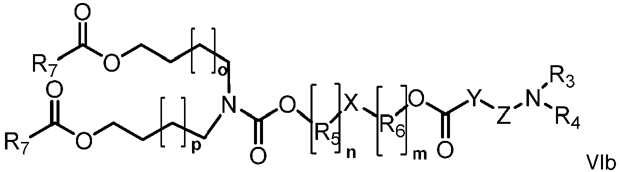 wherein
R₃ and R₄ are each independently a -C₁₋₆alkyl; or R₃ and R₄ taken together with the N atom to which they are attached form a 5-10 membered aromatic or non-aromatic heterocycle; said heterocycle may further optionally comprise one or more additional N atoms, and/or may optionally be substituted with from 1-3 substituents selected from: -C₁₋₆alkyl; and
each R₅ and R₆ is independently selected from -CH₂-, and -O-CH₂-;
each R₇ is independently selected from -C₁₋₂₀alkyl, -C₂₋₂₀alkenyl, -C₂₋₂₀alkynyl; wherein each of said -C₁₋₂₀alkyl, -C₂₋₂₀alkenyl, -C₂₋₂₀alkynyl may optionally further comprise one or more heteroatoms and/or may optionally be substituted with from 1 to 3 -O-(C=O)-R₇, -C₁₋₂₀alkyl, -C₂₋₂₀alkenyl, -C₂₋₂₀alkynyl; and the total number of C atoms in both R₇ moieties together is at least 5;
m and n are each independently an integer selected from 1, 2, 3 and 4;
o and p are each independently an integer selected from 1-10;
Y is selected from -NH- and -O-; and
Z is -C₁₋₆alkylene-.

In still a further embodiment, the present invention provides an ionizable lipid selected from the list comprising:

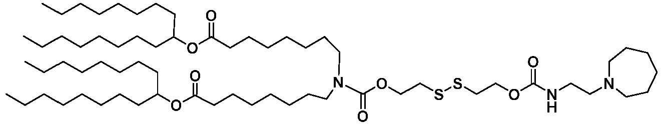

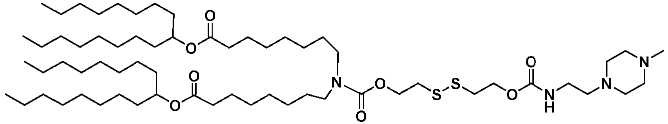

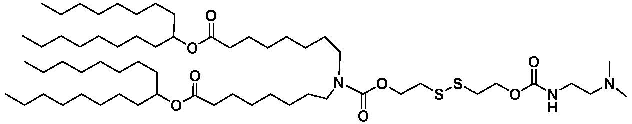

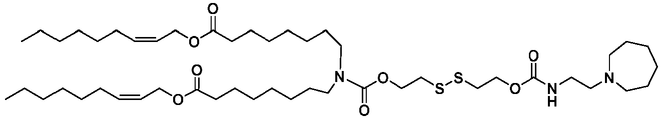

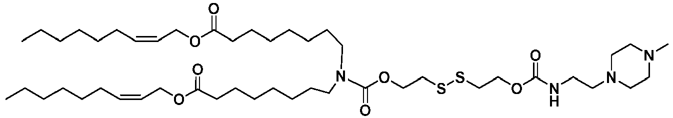

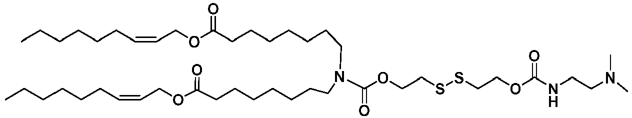

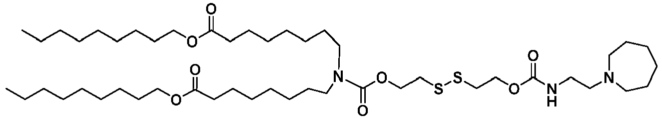

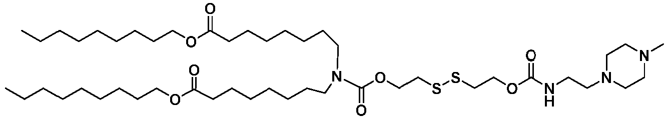

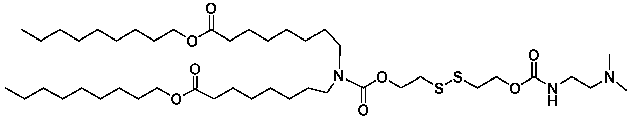

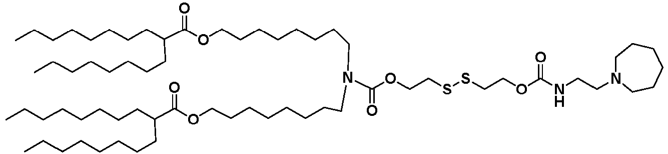

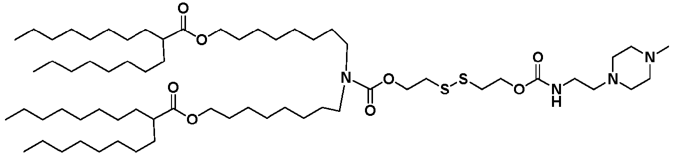

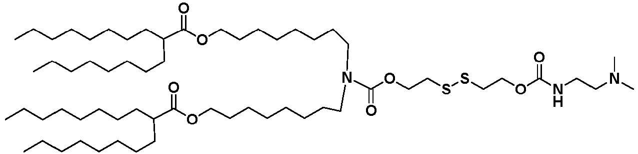

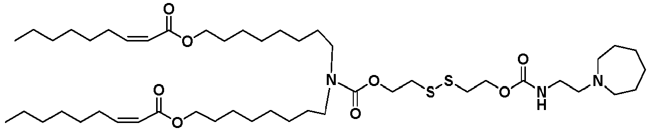

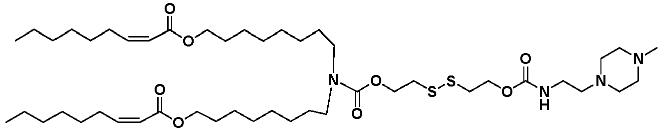

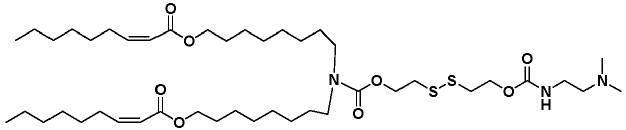

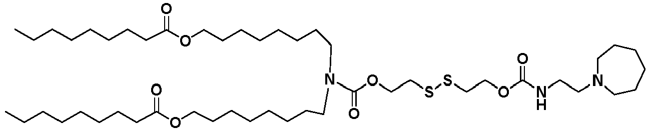

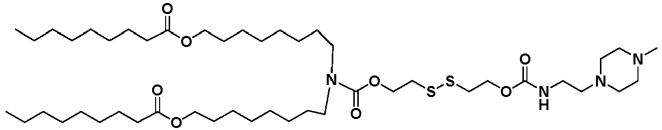

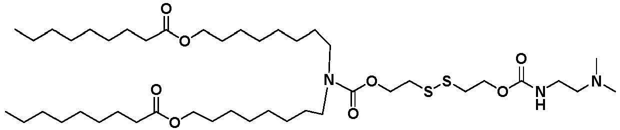

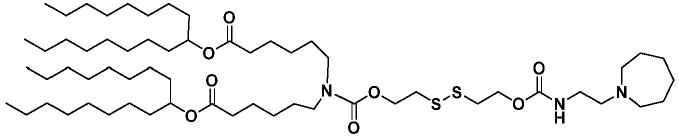

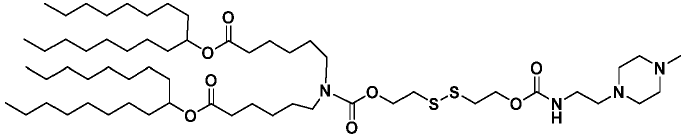

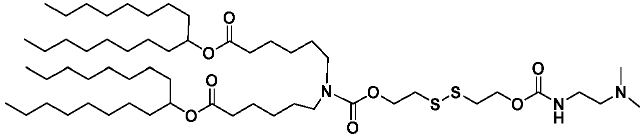

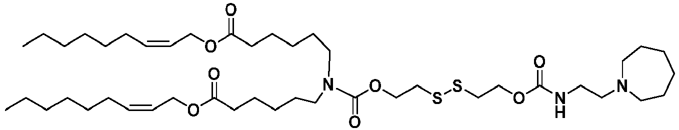

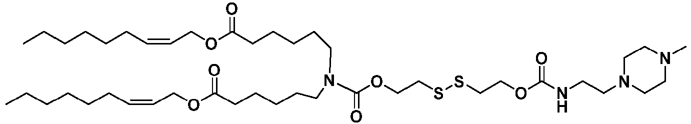

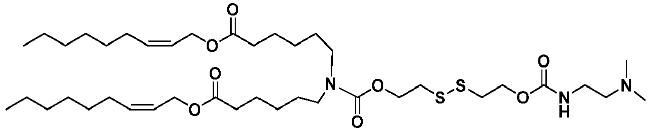

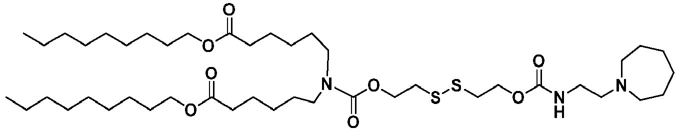

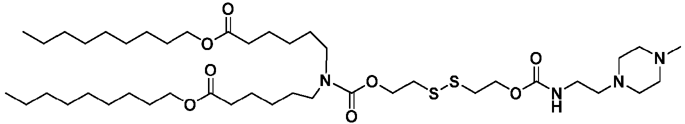

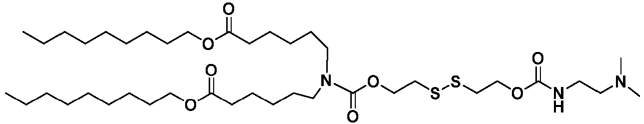

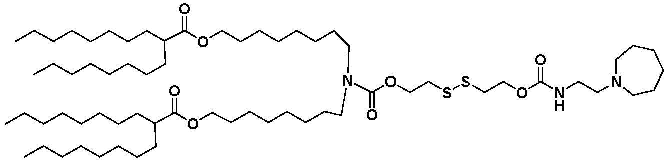

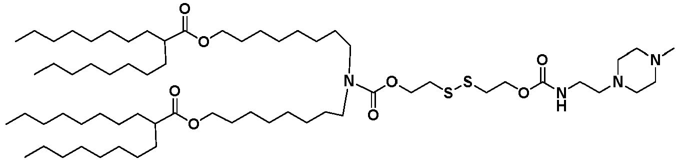

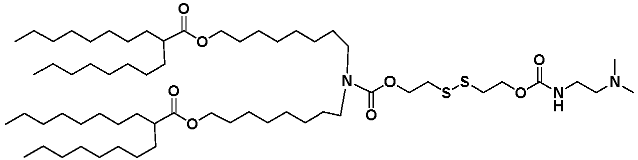

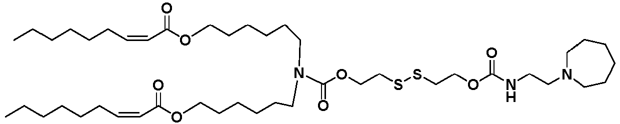

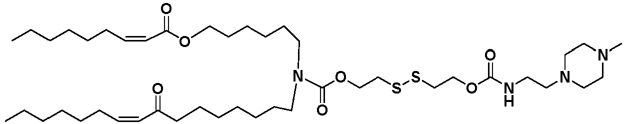

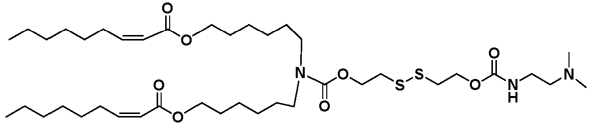

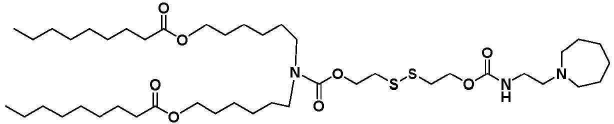

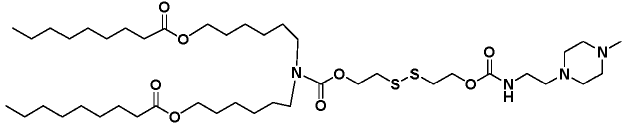

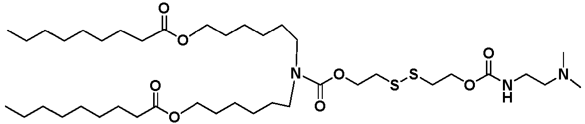

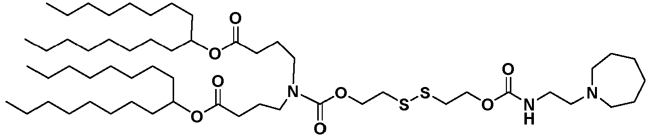

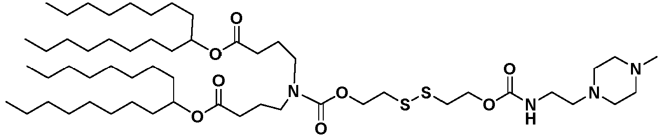

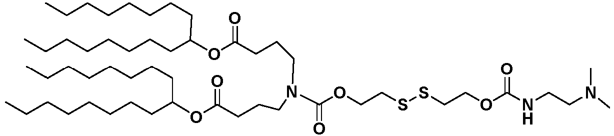

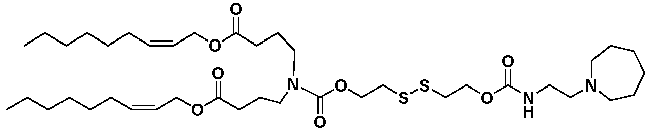

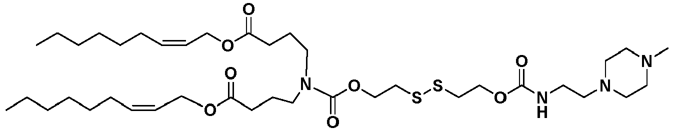

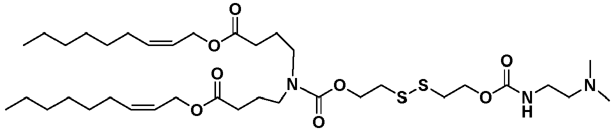

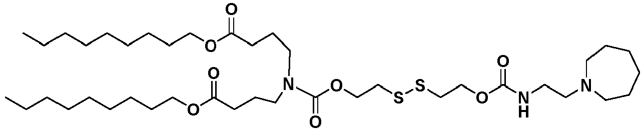

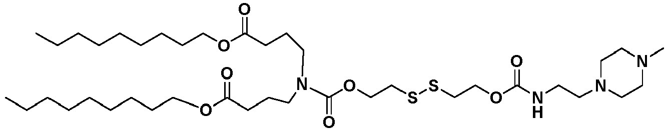

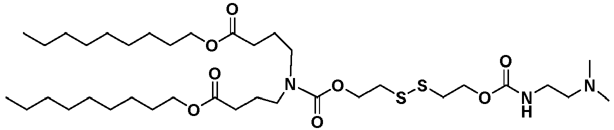

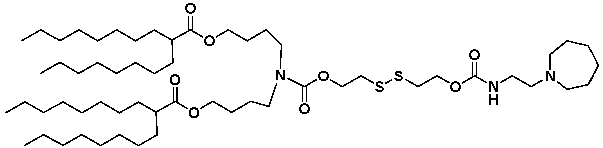

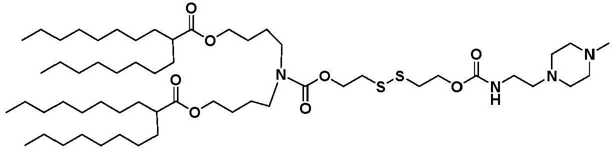

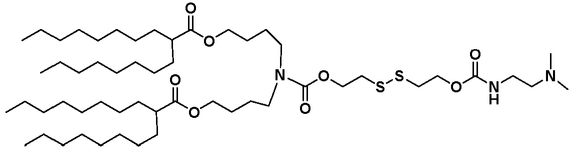

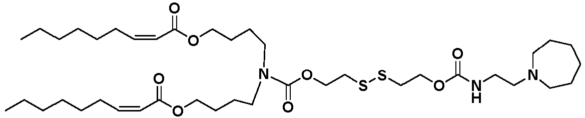

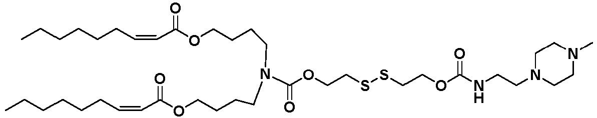

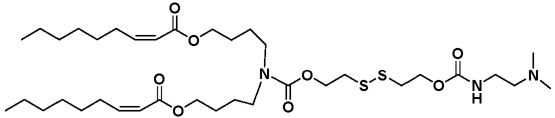

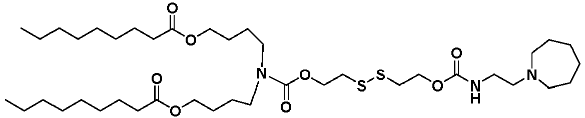

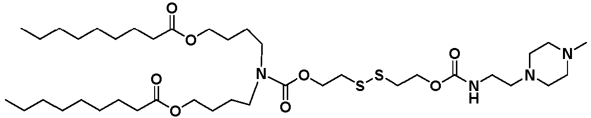

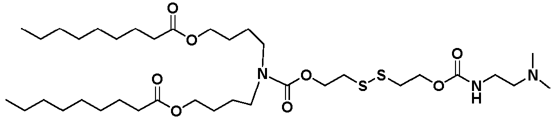

### BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.
**Fig. 1****:** Relative Mean Fluorescence Intensity (measured as the fold-increase in eGFP MFI compared to untreated cells) of eGFP expression in HEK293T cells upon incubation with the indicated LNPs at mRNA conc. of 50 ng and 200 ng/well.
**Fig. 2****.** Relative MFI of eGFP expression upon transfection of different cell types with the indicated LNPs, i.e. HEK293T cells (A), TS/A cells (B), CT26 cells (C) and B16F10 cells (D).
**Fig. 3****.** Viability of different cell types after transfection with the indicated LNPs, i.e. HEK293T (A) and CT26 (B).
**Fig. 4****.** Relative MFI of eGFP expression upon transfection of different cell types with the indicated LNPs, i.e. HEK293T (A) and CT26 (B).
**Fig. 5****.** Fluc mRNA expression in CT26 tumors (A) or liver (B) after injection in tumors, as measured by in vivo bioluminescence (photons/s/cm2/sr), and body weight (C) of mice prior and after intratumoral administration of S-Ac-DOg and MC-3 based LNPs
**Fig. 6****.** Fluc mRNA expression in B16F10 tumors (A) or liver (B) after injection in tumors, as measured by in vivo bioluminescence (photons/s/cm2/sr), and tumor/liver ratio (C) of Fluc expression after intratumoral injection of B16F10 tumors with the respective LNPs.
**Fig. 7****.** Flow cytometric assessment of the percentages of E7-specific CD8 T cells after intramuscular immunization with E7 mRNA encapsulated in LNPs with the respective ionizable lipids.
**Fig. 8****.** Percentage of E7-specific CD8 T cells measured in blood by flow cytometry after intramuscular immunization of C57BL/6 mice with mRNA LNPs comprising the indicated ionizable lipids. All LNPs were formulated at a lipid molar ratio ionizable lipid/DSPC/DMG-PEG2000 of 50/10/38.5/1.5. Mice received 2 immunizations with 5 µg mRNA at days 1 and 7.
**Fig. 9****.** Muscle thickness at the injection site measured prior to injection (d0), 1 day (d1) and 4 days (d4) after injection with the respective LNPs (5 µg E7 dose). All LNPs were formulated at a lipid molar ratio ionizable lipid/DSPC/DMG-PEG2000 of 50/10/38.5/1.5.
**Fig. 10****.** Anti-HA IgG1 and IgG2a antibody titers upon intramuscular immunization with the LNPs comprising the indicated ionizable lipids. Mice received 2 intramuscular immunizations with mRNA LNPs (2 µg HA) at days 1 and 21. Blood samples were obtained at days 21 and 35 for assessment of anti-HA antibody titers. LNPs containing the indicated ionizable lipids were formulated at a lipid molar ratio ionizable lipid/DSPC/DMG-PEG2000 of 50/10/38.5/1.5.
**Fig. 11****.** Percentages of splenic IFNg positive CD8 T cells upon intramuscular immunization with LNPs comprising the indicated ionizable lipids. Mice received 2 intramuscular immunizations with mRNA LNPs (2 µg HA) at days 1 and 21. Splenocytes were obtained at day 35 and either restimulated with a pool of overlapping HA peptides or left unstimulated. The percentage of IFNg+ CD8 T cells was subsequently determined by flow cytometry.
**Fig.12****.** Magnitude of the E7-specific CD8 T cell response as measured in blood upon intramuscular vaccination with LNPs containing S-Ac7-DOg, S-Ac7-DHDa or MC-3 as ionizable lipid. Mice received two immunizations at days 1 and 21 with 5 µg E7 mRNA. Blood samples were analyzed at days 7 and 27 by flow cytometry.
**Fig. 13****.** Flow cytometric assessment of the percentages of IFNg+, IFNg+ TNFa+, IFNg+ Granzyme b (Grnz)+ and IFNg+ CD107+ CD8 T cells in spleen upon in vitro stimulation with the E7-derived peptide RAHYNIVT. Mice received 2 immunization at days 1 and 21 with 5 µg E7 mRNA.
**Fig. 14****.** Percentage of Cy5-positive macrophages, dendritic cells (cDC1 and cDC2 subsets), B cells and T cells (CD4+ and CD8+ T cells subsets) in spleen, measured by flow cytometry 24 h post intravenous administration in C57BL/6 mice. Mice received a dose of 10 ug of peptide and 10 ug of IMDQ or equivalent. n=3.
**Fig. 15****.** Percentage of activation-marker positive dendritic cells (cDC1 and cDC2 subsets), B cells and T cells (CD4+ and CD8+ T cells subsets) in spleen, measured by flow cytometry 24 h post intravenous administration in C57BL/6 mice. Mice received a dose of 10 ug of peptide and 10 ug of IMDQ or equivalent. n=3.
**Fig. 16****.** Percentage of tetramer positive CD8+ T cells in the blood, measured by flow cytometry 1 week post intravenous administration in C57BL/6 mice of the second of two doses (2 week interval between dosing). Mice received a dose of 10 ug of peptide and 10 ug of IMDQ or equivalent. n=5.
**Fig. 17****.** Anti-S1 Spike protein IgG antibody titers upon intramuscular immunization with the LNPs comprising S-Ac7-DOg as the ionizable lipid. C57BL/6 mice received a single injection of LNP containing 25 ug of the TLR3 agonist polyl:C. 25 ug S1 Spike protein was either admixed or conjugated to the LNP surface through His₆-Ni2+ interaction. Blood samples were obtained 7 days post immunization and analyzed by ELISA.
**Fig.18****.** Anti-ovalbumin (OVA) IgG antibody titers upon intramuscular immunization with the LNPs comprising S-Ac7-DOg as the ionizable lipid. C57BL/6 mice received a single injection of LNP containing 10 ug of the TLR9 agonist CpG. 50 ug OVA was admixed to the LNP of Blood samples were obtained 7 days post immunization and analyzed by ELISA.

### DETAILED DESCRIPTION OF THE INVENTION

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Unless a context dictates otherwise, asterisks are used herein to indicate the point at which a mono- or bi-valent radical depicted is connected to the structure to which it relates and of which the radical forms part.

As already mentioned hereinbefore, in a first aspect, the present application provides a lipid, in particular an ionizable lipid represented by formula (I) 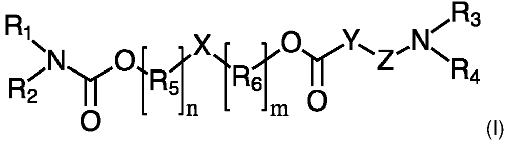 wherein
R₁ and R₂ are each independently selected from -H, -C₁₋₂₀alkyl, -C₂₋₂₀alkenyl, and -C₂₋₂₀alkynyl; wherein each of said -C₁₋₂₀alkyl, -C₂₋₂₀alkenyl, and -C₂₋₂₀alkynyl may optionally be substituted with from 1 to 3 - O-(C=O)-R₇, -C₁₋₂₀alkyl, -C₂₋₂₀alkenyl, and -C₂₋₂₀alkynyl; and wherein the total number of C atoms in R₁ and R₂ together is at least 8;
R₃ and R₄ are each independently a -C₁₋₆alkyl; or R₃ and R₄ taken together with the N atom to which they are attached form a 5-10 membered aromatic or non-aromatic heterocycle; said heterocycle may further optionally comprise one or more additional N atoms, and/or may optionally be substituted with from 1-3 substituents selected from: -C₁₋₆alkyl; and
each R₅ and R₆ is independently -CH₂-;
each R₇ is independently selected from -C₁₋₂₀alkyl, -C₂₋₂₀alkenyl, -C₂₋₂₀alkynyl; wherein each of said -C₁₋₂₀alkyl, -C₂₋₂₀alkenyl, -C₂₋₂₀alkynyl may optionally be substituted with from 1 to 3 -O-(C=O)-R₇, -C₁₋₂₀alkyl, -C₂₋₂₀alkenyl, -C₂₋₂₀alkynyl;
m and n are each independently an integer selected from 1, 2, 3 and 4;
X is selected from -O-, -S-, -S-S-, -O-(C=O)-, -O-(C=O)-O-, -(C=N-NH₂)-, -O-CR₈R₉-O-, and -S-CR₈R₉-S-;
each R₈ and R₉ is independently selected from -H, -C₁₋₆alkyl, and -C₃₋₆cycloalkyl;
Y is selected from -NH- and -O-; and
Z is -C₁₋₆alkylene-.

Accordingly, the present application also provides a lipid, in particular an ionizable lipid represented by formula (I) 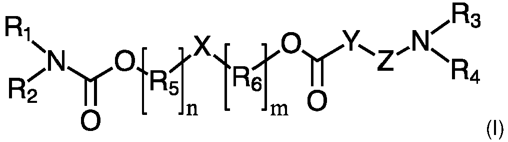 wherein
R₁ and R₂ are each independently selected from -H, -C₁₋₂₀alkyl, -C₂₋₂₀alkenyl, and -C₂₋₂₀alkynyl; wherein each of said -C₁₋₂₀alkyl, -C₂₋₂₀alkenyl, and -C₂₋₂₀alkynyl may optionally be substituted with from 1 to 3 -O-(C=O)-R₇, -(C=O)-O-R₇, -C₁₋₂₀alkyl, -C₂₋₂₀alkenyl, and -C₂₋₂₀alkynyl; and wherein the total number of C atoms in R₁ and R₂ together is at least 8;
R₃ and R₄ are each independently a -C₁₋₆alkyl; or R₃ and R₄ taken together with the N atom to which they are attached form a 5-10 membered aromatic or non-aromatic heterocycle; said heterocycle may further optionally comprise one or more additional N atoms, and/or may optionally be substituted with from 1-3 substituents selected from: -C₁₋₆alkyl; and
each R₅ and R₆ is independently-CH₂-;
each R₇ is independently selected from -C₁₋₂₀alkyl, -C₂₋₂₀alkenyl, -C₂₋₂₀alkynyl; wherein each of said -C₁₋₂₀alkyl, -C₂₋₂₀alkenyl, -C₂₋₂₀alkynyl may optionally be substituted with from 1 to 3 - O-(C=O)-R₇, -(C=O)-O-R₇, -C₁₋₂₀alkyl, -C₂₋₂₀alkenyl, -C₂₋₂₀alkynyl;
m and n are each independently an integer selected from 1, 2, 3 and 4;
X is selected from -O-, -S-, -S-S-, -O-(C=O)-, -O-(C=O)-O-, -(C=N-NH₂)-, -O-CR₈R₉-O-, and -S-CR₈R₉-S-;
each R₈ and R₉ is independently selected from -H, -C₁₋₆alkyl, and -C₃₋₆cycloalkyl;
Y is selected from -NH- and -O-; and
Z is -C₁₋₆alkylene-.

When describing the compounds/lipids of the present invention, the terms used are to be construed in accordance with the following definitions, unless a context dictates otherwise:
The term "alkyl" by itself or as part of another substituent refers to a fully saturated hydrocarbon of Formula CₓH₂ₓ₊₁ wherein x is a number greater than or equal to 1. Generally, alkyl groups of this invention comprise from 1 to 20 carbon atoms. Alkyl groups may be linear or branched and may be substituted as indicated herein. When a subscript is used herein following a carbon atom, the subscript refers to the number of carbon atoms that the named group may contain. Thus, for example, C₁₋₄alkyl means an alkyl of one to four carbon atoms. Examples of alkyl groups are methyl, ethyl, n-propyl, i-propyl, butyl, and its isomers (e.g. n-butyl, i-butyl and t-butyl); pentyl and its isomers, hexyl and its isomers, heptyl and its isomers, octyl and its isomers, nonyl and its isomers; decyl and its isomers, undecyl and its isomers, dodecyl and its isomers, tridecyl and its isomers, tetradecyl and its isomers, pentadecyl and its isomers, hexadecyl and its isomers, heptadecyl and its isomers, octadecyl and its isomers, nonadecyl and its isomers, eicosanyl and its isomers.

The term "optionally substituted alkyl" refers to an alkyl group optionally substituted with one or more substituents (for example 1 to 4 substituents, for example 1, 2, 3, or 4 substituents) at any available point of attachment. Non-limiting examples of such substituents include esters, carboxylic acids, alkyl moieties, alkene moieties, alkyne moieties, and the like.

In the context of the present invention, the alkyl, alkene and alkyne moieties as defined herein may also further comprise one or more heteroatoms, in that for example a C atom in an alkyl, alkene or alkyne chain is replaced by a heteroatom, such as selected from N, S or O.

The term "alkenyl" or "alkene", as used herein, unless otherwise indicated, means straight-chain, cyclic, or branched-chain hydrocarbon radicals containing at least one carbon-carbon double bond. Examples of alkenyl radicals include ethenyl, propenyl, isopropenyl, butenyl, isobutenyl, pentenyl, hexenyl, hexadienyl, be it in the terminal or internal positions and the like. Generally, alkenyl or alkene moieties of the present invention comprise from 2 to 20 C atoms. An optionally substituted alkenyl refers to an alkenyl having optionally one or more substituents (for example 1, 2, 3 or 4), selected from those defined above for substituted alkyl.

The term "alkynyl" or "alkyne", as used herein, unless otherwise indicated, means straight-chain or branched-chain hydrocarbon radicals containing at least one carbon-carbon triple bond. Examples of alkynyl radicals include ethynyl, E- and Z-propynyl, isopropynyl, E- and Z-butynyl, E- and Z-isobutynyl, E- and Z-pentynyl, E, Z-hexynyl, and the like. Generally alkenyl or alkene moieties of the present invention comprise from 2 to 20 C atoms. An optionally substituted alkynyl refers to an alkynyl having optionally one or more substituents (for example 1, 2, 3 or 4), selected from those defined above for substituted alkyl.

The term "cycloalkyl" by itself or as part of another substituent is a cyclic alkyl group, that is to say, a monovalent, saturated, or unsaturated hydrocarbyl group having 1, 2, or 3 cyclic structure. Cycloalkyl includes all saturated or partially saturated (containing 1 or 2 double bonds) hydrocarbon groups containing 1 to 3 rings, including monocyclic, bicyclic, or polycyclic alkyl groups. Cycloalkyl groups may comprise 3 or more carbon atoms in the ring and generally, according to this invention comprise from 3 to 15 atoms. Examples of cycloalkyl groups include but are not limited to cyclopropyl, cyclobutyl, cyclopentyl, cyclohexyl, cycloheptyl, cyclooctyl, cyclononyl, adamantanyl and cyclodecyl with cyclopropyl being particularly preferred. An "optionally substituted cycloalkyl" refers to a cycloalkyl having optionally one or more substituents (for example 1 to 3 substituents, for example 1, 2, 3 or 4 substituents), selected from those defined above for substituted alkyl.

Where alkyl groups as defined are divalent, i.e., with two single bonds for attachment to two other groups, they are termed "alkylene" groups. Non-limiting examples of alkylene groups includes methylene, ethylene, methylmethylene, trimethylene, propylene, tetramethylene, ethylethylene, 1,2-dimethylethylene, pentamethylene and hexamethylene. Similarly, where alkenyl groups as defined above and alkynyl groups as defined above, respectively, are divalent radicals having single bonds for attachment to two other groups, they are termed "alkenylene" and "alkynylene" respectively.

The term "heterocycle" as used herein by itself or as part of another group refers to non-aromatic, fully saturated or partially unsaturated cyclic groups (for example, 3 to 13 member monocyclic, 7 to 17 member bicyclic, or 10 to 20 member tricyclic ring systems, or containing a total of 3 to 10 ring atoms) which have at least one heteroatom in at least one carbon atom-containing ring. Each ring of the heterocyclic group containing a heteroatom may have 1, 2, 3 or 4 heteroatoms selected from nitrogen atoms, oxygen atoms and/or sulfur atoms, where the nitrogen and sulfur heteroatoms may optionally be oxidized and the nitrogen heteroatoms may optionally be quaternized. The heterocyclic group may be attached at any heteroatom or carbon atom of the ring or ring system, where valence allows. The rings of multi-ring heterocycles may be fused, bridged and/or joined through one or more spiro atoms. An optionally substituted heterocyclic refers to a heterocyclic having optionally one or more substituents (for example 1 to 4 substituents, or for example 1, 2, 3 or 4), selected from those defined above for substituted alkyl. Non-limiting examples of heterocycle comprise: piperidinyl, azepanyl, morpholinyl,... The term "aryl" (herein also referred to as aromatic heterocycle) as used herein refers to a polyunsaturated, aromatic hydrocarbyl group having a single ring (i.e. phenyl) or multiple aromatic rings fused together (e.g. naphthalene or anthracene) or linked covalently, typically containing 6 to 10 atoms; wherein at least one ring is aromatic. The aromatic ring may optionally include one to three additional rings (either cycloalkyl, heterocyclyl, or heteroaryl) fused thereto. Aryl is also intended to include the partially hydrogenated derivatives of the carbocyclic systems enumerated herein. Non-limiting examples of aryl comprise phenyl, ....

The aryl ring or heterocycle as defined herein can optionally be substituted by one or more substituents (for example 1 to 5 substituents, for example 1, 2, 3 or 4) at any available point of attachment. Non-limiting examples of such substituents are selected from halogen, hydroxyl, oxo, nitro, amino, hydrazine, aminocarbonyl, azido, cyano, alkyl, cycloalkyl, alkenyl, alkynyl, cycloalkylalkyl, alkylamino, alkoxy, -SO₂-NH₂, aryl, heteroaryl, aralkyl, haloalkyl, haloalkoxy, alkoxycarbonyl, alkylaminocarbonyl, heteroarylalkyl, alkylsulfonamide, heterocyclyl, alkylcarbonylaminoalkyl, aryloxy, alkylcarbonyl, acyl, arylcarbonyl, aminocarbonyl, alkylsulfoxide, -SO₂R^{a}, alkylthio, carboxyl, and the like, wherein R^{a} is alkyl or cycloalkyl. Where a carbon atom in an aryl group is replaced with a heteroatom, the resultant ring is referred to herein as a heteroaryl ring.

The term "heteroaryl" as used herein by itself or as part of another group refers but is not limited to 5 to 12 carbon-atom aromatic rings or ring systems containing 1 to 3 rings which are fused together or linked covalently, typically containing 5 to 8 atoms; at least one of which is aromatic in which one or more carbon atoms in one or more of these rings can be replaced by oxygen, nitrogen or sulfur atoms where the nitrogen and sulfur heteroatoms may optionally be oxidized and the nitrogen heteroatoms may optionally be quaternized. Such rings may be fused to an aryl, cycloalkyl, heteroaryl or heterocyclyl ring. Non-limiting examples of such heteroaryl include: piridinyl, azepinyl,...

An "optionally substituted heteroaryl" refers to a heteroaryl having optionally one or more substituents (for example 1 to 4 substituents, for example 1, 2, 3 or 4), selected from those defined above for substituted aryl.

The term "oxo" as used herein refers to the group =O.

The term "alkoxy" or "alkyloxy" as used herein refers to a radical having the formula -OR^{b} wherein R^{b} is alkyl. Preferably, alkoxy is C₁-C₁₀ alkoxy, C₁-C₆ alkoxy, or C₁-C₄ alkoxy. Non-limiting examples of suitable alkoxy include methoxy, ethoxy, propoxy, isopropoxy, butoxy, isobutoxy, sec-butoxy, tert-butoxy, pentyloxy and hexyloxy. Where the oxygen atom in an alkoxy group is substituted with sulfur, the resultant radical is referred to as thioalkoxy. "Haloalkoxy" is an alkoxy group wherein one or more hydrogen atoms in the alkyl group are substituted with halogen. Non-limiting examples of suitable haloalkoxy include fluoromethoxy, difluoromethoxy, trifluoromethoxy, 2,2,2-trifluoroethoxy, 1,1,2,2-tetrafluoroethoxy, 2-fluoroethoxy, 2-chloroethoxy, 2,2-difluoroethoxy, 2,2,2-trichloroethoxy; trichloromethoxy, 2-bromoethoxy, pentafluoroethyl, 3,3,3-trichloropropoxy, 4,4,4-trichlorobutoxy.

The term "carboxy" or "carboxyl" or "hydroxycarbonyl" by itself or as part of another substituent refers to the group -CO₂H. Thus, a carboxyalkyl is an alkyl group as defined above having at least one substituent that is -CO₂H.

The term "alkoxycarbonyl" by itself or as part of another substituent refers to a carboxy group linked to an alkyl radical i.e. to form -C(=O)OR^{e}, wherein R^{e} is as defined above for alkyl.

The term "alkylcarbonyloxy" by itself or as part of another substituent refers to a -O-C(=O)R^{e} wherein R^{e} is as defined above for alkyl.

Whenever the term "substituted" is used in the present invention, it is meant to indicate that one or more hydrogens on the atom indicated in the expression using "substituted" is replaced with a selection from the indicated group, provided that the indicated atom's normal valency is not exceeded, and that the substitution results in a chemically stable compound, i.e. a compound that is sufficiently robust to survive isolation to a useful degree of purity from a reaction mixture, and formulation into a therapeutic agent.

Where groups may be optionally substituted, such groups may be substituted once or more, and preferably once, twice or thrice. Substituents may be selected from, for example, the group comprising halogen, hydroxyl, oxo, nitro, amido, carboxy, amino, cyano haloalkoxy, and haloalkyl.

As used herein the terms such as "alkyl, aryl, or cycloalkyl, each being optionally substituted with" or "alkyl, aryl, or cycloalkyl, optionally substituted with" refers to optionally substituted alkyl, optionally substituted aryl, and optionally substituted cycloalkyl.

Furthermore, where groups are divalent, i.e. have two single bonds for attachment to two other groups, each occurrence thereof may be present in either of both directions in the molecule, even if not specifically indicated in the structural formulae or definition of R groups. For example -CH₂- and -O-CH₂- as part of R₅ means that R₅ may for example be represented by -O-CH₂-O-CH₂-, -CH₂-O-CH₂-O-, but also -O-CH₂-CH₂-O-, and so forth. In particular, any combination of -CH₂-, -O-CH₂- and -CH₂-O- moieties which is chemically feasible is envisaged within the context of the present application for R₅ and R₆.

In the context of the present invention, the term lipid is meant to be a chemically defined substance that is insoluble in water but soluble in amongst others alcohol, ether and chloroform. Ionizable or cationic lipids are lipids that are typically composed of three section: an amine head group, a linker moiety and a hydrophobic tail. The term "ionizable" (or alternatively cationic) in the context of a compound or lipid means the presence of any uncharged group in said compound or lipid which is capable of dissociating by yielding an ion (usually an H⁺ ion) and thus itself becoming positively charged. Alternatively, any uncharged group in said compound or lipid may yield an electron and thus becoming negatively charged.

In the context of the present invention, the linker moiety may be selected from a variety of different linkers, however, disulfide, ketal and ether linkers are particularly preferred. Accordingly, and in order to obtain their lipid character, the compounds of the present invention comprise a lipid tail being represented by R₁ and R₂, wherein the total number of C atoms for both groups combined is at least 8, such as at least 9, at least 10, at least 11, at least 12, at least 13, at least 14, at least 15, at least 16, at least 17, at least 18, at least 19 or at least 20.

Accordingly, in the context of the present invention, R₁ may for example contain 3 C atoms, while R₂ may contain 5 C atoms, thereby the total number of C atoms for both groups combined is at least 8. This also means that R₁ and R₂ do not need to be identical, while in a specific embodiment, they may be identical to each other.

The present invention provides 2 different categories of lipids, i.e. those in which the lipid tail is directly attached to the amide moiety (represented by formulae IVa, IVb, and IVc), and those in which the lipid tail is attached to the amide moiety through carboxylic acid-containing linker moieties (represented by formulae II, Illa, IIIb, and IIIc).

Accordingly, in a specific embodiment, the present application provides a lipid, in particular an ionizable lipid as defined herein and being represented by formula (II) 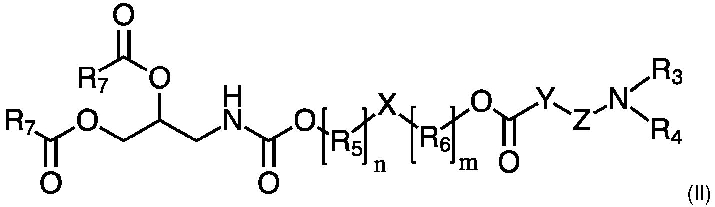 wherein
R₃ and R₄ are each independently a -C₁₋₆alkyl; or R₃ and R₄ taken together with the N atom to which they are attached form a 5-10 membered aromatic or non-aromatic heterocycle; said heterocycle may further optionally comprise one or more additional N atoms, and/or may optionally be substituted with from 1-3 substituents selected from: -C₁₋₆alkyl; and
each R₅ and R₆ is independently-CH₂-;
each R₇ is independently selected from -C₁₋₂₀alkyl, -C₂₋₂₀alkenyl, -C₂₋₂₀alkynyl; wherein each of said -C₁₋₂₀alkyl, -C₂₋₂₀alkenyl, -C₂₋₂₀alkynyl may optionally be substituted with from 1 to 3 -O-(C=O)-R₇, -(C=O)-O-R₇, -C₁₋₂₀alkyl, -C₂₋₂₀alkenyl, -C₂₋₂₀alkynyl; and the total number of C atoms in both R₇ moieties together is at least 5;
m and n are each independently an integer selected from 1, 2, 3 and 4;
X is selected from -O-, -S-, -S-S-, -O-(C=O)-, -O-(C=O)-O-, -(C=N-NH₂)-, -O-CR₈R₉-O-, and -S-CR₈R₉-S-;
each R₈ and R₉ is independently selected from -H, -C₁₋₆alkyl and -C₃₋₆cycloalkyl;
Y is selected from -NH- and -O-; and
Z is -C₁₋₆alkylene-.

In yet a further embodiment, the present application provides a lipid, in particular an ionizable lipid as defined herein and being represented by anyone of formula (Illa), (IIIb), or (IIIc) 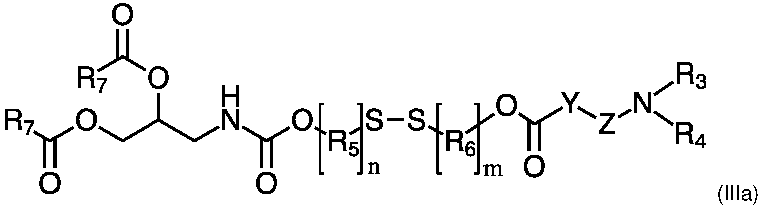 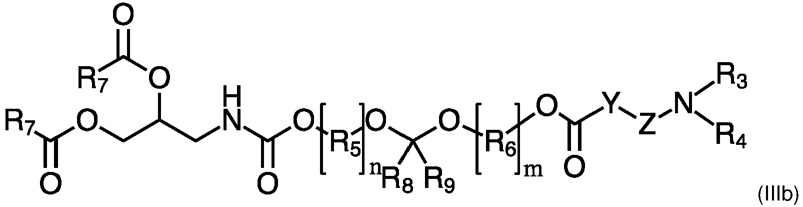 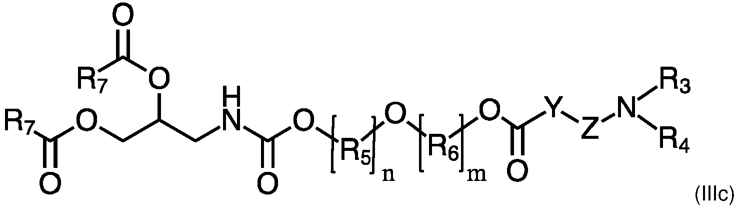 wherein
R₃ and R₄ are each independently a -C₁₋₆alkyl; or R₃ and R₄ taken together with the N atom to which they are attached form a 5-10 membered aromatic or non-aromatic heterocycle; said heterocycle may further optionally comprise one or more additional N atoms, and/or may optionally be substituted with from 1-3 substituents selected from: -C₁₋₆alkyl; and
each R₅ and R₆ is independently -CH₂-;
each R₇ is independently selected from -C₁₋₂₀alkyl, -C₂₋₂₀alkenyl, -C₂₋₂₀alkynyl; wherein each of said -C₁₋₂₀alkyl, -C₂₋₂₀alkenyl, -C₂₋₂₀alkynyl may optionally be substituted with from 1 to 3 -O-(C=O)-R₇, -(C=O)-O-R₇, -C₁₋₂₀alkyl, -C₂₋₂₀alkenyl, -C₂₋₂₀alkynyl; and the total number of C atoms in both R₇ moieties together is at least 5;
m and n are each independently an integer selected from 1, 2, 3 and 4;
each R₈ and R₉ is independently selected from -H, -C₁₋₆alkyl and -C₃₋₆cycloalkyl;
Y is selected from -NH- and -O-; and
Z is -C₁₋₆alkylene-.

The present invention further provides a lipid, in particular an ionizable lipid as defined herein and being selected from the list comprising:

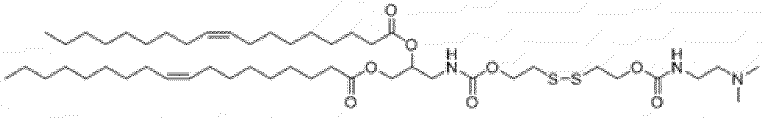

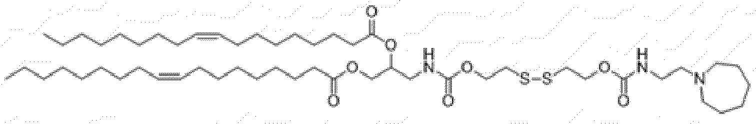

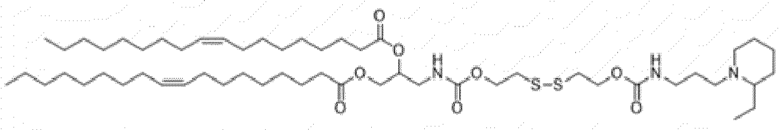

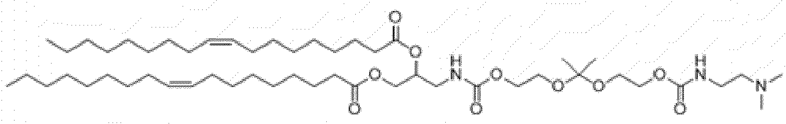

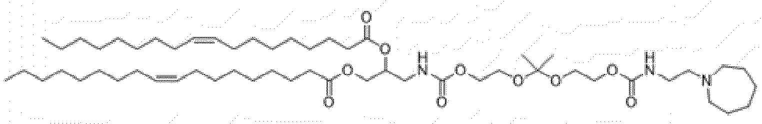

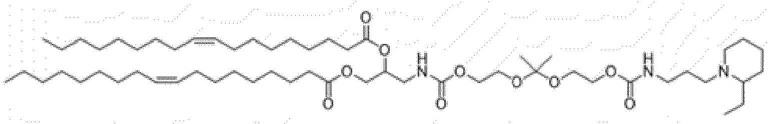

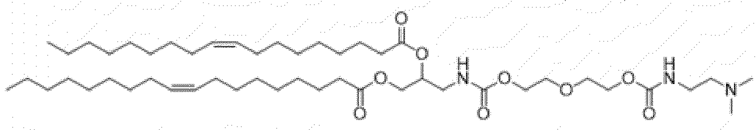

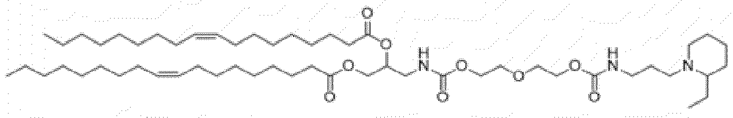

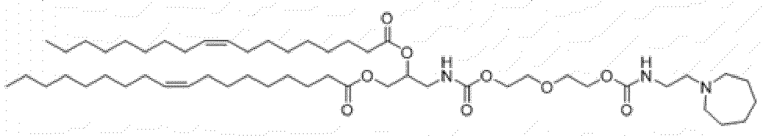

In yet a further embodiment, the present application provides a lipid, in particular an ionizable lipid as defined herein and being represented by anyone of formula (IVa), (IVb), and (IVc) 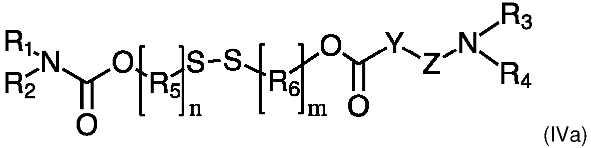 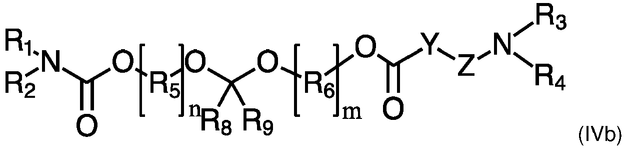 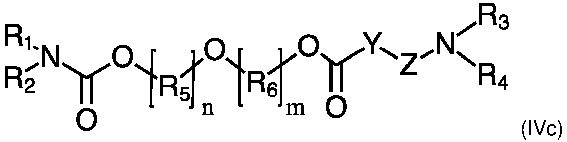 wherein
R₁ and R₂ are each independently selected from -H, -C₁₋₂₀alkyl, -C₂₋₂₀alkenyl, and -C₂₋₂₀alkynyl; wherein each of said -C₁₋₂₀alkyl, -C₂₋₂₀alkenyl, and -C₂₋₂₀alkynyl may optionally be substituted with from 1 to 3 -O-(C=O)-R₇, -O-(C=O)-R₇, -C₁₋₂₀alkyl, -C₂₋₂₀alkenyl, and -C₂₋₂₀alkynyl; and wherein the total number of C atoms in R₁ and R₂ together is at least 8;
R₃ and R₄ are each independently a -C₁₋₆alkyl; or R₃ and R₄ taken together with the N atom to which they are attached form a 5-10 membered aromatic or non-aromatic heterocycle; said heterocycle may further optionally comprise one or more additional N atoms, and/or may optionally be substituted with from 1-3 substituents selected from: -C₁₋₆alkyl; and
each R₅ and R₆ is independently-CH₂-;
m and n are each independently an integer selected from 1, 2, 3 and 4;
each R₈ and R₉ is independently selected from -H, -C₁₋₆alkyl and -C₃₋₆cycloalkyl;
Y is selected from -NH- and -O-; and
Z is -C₁₋₆alkylene-.

In a further embodiment, the present invention provides a lipid, in particular an ionizable lipid as defined herein and being selected from the list comprising:

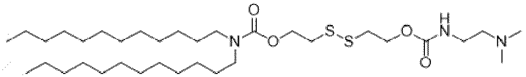

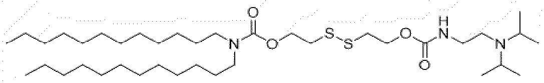

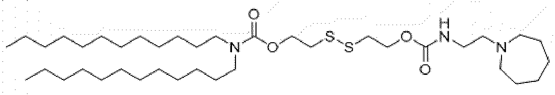

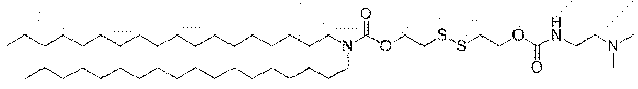

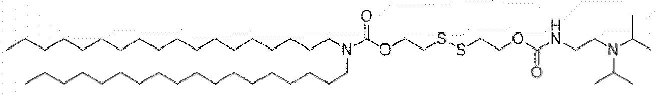

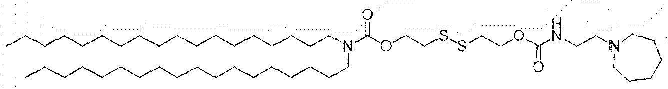

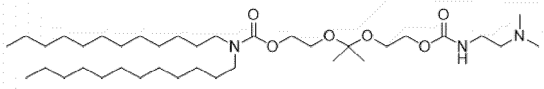

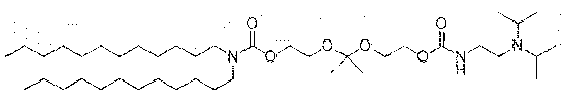

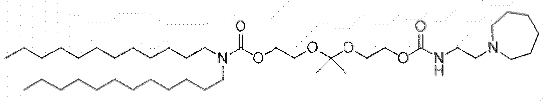

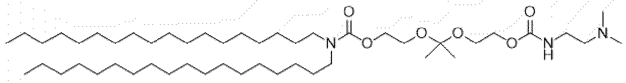

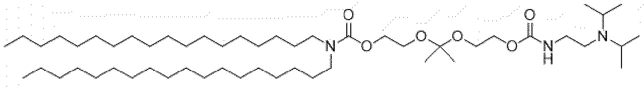

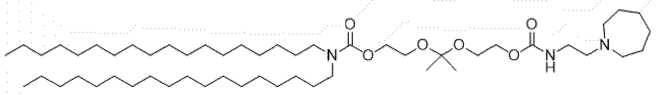

In yet a further embodiment, the present invention provides a lipid, in particular an ionizable lipid as defined herein and being represented by formula (V) 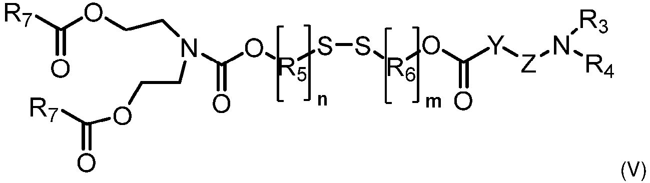 wherein
R₃ and R₄ are each independently a -C₁₋₆alkyl; or R₃ and R₄ taken together with the N atom to which they are attached form a 5-10 membered aromatic or non-aromatic heterocycle; said heterocycle may further optionally comprise one or more additional N atoms, and/or may optionally be substituted with from 1-3 substituents selected from: -C₁₋₆alkyl; and
each R₅ and R₆ is independently-CH₂-;
each R₇ is independently selected from -C₁₋₂₀alkyl, -C₂₋₂₀alkenyl, -C₂₋₂₀alkynyl; wherein each of said -C₁₋₂₀alkyl, -C₂₋₂₀alkenyl, -C₂₋₂₀alkynyl may optionally be substituted with from 1 to 3 -O-(C=O)-R₇, -O-(C=O)-R₇, -C₁₋₂₀alkyl, -C₂₋₂₀alkenyl, -C₂₋₂₀alkynyl; and the total number of C atoms in both R₇ moieties together is at least 5;
m and n are each independently an integer selected from 1, 2, 3 and 4;
Y is selected from -NH- and -O-; and
Z is -C₁₋₆alkylene-.

The present invention further provides a lipid, in particular an ionizable lipid as defined herein and being selected from the list comprising:

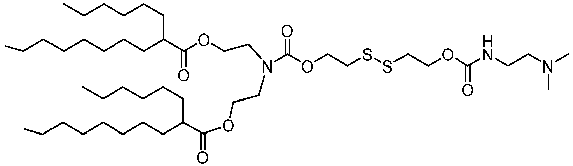

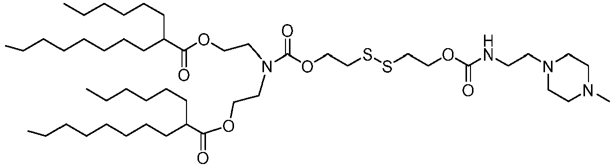

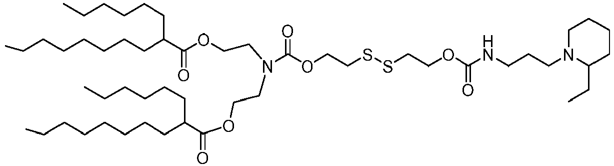

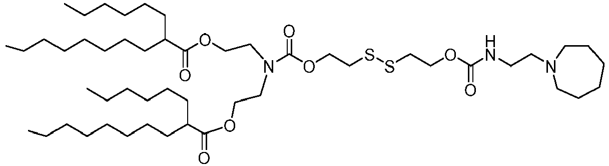

In yet a further embodiment, the present invention provides a lipid, in particular an ionizable lipid as defined herein and being represented by anyone of formula (Vla) or (VIb) 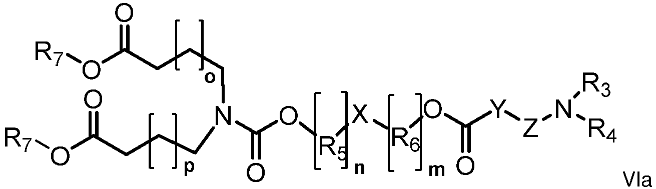 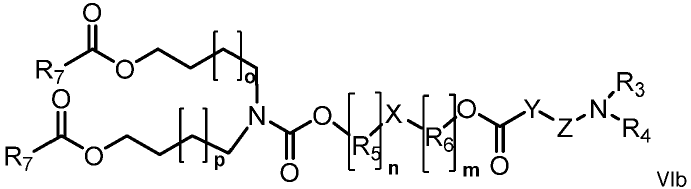 wherein
R₃ and R₄ are each independently a -C₁₋₆alkyl; or R₃ and R₄ taken together with the N atom to which they are attached form a 5-10 membered aromatic or non-aromatic heterocycle; said heterocycle may further optionally comprise one or more additional N atoms, and/or may optionally be substituted with from 1-3 substituents selected from: -C₁₋₆alkyl; and
each R₅ and R₆ is independently -CH₂-;
each R₇ is independently selected from -C₁₋₂₀alkyl, -C₂₋₂₀alkenyl, -C₂₋₂₀alkynyl; wherein each of said -C₁₋₂₀alkyl, -C₂₋₂₀alkenyl, -C₂₋₂₀alkynyl may optionally be substituted with from 1 to 3 -O-(C=O)-R₇, -O-(C=O)-R₇, -C₁₋₂₀alkyl, -C₂₋₂₀alkenyl, -C₂₋₂₀alkynyl; and the total number of C atoms in both R₇ moieties together is at least 5;
m and n are each independently an integer selected from 1, 2, 3 and 4;
o and p are each independently an integer selected from 1-10;
Y is selected from -NH- and -O-; and
Z is -C₁₋₆alkylene-.

In a very specific embodiment of the present invention one or more of the following applies:
R₁ and R₂ are each independently selected from -H, -C₁₋₂₀alkyl, -C₂₋₂₀alkenyl, and -C₂₋₂₀alkynyl; wherein each of said -C₁₋₂₀alkyl, -C₂₋₂₀alkenyl, and -C₂₋₂₀alkynyl may be substituted with from 1 to 3 -O-(C=O)-R₇, -C₁₋₂₀alkyl, -C₂₋₂₀alkenyl, and -C₂₋₂₀alkynyl; and wherein the total number of C atoms in R₁ and R₂ together is at least 8;
R₁ and R₂ are each independently selected from -H, -C₁₋₂₀alkyl, -C₂₋₂₀alkenyl, and -C₂₋₂₀alkynyl; wherein each of said -C₁₋₂₀alkyl, -C₂₋₂₀alkenyl, and -C₂₋₂₀alkynyl may optionally be substituted with from 1 to 3 -O-(C=O)-R₇, -(C=O)-O-R₇, -C₁₋₂₀alkyl, -C₂₋₂₀alkenyl, and -C₂₋₂₀alkynyl; and wherein the total number of C atoms in R₁ and R₂together is at least 8;
R₃ and R₄ are each independently a -C₁₋₆alkyl; or R₃ and R₄ taken together with the N atom to which they are attached form a 5-10 membered aromatic or non-aromatic heterocycle; said heterocycle may further optionally comprise one or more additional N atoms, and/or may optionally be substituted with from 1-3 substituents selected from: -C₁₋₆alkyl; and
each R₅ and R₆ is independently-CH₂-;
each R₇ is independently selected from -C₁₋₂₀alkyl, -C₂₋₂₀alkenyl, -C₂₋₂₀alkynyl; wherein each of said -C₁₋₂₀alkyl, -C₂₋₂₀alkenyl, -C₂₋₂₀alkynyl may optionally be substituted with from 1 to 3 -O-(C=O)-R₇, -C₁₋₂₀alkyl, -C₂₋₂₀alkenyl, -C₂₋₂₀alkynyl;
each R₇ is independently selected from -C₁₋₂₀alkyl, -C₂₋₂₀alkenyl, -C₂₋₂₀alkynyl; wherein each of said -C₁₋₂₀alkyl, -C₂₋₂₀alkenyl, -C₂₋₂₀alkynyl may optionally be substituted with from 1 to 3 - O-(C=O)-R₇, -(C=O)-O-R₇, -C₁₋₂₀alkyl, -C₂₋₂₀alkenyl, -C₂₋₂₀alkynyl;
m and n are each independently an integer selected from 1, 2, 3 and 4;
X is selected from -O-, -S-, -S-S-, -O-(C=O)-, -O-(C=O)-O-, -(C=N-NH₂)-, -O-CR₈R₉-O-, and -S-CR₈R₉-S-;
each R₈ and R₉ is independently selected from -H, -C₁₋₆alkyl and -C₃₋₆cycloalkyl;
Y is selected from -NH- and -O-; and
Z is -C₁₋₆alkylene-.

In another particular embodiment, the present invention provides a lipid, in particular an ionizable lipid as defined herein and being selected from the list comprising:

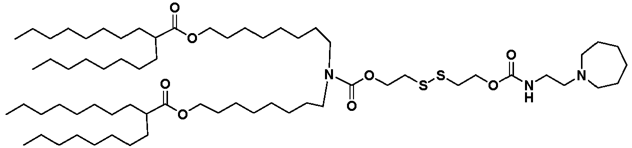

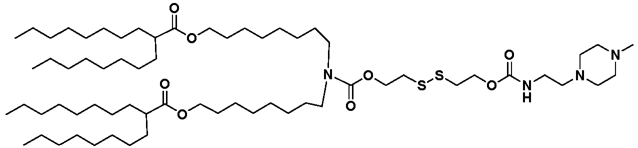

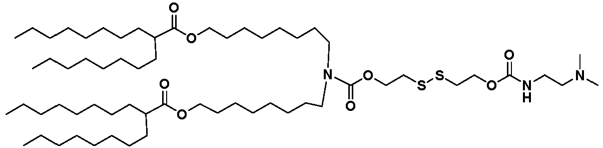

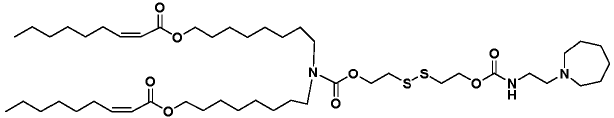

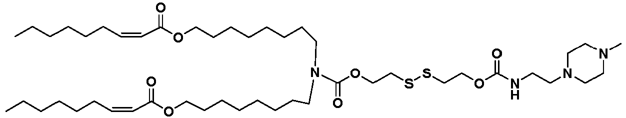

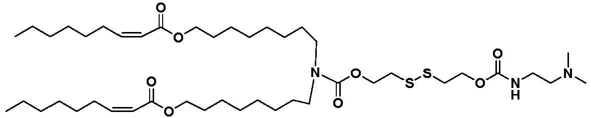

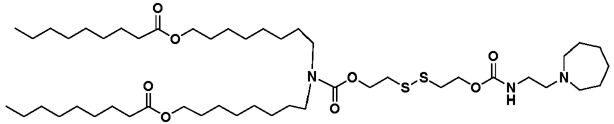

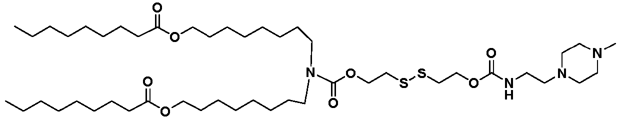

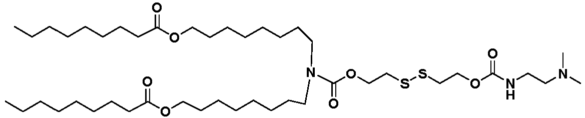

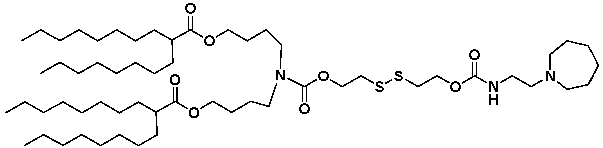

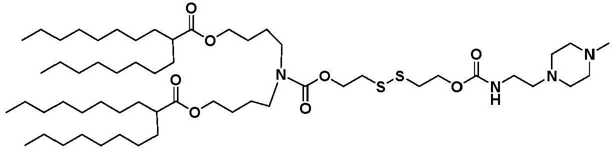

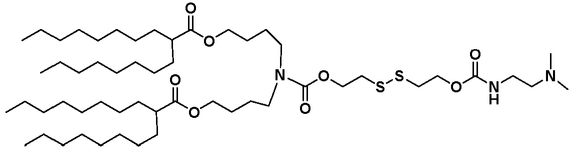

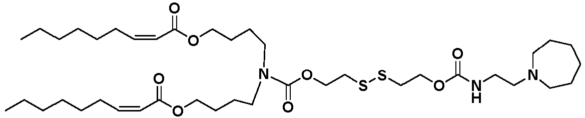

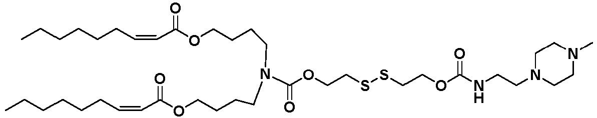

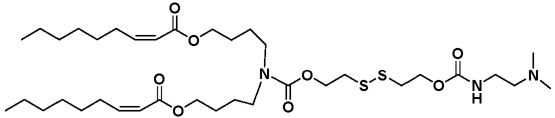

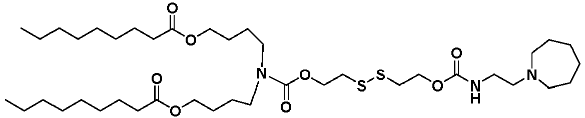

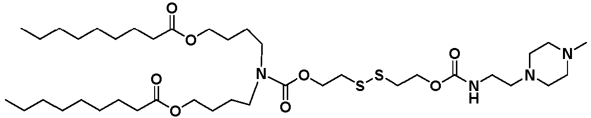

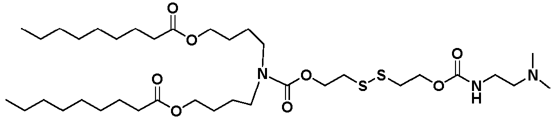

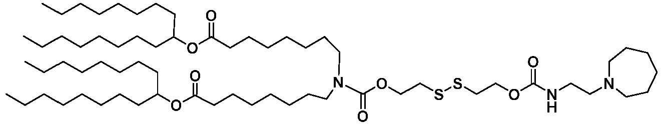

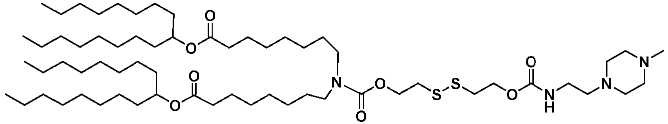

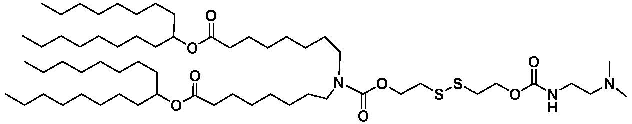

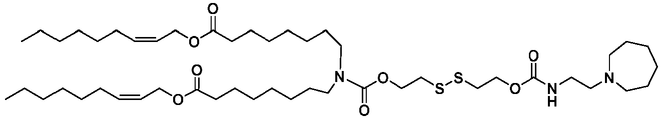

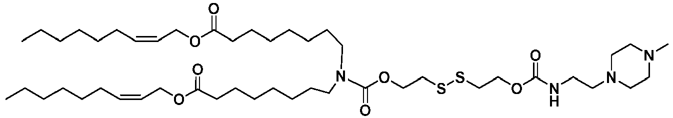

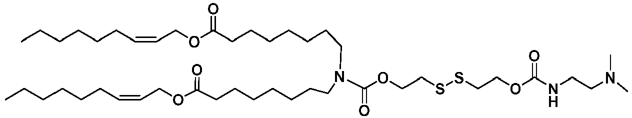

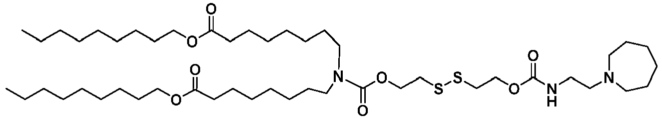

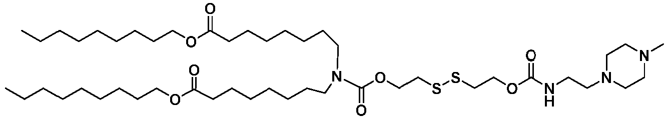

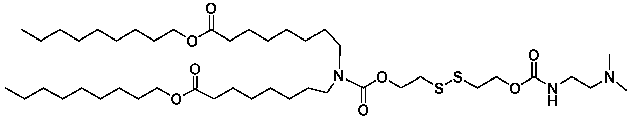

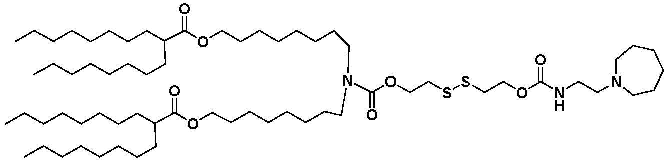

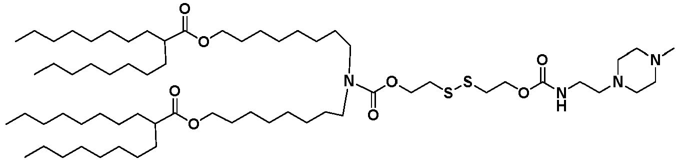

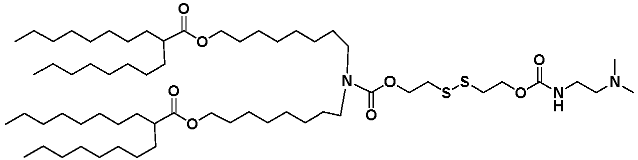

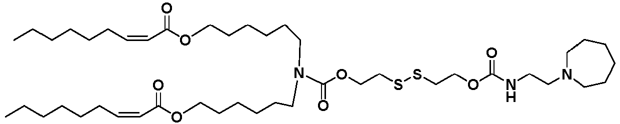

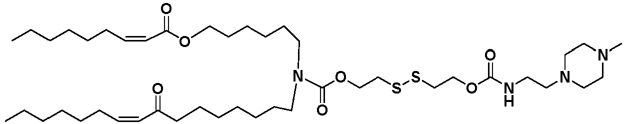

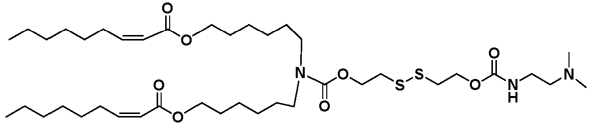

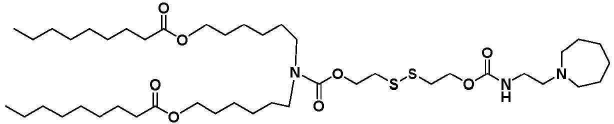

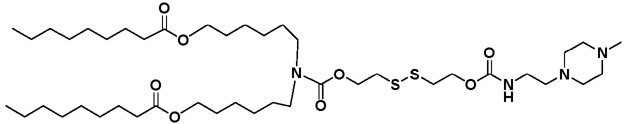

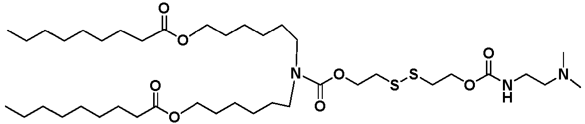

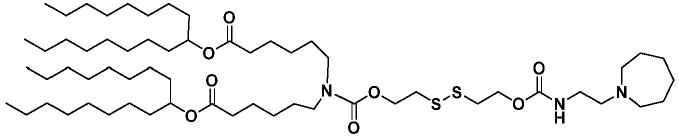

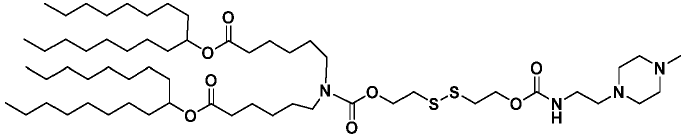

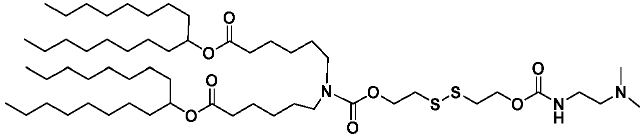

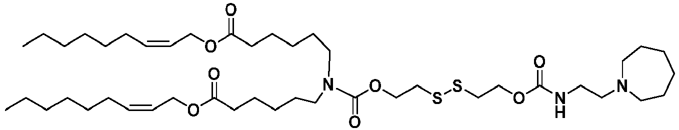

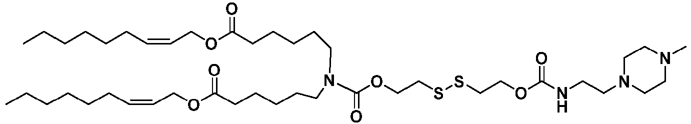

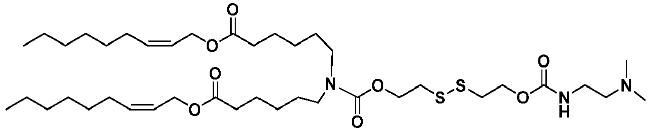

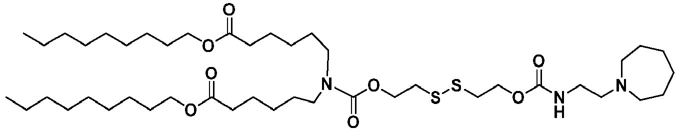

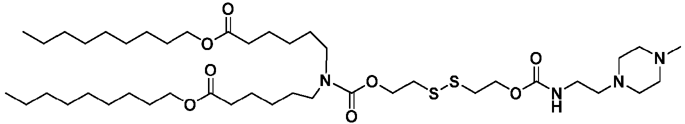

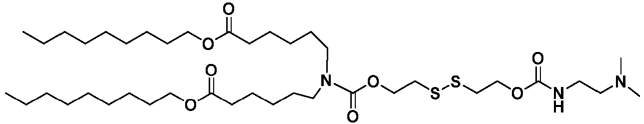

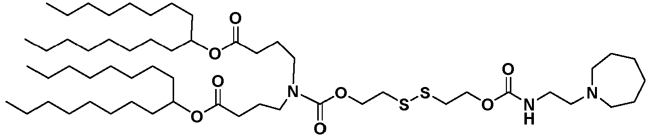

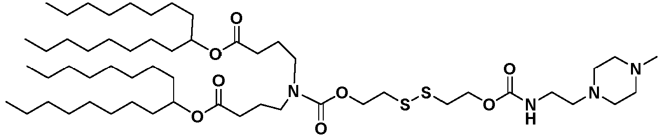

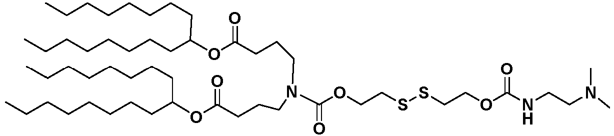

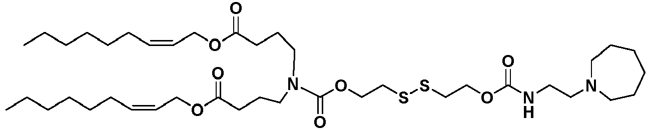

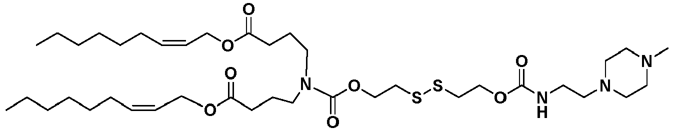

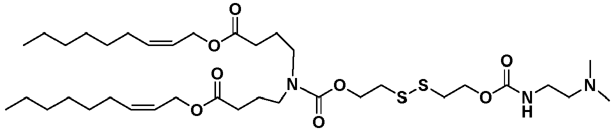

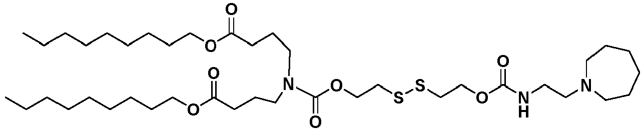

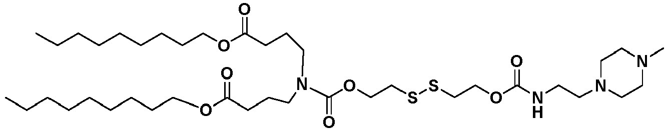

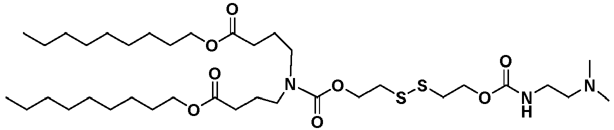

All of the lipids as defined herein may occur as different isomers/stereomers. In particular, the lipids as defined herein may occur in the trans or cis configuration, such as when they contain double bonds. In a preferred embodiment, the lipids as defined herein occur in the cis configuration. In the context of the present invention, the term 'cis' indicates that the functional groups are on the same side of a plane, whereas 'trans' means that they are on opposite sides.

In yet a further embodiment, the present invention provides a lipid, in particular an ionizable lipid as defined herein, wherein the total number of C atoms in R₁ and R₂ together is at least 14, such as at least 15, at least 17, at least 18, at least 19 or at least 20.

The present invention further provides a lipid, in particular an ionizable lipid as defined herein; wherein each R₅ and R₆ is independently -CH₂-, i.e. both groups are -CH₂-.

In a further embodiment, the present invention provides a lipid, in particular an ionizable lipid as defined herein; wherein m and n are the same, being an integer selected from 1, 2, 3 and 4; such as 1 or 2 or 3 or 4; preferably 2.

In yet a further embodiment, the present invention provides a lipid, in particular an ionizable lipid as defined herein; wherein Y is -NH-.

In a further aspect, the present invention provides a lipid nanoparticle or lipid nanoparticle composition comprising a lipid, in particular an ionizable lipid as defined herein.

In the context of the present invention, the term lipid nanoparticle (LNP), also termed solid lipid nanoparticles, is meant to be a nanoparticle comprising lipids. They are often used as a pharmaceutical drug delivery system or pharmaceutical formulation. LNPs as drug delivery vehicle were first approved in 2018, and are currently used in several candidate RNA based vaccines. A lipid nanoparticle is typically spherical with an average diameter between 10 and 1000 nanometers, and possesses a lipid core matrix that can solubilize lipophilic molecules. The term lipid is used here in a broader sense and includes triglycerides, diglycerides, monoglycerides, fatty acids, steroids (e.g. cholesterol) and waxes. Biological membrane lipids such as phospholipids, sphingomyelins, bile acids and sterols are typically used as stabilizers in LNPs.

As used herein, the term "nanoparticle" refers to any particle having a diameter making the particle suitable for systemic, in particular intravenous administration, of, in particular, nucleic acids, typically having a diameter of less than 1000 nanometers (nm), preferably less than 500 nm, even more preferably less than 200 nm, such as for example between 50 and 200 nm; preferably between 80 and 160 nm.

Accordingly, in the context of the present invention, the nanoparticles as disclosed herein further comprise one or more additional lipids either or not acting as stabilizers, such as a phospholipid, a sterol and/or a PEG lipid.

In the context of the present invention, the term "PEG lipid" or alternatively "PEGylated lipid" is meant to be any suitable lipid modified with a PEG (polyethylene glycol) group. Particularly suitable PEG lipids in the context of the present invention are characterized in being C18-PEG lipids, C14-PEG lipids (e.g. DMG-PEG or DMG-PEG2000) or C16-PEG lipids.

C18-PEG lipids contain a polyethylene glycol moiety, which defines the molecular weight of the lipids, as well as a fatty acid tail comprising 18 C-atoms. In a particular embodiment, said C18-PEG2000 lipid is selected from the list comprising: a (distearoyl-based)-PEG2000 lipid such as DSG-PEG2000 lipid (2-distearoyl-*rac*-glycero-3-methoxypolyethylene glycol-2000) or DSPE-PEG2000 lipid (1,2-distearoyl-*sn*-glycero-3-phosphoethanolamine-N-[methoxy(polyethylene glycol)-2000]), or a (dioleolyl-based)-PEG2000 lipid such as DOG-PEG2000 lipid (1,2-Dioleolyl-rac-glycerol) or DOPE-PEG2000 lipid (1,2-dioleoyl-sn-glycero-3-phosphoethanolamine-N-[amino(polyethylene glycol)-2000])

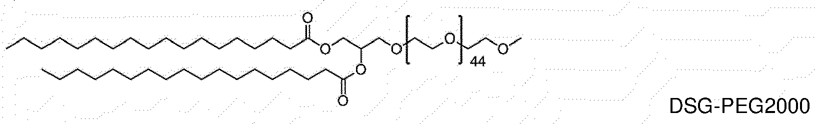

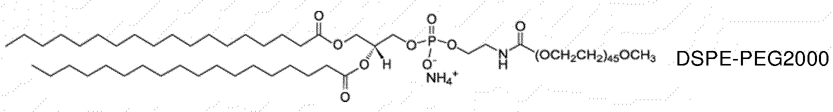

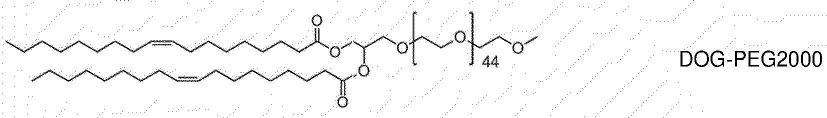

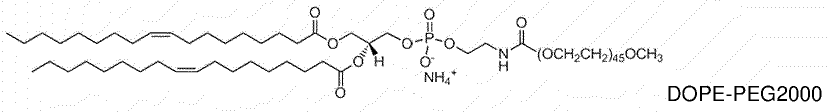

C14-PEG lipids contain a polyethylene glycol moiety, which defines the molecular weight of the lipids, as well as a fatty acid tail comprising 14 C-atoms. In a particular embodiment, said C14-PEG2000 lipid is based on dimyristoyl, i.e. having 2 C14 tails, such as selected from the list comprising: a (dimyristoyl-based)-PEG2000 lipid such as DMG-PEG2000 lipid (1,2-dimyristoyl-*rac*-glycero-3-methoxypolyethylene glycol-2000) or 2-Dimyristoyl-*sn*-Glycero-3-Phosphoethanolamine glycol-2000 (DMPE-PEG2000).

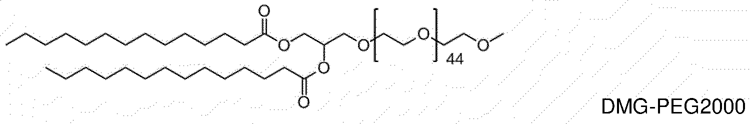

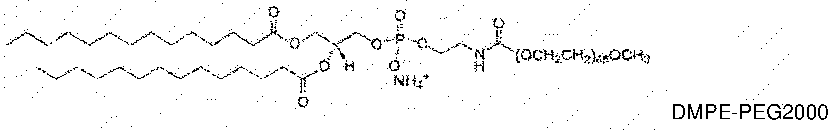

In the context of the present invention, the term "phospholipid" is meant to be a lipid molecule consisting of two hydrophobic fatty acid "tails" and a hydrophilic "head" consisting of a phosphate group. The two components are most often joined together by a glycerol molecule, hence, the phospholipid of the present invention is preferably a glycerol-phospholipid. Furthermore, the phosphate group is often modified with simple organic molecules such as choline (i.e. rendering a phosphocholine) or ethanolamine (i.e. rendering a phosphoethanolamine).

Suitable phospholipids within the context of the invention can be selected from the list comprising: 1,2-Dioleoyl-sn-glycero-3-phosphoethanolamine (DOPE), 1,2-Dioleoyl-sn-glycero-3-phosphocholine (DOPC), 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C 16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), sphingomyelin, and mixtures thereof.

In a more specific embodiment, said phospholipid is selected from the list comprising: 1,2-Dioleoyl-sn-glycero-3-phosphoethanolamine (DOPE), 1,2-Dioleoyl-sn-glycero-3-phosphocholine (DOPC), and mixtures thereof.

In the context of the present invention, the term "sterol", also known as steroid alcohol, is a subgroup of steroids that occur naturally in plants, animals and fungi, or can be produced by some bacteria. In the context of the present invention, any suitable sterol may be used, such as selected from the list comprising cholesterol, ergosterol, campesterol, oxysterol, antrosterol, desmosterol, nicasterol, sitosterol and stigmasterol; preferably cholesterol.

In a specific embodiment of the present invention one or more of the following applies:
- said LNP comprises about and between 35 mol% and 65 mol% of said ionizable lipid;
- said LNP comprises about and between 5 mol% and 25 mol% of said phospholipid;
- said LNP comprises about and between 0.5 mol% and 3.0 mol% of said PEG lipid;
balanced by the amount of said sterol.

In yet a further embodiment of the present invention, the lipid nanoparticle or lipid nanoparticle composition as defined herein further comprises a cargo molecule such as a pharmaceutically active agent (e.g. small molecule) or a biomolecule, such as a peptide, protein or a nucleic acid. In a particular embodiment, the cargo may be a nucleic acid, such as DNA or RNA; preferably mRNA. In another particular embodiment, the cargo may be a TLR agonist, such as for example the TLR3 agonist polyl:C, or the TLR9 agonist CpG.

Prior to being loaded in the lipid nanoparticles, the cargo molecules may further be modified to induce an overall polyanionic nature to the molecules. This can for example be done by bonding them to a Glu10 moiety as exemplified in the examples part. The Glu10 moiety is a moiety of 10 glutamic acids which increases the polyanionic nature of the molecule to which it is attached.

Accordingly, the lipid nanoparticles and lipid nanoparticle compositions of the present invention are particularly suitable for the intracellular delivery of their cargo molecules. Hence, the present invention provides the use of the lipid nanoparticles and lipid nanoparticle compositions as defined herein for the intracellular delivery of cargo molecules.

In a particular embodiment, the lipid nanoparticle or lipid nanoparticle composition as defined herein further comprises a nucleic acid, preferably mRNA.

A "nucleic acid" in the context of the invention is a deoxyribonucleic acid (DNA) or preferably a ribonucleic acid (RNA), more preferably mRNA. Nucleic acids include according to the invention genomic DNA, cDNA, mRNA, recombinantly produced and chemically synthesized molecules. A nucleic acid may according to the invention be in the form of a molecule which is single stranded or double stranded and linear or closed covalently to form a circle. A nucleic acid can be employed for introduction into, i.e. transfection of cells, for example, in the form of RNA which can be prepared by in vitro transcription from a DNA template. The RNA can moreover be modified before application by stabilizing sequences, capping, and/or polyadenylation.

In the context of the present invention, the term "RNA" relates to a molecule which comprises ribonucleotide residues and preferably being entirely or substantially composed of ribonucleotide residues. "Ribonucleotide" relates to a nucleotide with a hydroxyl group at the 2'-position of a β-D-ribofuranosyl group. The term includes double stranded RNA, single stranded RNA, isolated RNA such as partially purified RNA, essentially pure RNA, synthetic RNA, recombinantly produced RNA, as well as modified RNA that differs from naturally occurring RNA by the addition, deletion, substitution and/or alteration of one or more nucleotides. Such alterations can include addition of non-nucleotide material, such as to the end(s) of a RNA or internally, for example at one or more nucleotides of the RNA. Nucleotides in RNA molecules can also comprise non-standard nucleotides, such as non-naturally occurring nucleotides or chemically synthesized nucleotides or deoxynucleotides. These altered RNAs can be referred to as analogs. Nucleic acids may be comprised in a vector. The term "vector" as used herein includes any vectors known to the skilled person including plasmid vectors, cosmid vectors, phage vectors such as lambda phage, viral vectors such as adenoviral or baculoviral vectors, or artificial chromosome vectors such as bacterial artificial chromosomes (BAC), yeast artificial or analogs of naturally-occurring RNA.

According to the present invention, the term "RNA" includes and preferably relates to "mRNA" which means "messenger RNA" and relates to a "transcript" which may be produced using DNA as template and encodes a peptide or protein. mRNA typically comprises a 5' untranslated region (5' -UTR), a protein or peptide coding region and a 3' untranslated region (3'-UTR). mRNA has a limited halftime in cells and in vitro. Preferably, mRNA is produced by in vitro transcription using a DNA template. In one embodiment of the invention, the RNA is obtained by in vitro transcription or chemical synthesis. The in vitro transcription methodology is known to the skilled person. For example, there is a variety of in vitro transcription kits commercially available.

In a further aspect, the present invention provides a pharmaceutical composition comprising one or more LNP's as defined herein and a pharmaceutically acceptable agent, such as a carrier orexcipient. Such pharmaceutical compositions are particularly suitable as a vaccine. Thus, the invention also provides a vaccine comprising one or more LNP's according to the present invention.

In the context of the present invention, the term "vaccine" as used herein is meant to be any preparation intended to provide adaptive immunity (antibodies and/or T cell responses) against a disease. To that end, a vaccine as meant herein contains at least one nucleic acid molecule, e.g. mRNA molecule encoding an antigen to which an adaptive immune response is mounted. This antigen can be present in the format of a weakened or killed form of a microbe, a protein or peptide, or an antigen encoding a nucleic acid. An antigen in the context of this invention is meant to be a protein or peptide recognized by the immune system of a host as being foreign, thereby stimulating the production of antibodies against is, with the purpose of combating such antigens. Vaccines can be prophylactic (example: to prevent or ameliorate the effects of a future infection by any natural or "wild" pathogen), or therapeutic (example, to actively treat or reduce the symptoms of an ongoing disease). The administration of vaccines is called vaccination.

The vaccine of the invention may be used for inducing an immune response, in particular an immune response against a disease-associated antigen or cells expressing a disease-associated antigen, such as an immune response against cancer. Accordingly, the vaccine may be used for prophylactic and/or therapeutic treatment of a disease involving a disease-associated antigen or cells expressing a disease- associated antigen, such as cancer. Preferably said immune response is a T cell response. In one embodiment, the disease-associated antigen is a tumor antigen. The antigen encoded by the RNA comprised in the nanoparticles described herein preferably is a disease-associated antigen or elicits an immune response against a disease-associated antigen or cells expressing a disease-associated antigen.

The present invention also provides the LNP's, pharmaceutical compositions and vaccines according to this invention for use in human or veterinary medicine. The use of the LNP's, pharmaceutical compositions and vaccines according to this invention for human or veterinary medicine is also intended. Finally, the invention provides a method for the prophylaxis and treatment of human and veterinary disorders, by administering the LNP's, pharmaceutical compositions and vaccines according to this invention to a subject in need thereof.

The present invention further provides the use of an LNP, a pharmaceutical composition or a vaccine according to the present invention for the immunogenic delivery of said one or more nucleic acid molecules. As such the LNP's, pharmaceutical compositions and vaccine of the present invention are highly useful in the treatment several human and veterinary disorders. Thus, the present invention provides the LNP's, pharmaceutical compositions and vaccines of the present invention for use in the treatment of cancer or infectious diseases.

The lipid nanoparticles of the present invention may be prepared in accordance with the protocols as specified in the Examples part. More generally, the LNP's may be prepared using a method comprising:
- preparing a first alcoholic composition comprising said ionizable lipid, said phospholipid, said sterol, said PEG lipid, and a suitable alcoholic solvent;
- preparing a second aqueous composition comprising said one or more nucleic acids and an aqueous solvent; and
- mixing said first and second composition in a microfluidic mixing device.

In further detail, the lipid components are combined in suitable concentrations in an alcoholic vehicle such as ethanol. Thereto, an aqueous composition comprising the nucleic acid is added, and subsequently loaded in a microfluidic mixing device.

The aim of microfluidic mixing is to achieve thorough and rapid mixing of multiple samples (i.e. lipid phase and nucleic acid phase) in a microscale device. Such sample mixing is typically achieved by enhancing the diffusion effect between the different species flows. Thereto several microfluidic mixing devices can be used, such as for example reviewed in Lee et al., 2011. A particularly suitable microfluidic mixing device according to the present invention is the NanoAssemblr from Precision Nanosystems.

Other technologies suitable for preparing the LNP's of the present invention include dispersing the components in a suitable dispersing medium, for example, aqueous solvent and alcoholic solvent, and applying one or more of the following methods: ethanol dilution method, a simple hydration method, sonication, heating, vortex, an ether injecting method, a French press method, a cholic acid method, a Ca²⁺ fusion method, a freeze-thaw method, a reversed-phase evaporation method, T-junction mixing, Microfluidic Hydrodynamic Focusing, Staggered Herringbone Mixing, and the like.

The ionizable lipids of the present invention can be prepared according to the reaction schemes provided in the examples hereinafter, but those skilled in the art will appreciate that these are only illustrative for the invention and that the compounds of this invention can be prepared by any of several standard synthetic processes commonly used by those skilled in the art of organic chemistry.

### EXAMPLES

### EXAMPLE 1 : PREPARATION OF THE LIPIDS

### 1. General information

Unless otherwise stated, all glassware was oven dried before use and all reactions were carried out under an argon atmosphere using standard Schlenk-techniques. Dry solvents were purchased from Acros Organics or Sigma-Aldrich and used without further purification. All reagents were purchased from commercial sources and were used without further purification unless otherwise stated. Reaction progress was monitored by thin layer chromatography (TLC) performed on aluminum plates coated with Kieselgel F254 with 0.2 mm thickness. Visualization was achieved by ultraviolet light (254 nm) or by staining with potassium permanganate. Flash column chromatography was performed using silica gel 60 (230-400 mesh, Merck ans co.). Mass spectra were obtained using a Finnigan MAT 8200 (70 eV), an Agilent 5973 (70 eV), using electrospray ionization (ESI) or electron impact ionization (EI). All 1H NMR, 13C NMR were recorded on a Bruker AV-400 in Chloroform-d1 or DMSO-d6. Chemical shifts are given in parts per million (ppm), referenced to tetramethylsilane using the solvent peak as internal standard (CDCl₃: ¹H = 7.26 ppm, ¹³C = 77.16 ppm; CD₃SOCD₃: ¹H = 2.50 ppm, ¹³C = 39.52 ppm). Coupling constants were quoted in Hz. ¹H NMR splitting patterns were designated as singlet (s), broad (brd), doublet (d), triplet (t), quartet (q), pentet (p), sextet (se), septet (sep), octet (o) or combinations thereof. Splitting patterns that could not be interpreted were designated as multiplet (m).

### 2. Synthesis of lipids

**2.1, The general route for the synthesis of lipids represented by the structure of formula I, and more specifically IVa is shown below.**

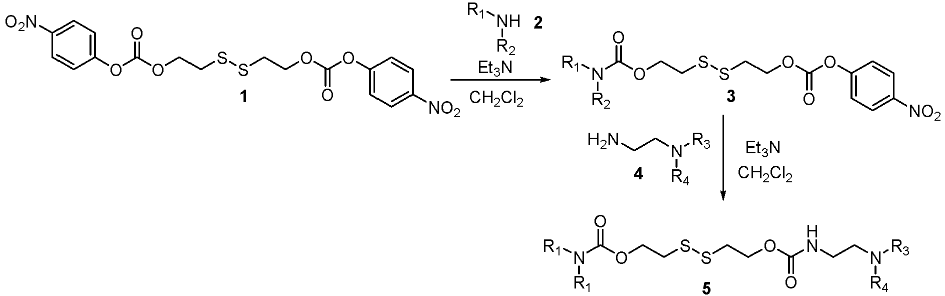

### Synthesis of compound 3

The amine **2** (1.0 equiv.) and Et₃N (1.5 equiv.) were first dissolved in CHCl₃/Hexane/THF (1/1/1). Then the prepared solution was dropwise added to a stirred solution of compound **1 (Amano et al., 2017)** (3.0 equiv.) in CH₂Cl₂ at 0 °C. The resulting mixture was stirred vigorously and allowed to warm to room temperature over 2 h. Then the solvent was removed under vacuum, and the remaining residue was dissolved in CH₂Cl₂, washed with sat. citric acid (aq.), brine and dried over Na₂SO₄, filtered and concentrated. The crude product was purified by silica gel column chromatography (hexane/ethyl acetate = 15:1 to 10:1) to afford compound **3.**

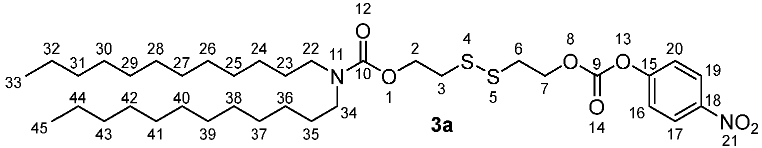

**Yield:** 62%. Colorless oil. **¹H NMR (400 MHz, Chloroform-*d*)** δ 8.29 (d, *J* = 9.2 Hz, 2H, **H17 & H19),** 7.40 (d, *J =* 9.3 Hz, 2H, **H16 & H20),** 4.54 (t, *J =* 6.6 Hz, 2H, OCH₂), 4.34 (t, *J =* 6.4 Hz, 2H, OC**H**₂), 3.18 (brd, 4H, **H22 & H34),** 3.03 (t, *J =* 6.6 Hz, 2H, SC**H**₂), 2.97 (t, *J =* 6.4 Hz, 2H, SC**H**₂), 1.54-1.48 (m, 4H, **H23 & H35),** 1.25 (brd, 36H), 0.88 (t, *J=* 6.7 Hz, 6H, **H33 & H45)** ppm. **¹³C NMR (100 MHz, Chloroform-*d*)** δ 156.0 (**C**=O), 155.6 (Ar-**C**, quaternary), 152.5 (**C**=O), 125.5 (Ar**C**-H), 121.9 (Ar**C**-H), 67.0 **(C2 or C7),** 62.8 **(C2 or C7),** 47.8 **(C22 or C34),** 47.2 **(C22 or C34),** 38.2 **(C3 or C6),** 36.9 **(C3 or C6),** 32.1, 29.81, 29.79, 29.76, 29.6, 29.5, 28.8 **(C23 or C35),** 28.3 **(C23 or C35),** 27.0, 22.8, 14.3 **(C33 & C45)** ppm. **LRMS (ESI) (m/z):** calculated for [M+H]⁺ (C₃₆H₆₃O₇N₂S₂) requires 699.4, found: 699.4.

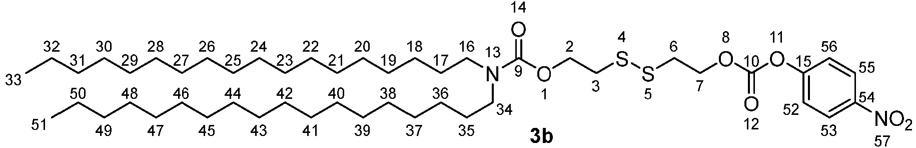

**Yield:** 58%. Colorless oil. **¹H NMR (400 MHz, Chloroform-*d*)** δ 8.28 (d, *J =* 9.2 Hz, 2H, **H53 & H55),** 7.40 (d, *J =* 9.2 Hz, 2H, **H52 & H56),** 4.54 (t, *J =* 6.6 Hz, 2H, O**CH₂**)**,** 4.34 (t, *J =* 6.5 Hz, 2H, **OCH₂),** 3.21-3.15 (m, 4H, **H16** & **H34),** 3.03 (t, *J =* 6.6 Hz, 2H, S**CH₂**), 2.97 (t, *J* = 6.4 Hz, 2H, S**CH₂**), 1.53-1.49 (m, 4H, **H17 & H35),** 1.25 (brd, 60H), 0.88 (t, *J =* 6.7 Hz, 6H, **H33 & H51)** ppm. **¹³C NMR (100 MHz, Chloroform-*d*)** δ 156.0 (**C**=O), 155.6 (Ar-**C**, quaternary), 152.4 (**C**=O), 125.5 (Ar**C**-H), 121.9 (Ar**C**-H), 67.0 **(C2 or C7),** 62.8 **(C2 or C7),** 47.8 **(C16 or C34),** 47.2 **(C16 or C34),** 38.2 (S**C**H₂), 36.9 (S**C**H₂), 32.1, 29.86, 29.83, 29.81, 29.77, 29.57, 29.51, 28.8 **(C17 or C35),** 28.3 **(C17 or C35),** 27.0, 22.8, 14.3 **(C33 & C51)** ppm. **LRMS (ESI) (m/z):** calculated for [M+H]⁺ (C₄₈H₈₇O₇N₂S₂) requires 867.6, found: 867.5.

### Synthesis of compound 5

To a stirred solution of compound **3** (1.0 equiv.) in CH₂Cl₂ at room temperature was added amine **4** (1.2 equiv.), followed by Et₃N (1.5 equiv.). The reaction mixture was vigorously stirred for 2 h at room temperature. The organic phase was first washed by sat. Na₂CO₃ (aq.) till its color turned to off white, then washed by brine, dried over Na₂SO₄, filtered and concentrated. The resulting residue was purified by silica gel column chromatography (hexane/ethyl acetate = 3:1 to 1:1, then changed to CH₂Cl₂ /CH₃OH = 15:1) to afford compound **5. The names of Compound 5 are given just below their structures.**

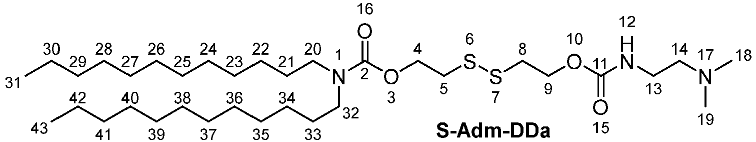

**Yield:** 75%. Colorless oil. **¹H NMR (400 MHz, Chloroform-*d*)** δ 5.48 (brd, 1H, **H12),** 4.34-4.29 (m, 4H, O**CH₂**), 3.30 (q, *J =* 5.8 Hz, 2H, H13), 3.21-3.14 (m, 4H, **H20 & H32),** 2.95-2.90 (m, 4H, **H5 & H8),** 2.48 (t, *J* = 6.2 Hz, 2H, **H14),** 2.28 (s, 6H, **H18 & H19**), 1.54-1.47 (m, 4H, **H21 & H33),** 1.26 (s, 36H), 0.88 (t, *J =* 6.7 Hz, 6H, **H31 & H43)** ppm. **¹³C NMR (100 MHz, Chloroform-*d*)** δ 156.0 **(C2 & C11),** 63.0 (**OCH₂**), 62.6 (**OCH₂**), 58.3 **(C14),** 47.8 **(C20 or C32),** 47.2 **(C20 or C32),** 45.2 **(C18 & C19),** 38.4 **(C13),** 38.2 **(C5 or C8),** 38.0 **(C5 or C8),** 32.1, 29.82, 29.79, 29.76, 29.6, 29.5, 28.8 **(C21 or C33),** 28.3 **(C21 or C33),** 27.0, 22.8, 14.3 **(C31 & C43)** ppm. **LRMS (ESI) (m/z):** calculated for [M+H]⁺ (C₃₄H₇₀O₄N₃S₂) requires 648.5, found: 648.4.

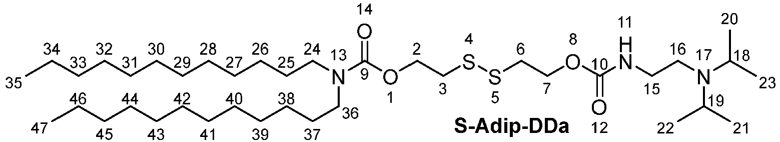

**Yield:** 55%. Colorless oil. **¹H NMR (400 MHz, Chloroform-*d*)** δ **¹H NMR (400 MHz, Chloroform-*d*)** δ 4.33-4.28 (m, 4H, **H2 & H7),** 3.60 (brd, 2H), 3.21-3.14 (brd, 4H, **H24 & H36),** 3.07 (brd, 2H), 2.94-2.88 (m, 4H, **H3 & H6),** 1.62-1.25 (m, 55H), 0.88 (t, *J =* 6.7 Hz, 6H, **H35 & H47)** ppm. **¹³C NMR (100 MHz, Chloroform-*d*)** δ 156.0 **(C9 & C10),** 63.0 **(C2 & C7),** 47.8 **(C24 or C36),** 47.2 **(C24 or C36),** 38.1 **(C3 & C6),** 32.1, 29.82, 29.79, 29.76, 29.6, 29.5, 28.8, 28.3, 27.0, 22.8, 14.3 **(C35 & C47)** ppm. **LRMS (ESI) (m/z):** calculated for [M+H]⁺ (C₃₃H₇₃O₄N₃S₂) requires 704.5, found: 704.5.

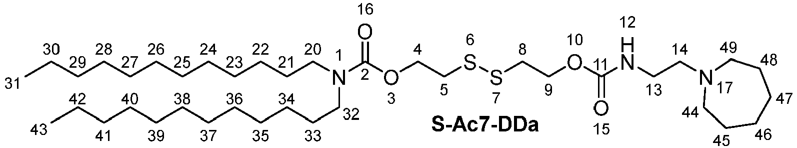

**Yield:** 51%. Colorless solid. **¹H NMR (400 MHz, Chloroform-*d*) δ** 4.34-4.29 (m, 4H, **H4 & H9),** 3.32(brd, 2H, **H13),** 3.21-3.14 (m, 4H, **H20 & H32),** 2.95-2.91 (m, 4H, **H5 & H8),** 2.77-2.64 (m, 6H, **H14, H44 & H49),** 1.71 (brd, 4H), 1.62 (brd, 4H), 1.51 (t, *J =* 7.3 Hz, 4H, **H21 & H33),** 1.26 (brd, 36H), 0.88 (t, *J* = 6.7 Hz, 6H, **H49 & H55)** ppm. **¹³C NMR (100 MHz, Chloroform-*d*)** δ 156.0 **(C2 & C11),** 63.0 (**OCH₂**), 62.7 (**OCH₂**), 56.8 **(C14),** 55.4 **(C44 & C49),** 47.8 **(C20 or C32),** 47.2 **(C20 or C32),** 38.1 **(C5 or C8 & C13),** 38.0 **(C5 or C8),** 32.1, 29.81, 29.79, 29.76, 29.57, 29.50, 28.8 **(C21 or C33),** 28.3 **(C21 or C33),** 27.1 **(C47 or C48),** 27.0 (C47 **or C48),** 22.8, 14.3 **(C31 & C43)** ppm. **LRMS (ESI) (m/z):** calculated for [M+H]⁺ (C₃₈H₇₆O₄N₃S₂) requires 702.5, found: 702.5.

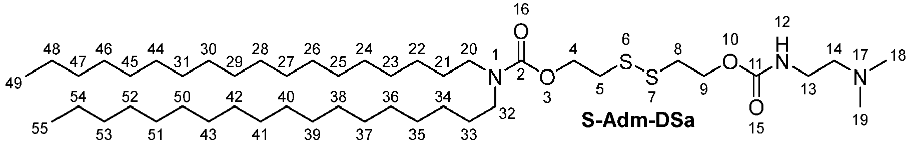

**Yield:** 81%. Colorless oil. **¹H NMR (400 MHz, Chloroform-*d*) δ** 5.48 (s, 1H, **H12),** 4.34-4.29 (m, 4H, **H4 & H9),** 3.30-3.28 (m, 2H, **H13),** 3.21 -3.14 (m, 4H, **H20 & H32),** 2.95-2.90 (m, 4H, **H5 & H8),** 2.48-2.46 (m, 2H, **H14),** 2.28 (s, 6H, **H18 & H19),** 1.53-1.49 (m, 4H, **H21 & H33),** 1.25 (brd, 60H), 0.88 (t, *J =* 6.7 Hz, 6H, **H49 & H55)** ppm. **¹³C NMR (100 MHz, Chloroform-*d*)** δ 156.0 **(C2 & C11),** 63.0 (**OCH₂**), 62.6 (**OCH₂**), 58.3 **(C14),** 47.8 **(C20 or C32),** 47.2 **(C20 or C32),** 45.2 **(C18 & C19),** 38.4 **(C13),** 38.2 **(C5 or C8),** 38.0 **(C5 or C8),** 32.1, 29.86, 29.83, 29.81, 29.77, 29.6, 29.5, 28.8 **(C21 or C33),** 28.3 **(C21 or C33),** 27.0, 22.8, 14.3 **(C49 & C55)** ppm. **LRMS (ESI)** (m/z): calculated for [M+H]⁺ (C₄₆H₉₄O₄N₃S₂) requires 816.7, found: 816.6.

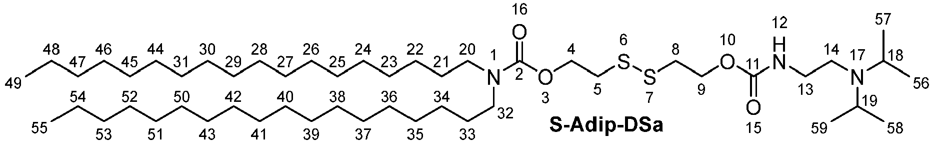

**Yield:** 89%. Colorless oil. **¹H NMR (400 MHz, Chloroform-*d*) δ** 5.30 (brd, 1H, **H12),** 4.34-4.28 (m, 4H, **H4 & H9),** 3.21- 3.11 (m, 6H, **H13, H20 & H32),** 3.02-2.96 (m, 2H, **H18 & H19),** 2.95-2.90 (m, 4H, **H5 & H8),** 2.56 (t, *J =* 6.2 Hz, 2H, **H14),** 1.54-1.47 (m, 4H, **H21 & H33),** 1.25 (brd, 60H), 1.00 (d, *J =* 6.6 Hz, 12H, **H56, H57, H58 & H59),** 0.88 (t, *J =* 6.7 Hz, 6H, **H49 & H55)** ppm. **¹³C NMR (100 MHz, Chloroform-*d*)** δ 156.0 **(C2 & C11),** 63.0 **(C4 or C9),** 62.6 **(C4 or C9),** 48.1 **(C18 & C19),** 47.8 **(C20 or C32),** 47.2 **(C20 or C32),** 43.8 **(C14),** 40.3 **(C13),** 38.2 **(C5 or C8),** 38.1 **(C5 or C8),** 32.1, 29.86, 29.83, 29.81, 29.78, 29.58, 29.52, 28.8 **(C21 or C33),** 28.3 **(C21 or C33),** 27.0, 22.8, 20.9 **(C56, C57, C58 & C59),** 14.3 **(C49 & C55)** ppm. **LRMS (ESI) (m/z):** calculated for [M+H]⁺ (C₅₀H₁₀₂O₄N₃S₂) requires 872.7, found: 872.6.

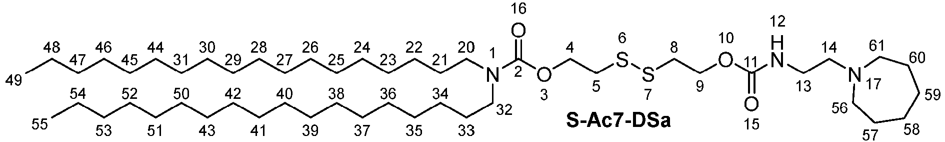

**Yield:** 70%. Colorless solid. **¹H NMR (400 MHz, Chloroform-*d*)** δ 4.34-4.29 (m, 4H, **H4 & H9),** 3.37 (brd, 2H, **H13),** 3.21-3.14 (m, 4H, **H20 & H32),** 2.95-2.91 (m, 4H, **H5 & H8),** 2.84-2.67 (m, 6H, **H14, H56 & H61),** 1.75 (brd, 4H), 1.64 (brd, 4H), 1.50 (q, *J =* 10.8, 6.5 Hz, 4H, **H21 & H33),** 1.25 (brd, 60H), 0.88 (t, *J =* 6.7 Hz, 6H, **H49 & H55)** ppm. **¹³C NMR (100 MHz, Chloroform-*d*)** δ 156.0 **(C2 & C11),** 63.0 (**OCH₂**), 62.8 (**OCH₂**), 56.9 **(C14),** 55.5 **(C56 & C61),** 47.8 **(C20 or C32),** 47.2 **(C20 or C32),** 38.1 **(C5 or C8 & C13),** 38.0 **(C5 or C8),** 32.1, 29.86, 29.83, 29.81, 29.78, 29.6, 29.5, 28.8 **(C21 or C33),** 28.3 **(C21 or C33),** 27.1 **(C60 or C59),** 27.0 **(C60 or C59),** 22.8, 14.3 **(C49 & C55)** ppm. **LRMS (ESI) (m/z):** calculated for [M+H]⁺ (C₅₀H₁₀₀O₄N₃S₂) requires 870.7, found: 870.7.

**2.2 The general route for the synthesis of lipids represented by the structure of formula I or more specifically IVb is shown below.**

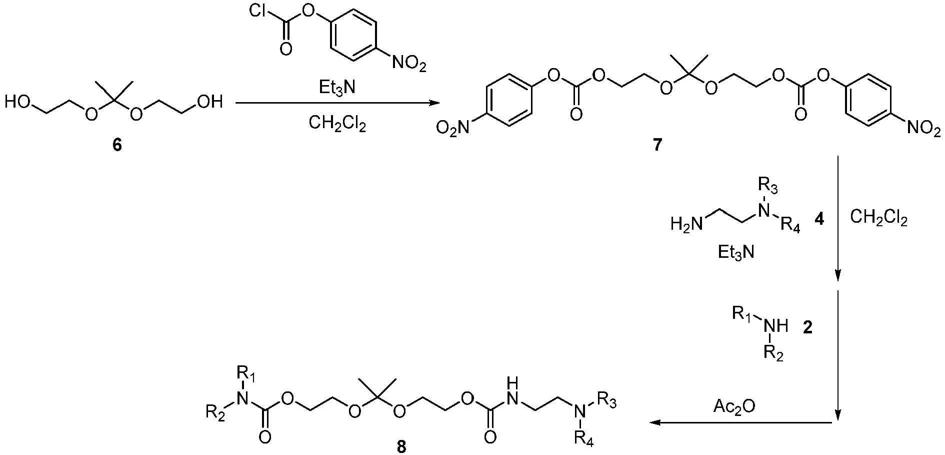

### Synthesis of compound 7

To a stirred solution of 4-nitrophenyl chloroformate (11.0 g, 54.5 mmol, 2.5 equiv.) in CH₂Cl₂ (80 mL) at 0 °C was dropwise added the solution of compound **6 (Shenoi et al., 2012)** (3.58 g, 21.8 mmol, 1.0 equiv.) and Et₃N (9.1 mL, 65.4 mmol, 3.0 equiv.) in CH₂Cl₂ (20 mL). The reaction mixture was vigorously stirred and allowed to warm to room temperature over 12 h. Then the reaction was quenched by sat. Na₂CO₃ (aq.). The organic phase was separated and washed by sat. Na₂CO₃ (aq.) till its color turned to offwhite, then washed by brine, dried over Na₂SO₄, filtered and concentrated to give compound **7** (6.00 g, 56% yield) as a yellowish solid which was used without further purification.

### Synthesis of compound 8

(1) To a stirred solution of compound **7** (3.0 equiv.) and Et₃N (3.0 equiv.) in CH₂Cl₂ at 0 °C was dropwise added amine **4** (1.0 equiv.). The reaction mixture was vigorously stirred at 0 °C for 5 h. Then the solvent was removed under reduced pressure. The residue was dissolved in a minimum amount of Et₂O and cooled at 0 °C. After 6 h, the excess compound 7 began to precipitate out from the solution. Then the solution was decanted and concentrated under reduced pressure. The resulting crude product was submitted to the above procedures two more times so that as much as compound **7** was precipitated out of the mixture. By this way, > 80% of compound **7** could be recycled and used.
(2) After 3 times precipitation, the above mixture was dissolved in CH₂Cl₂. To this solution, were added Et₃N (8.0 equiv.) and amine **2** pre-dissolved in CHCl₃/Hexane/THF (1.5 to 3.0 equiv. depending on the purity of the resulting mixture). The reaction mixture was vigorously stirred at room temperature for 5 h.
(3) Then Ac₂O (5.0 equiv.) was added to the mixture and the solution was further stirred at room temperature for another 5 h. Note: This step is to transform any excess amount of amine **2** to its acetyl amide, otherwise amine **2** will be very difficult to remove from the final product.
(4) Then the solvent was removed under reduced pressure, and redissolved in ethyl acetate. The organic phase was first washed by sat. Na₂CO₃ (aq.) till its color turned to off-white, then washed by brine, dried over Na₂SO₄, filtered and concentrated. The resulting residue was purified by silica gel column chromatography (CH₂Cl₂ /CH₃OH (+0.1% Et₃N) = 30:1 to 15:1) to afford compound **8. The names of Compound 8 are given just below their structures.**

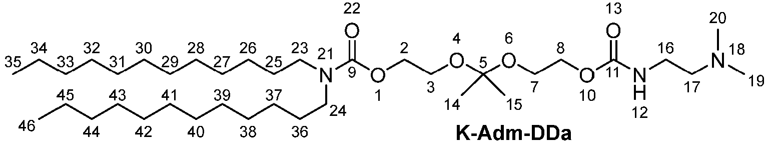

Yield: 28%. Colorless oil. ¹H NMR **(400 MHz, Chloroform-*d*)** δ 5.60 (s, 1H, **H12),** 4.20-4.15 (m, 4H, **H2 & H8),** 3.64-3.60 (m, 4H, **H3 & H7),** 3.28-3.23 (m, 2H, **H16),** 3.19-3.14 (m, 4H, **H23 & H24),** 2.42-2.39 (m, 2H, **H17),** 2.22 (s, 6H, **H19 & H20),** 1.53-1.47 (m, 4H, **H25 & H36),** 1.36 (s, 6H, **H14 & H15),** 1.26 (brd, 36H), 0.89-0.86 (t, 6H, **H35 & H46)** ppm. **¹³C NMR (100 MHz, Chloroform-*d*)** δ 156.5 **(C9 & C11),** 100.1 **(C5),** 64.5 **(C2 or C8),** 64.3 **(C2 or C8),** 59.6 **(C3 or C7),** 59.5 **(C3 or C7),** 58.5 **(C17),** 47.7 **(C23 or C24),** 47.1 **(C23 or C24),** 45.5 **(C19 & C20),** 38.7 **(C16),** 32.1, 29.80, 29.76, 29.61, 29.49, 28.8, 28.3, 27.0, 25.0 **(C14 & C15),** 22.8 **(C34 & C45),** 14.3 **(C35 & C46)** ppm. **LRMS (ESI) (m/z):** calculated for [M+H]⁺ (C₃₇H₇₆O₆N₃) requires 658.5, found: 658.5

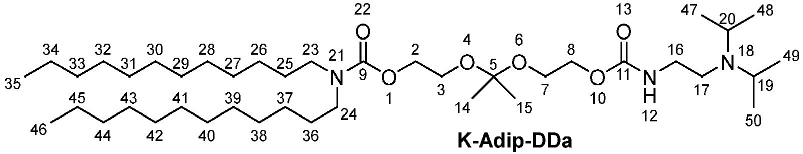

**Yield:** 21%. Colorless oil. **¹H NMR (400 MHz, Chloroform-*d*)** δ 5.53 (s, 1H, **H12),** 4.20-4.15 (m, 4H, **OCH₂),** 3.64-3.60 (m, 4H, OCH₂), 3.21-3.11 (m, 6H, **H23, H24 & H16),** 2.99 (p, *J =* 6.6 Hz, 2H, **H20 & H19),** 2.55 (t, *J* = 6.5 Hz, 2H, **H17),** 1.54-1.47 (m, 4H, **H25 & H36),** 1.36 (s, 6H, **H14 & H15),** 1.25 (brd, 36H), 0.99 (d, *J* = 6.6 Hz, 12H, **H47, H48, H49 & H50),** 0.88 (t, *J* = 6.7 Hz, 6H, **H49 & H55)** ppm. **¹³C NMR (100 MHz, Chloroform-*d*)** δ 156.4 **(C9 & C11),** 100.1 **(C5),** 64.5 (**OCH₂**), 64.2 (**OCH₂**), 59.5 (**OCH₂**), 48.3 **(C19 & C20),** 47.7 **(C23 or C24),** 47.1 **(C23 or C24),** 44.1 **(C17),** 40.7 **(C16),** 32.1, 29.81, 29.78, 29.62, 29.50, 28.8 **(C25 or C36),** 28.3 **(C25 or C36),** 27.0, 25.0 **(C14 & C15),** 22.8, 20.9 **(C47, C48, C49 & C50),** 14.3 **(C35 & C46)** ppm. **LRMS (ESI)** (m/z): calculated for [M+H]⁺ (C₄₁H₈₄O₆N₃) requires 714.6, found: 714.6.

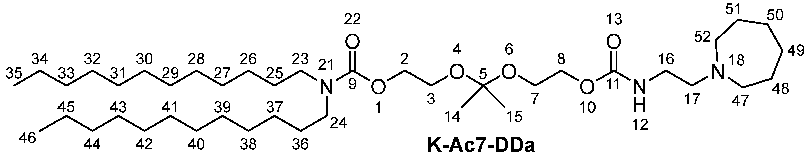

**Yield:** 25%. Colorless oil. **¹H NMR (400 MHz, Chloroform-*d*) δ** 5.64 (s, 1H, **H12),** 4.20-4.16 (m, 4H, **OCH₂),** 3.65-3.61 (m, 4H, **OCH₂),** 3.25-3.14 (m, 6H, **H23, H24 & H16),** 2.64 (brd, 6H, H17, **H47 & H52),** 1.59 (brd, 8H, **H48, H49, H50 & H51),** 1.54-1.47 (m, 4H, **H25 & H36),** 1.37 (s, 6H, **H14 & H15),** 1.25 (brd, 36H), 0.88 (t, *J* = 6.7 Hz, 6H, **H35 & H46)** ppm. **¹³C NMR (100 MHz, Chloroform-*d*)** δ 156.8 **(C9 or C11),** 156.4 **(C9 or C11),** 100.2 **(C5),** 64.5 (**OCH₂**), 64.2 (**OCH₂**), 59.6 (**OCH₂**), 56.8 **(C17),** 55.4 **(C47 & C52),** 47.8 **(C23 or C24),** 47.2 **(C23** or **C24),** 38.8 **(C16, deduced from HSQC),** 32.1, 29.82, 29.78, 29.6, 29.5, 28.8 **(C25 or C36),** 28.3 **(C25 or C36),** 27.1 **(C51 or C50),** 27.0 **(C51 or C50),** 25.0 **(C14 & C15),** 22.8, 14.3 **(C35 & C46)** ppm. **LRMS (ESI) (m/z):** calculated for [M+H]⁺ (C₄₁H₈₂O₆N₃) requires 712.6, found: 712.5.

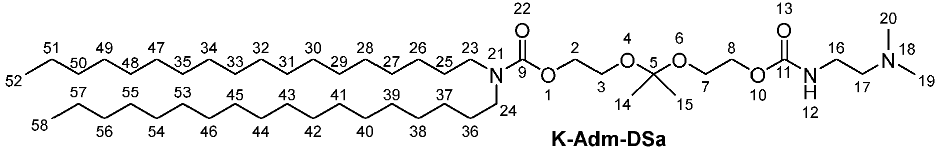

**Yield:** 21%. Colorless solid. **¹H NMR (400 MHz, Chloroform-*d*)** δ 5.60 (s, 1H, **H12),** 4.20-4.15 (m, 4H, **H2 & H8),** 3.64-3.60 (m, 4H, **H3 & H7),** 3.28-3.23 (m, 2H, **H16),** 3.19-3.14 (m, 4H, **H23 & H24),** 2.42-2.39 (m, 2H, **H17),** 2.22 (s, 6H, **H19 & H20),** 1.52-1.48 (m, 4H, **H25 & H36),** 1.36 (s, 6H, **H14 & H15),** 1.27 (brd, 60H), 0.89-0.86 (m, 6H, **H52 & H58)** ppm. **¹³C NMR (100 MHz, Chloroform-*d*) δ** 156.5 **(C9 & C11),** 100.1 **(C5),** 64.5 **(C2 or C8),** 64.3 **(C2 or C8),** 59.6 **(C3 or C7), 59.5 (C3 or C7),** 58.5 **(C17),** 47.7 **(C23 or C24),** 47.1 **(C23 or C24),** 45.5 **(C19 & C20),** 38.7 **(C16),** 32.1, 29.86, 29.83, 29.81, 29.78, 29.6, 29.5, 28.8, 28.3, 27.0, 25.0 **(C14 & C15),** 22.8 **(C51 & C57),** 14.3 **(C52 & C58)** ppm. **LRMS (ESI) (m/z):** calculated for [M+H]⁺ (C₄₉H₁₀₀O₆N₃) requires 826.7, found:826.7

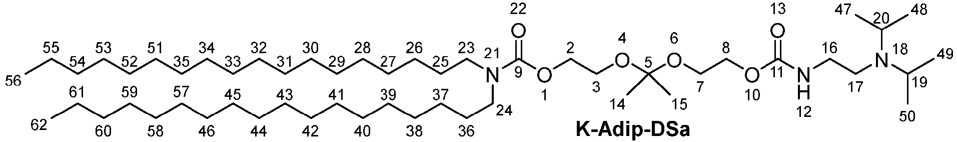

**Yield:** 38%. Colorless oil. **¹H NMR (400 MHz, Chloroform-*d*) δ** 5.52 (s, 1H, **H12),** 4.20-4.15 (m, 4H, **OCH₂),** 3.64-3.60 (m, 4H, **OCH₂),** 3.21-3.11 (m, 6H, **H23, H24 & H16),** 3.02-2.95 (m, 2H, **H20 & H19),** 2.55 (t, *J* = 6.6 Hz, 2H, **H17),** 1.54-1.47 (m, 4H, **H25 & H36),** 1.36 (s, 6H, **H14 & H15),** 1.25 (brd, 60H), 0.99 (d, *J =* 6.6 Hz, 12H, **H47, H48, H49 & H50),** 0.88 (t, *J =* 6.7 Hz, 6H, **H49 & H55)** ppm. **¹³C NMR (100 MHz, Chloroform-*d*) δ** 156.8 **(C9 or C11),** 156.4 **(C9 or C11),** 100.1 (C5), 64.5 (**OCH₂**), 64.2 (**OCH₂**), 59.5 (**OCH₂**), 48.3 **(C19 & C20),** 47.7 **(C23 or C24),** 47.1 **(C23 or C24),** 44.1 **(C17),** 40.7 **(C16),** 32.1, 29.85, 29.82, 29.80, 29.78, 29.6, 29.5, 28.8 **(C25 or C36),** 28.3 **(C25 or C36),** 27.0, 25.0 **(C14 & C15),** 22.8, 20.9 **(C47, C48, C49 & C50),** 14.3 **(C56 & C62)** ppm. **LRMS** (ESI) (m/z): calculated for [M+H]⁺ (C₅₃H₁₀₈O₆N₃) requires 882.8, found: 882.7.

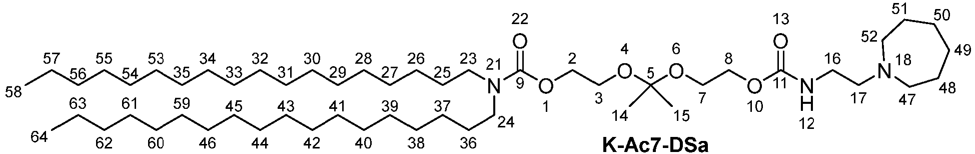

**Yield:** 22%. Colorless oil. **¹H NMR (400 MHz, Chloroform-d)** δ 5.59 (s, 1H, **H12),** 4.20-4.16 (m, 4H, **OCH₂),** 3.64-3.61 (m, 4H, **OCH₂),** 3.24-3.16 (m, 6H, **H23, H24 & H16),** 2.63-2.57 (m, 6H, **H17, H47 & H52),** 1.61-1.56 (m, 8H, **H48, H49, H50 & H51),** 1.54-1.47(m, 4H, **H25 & H36),** 1.37 (s, 6H, **H14 & H15),** 1.25 (brd, 60H), 0.87 (t, *J* = 6.8 Hz, 6H, **H58 & H64)** ppm. **¹³C NMR (100 MHz, Chloroform-*d*)** δ 156.7 **(C9 or C11),** 156.4 **(C9 or C11),** 100.2 **(C5),** 64.5 (**OCH₂**), 64.2 (**OCH₂**), 59.6 (**OCH₂**), 56.7 **(C17),** 55.4 **(C47 & C52),** 47.7 **(C23 or C24),** 47.1 **(C23 or C24),** 38.8 **(C16),** 32.1, 29.85, 29.82, 29.80, 29.78, 29.6, 29.5, 28.8 **(C25 or C36),** 28.4, 28.3 **(C25 or C36),** 27.1 **(C51 or C50),** 27.0 **(C51 or C50),** 25.0 **(C14 & C15),** 22.8, 14.3 **(C58 & C64)** ppm. **LRMS (ESI) (m/z):** calculated for [M+H]⁺ (C₅₃H₁₀₆O₆N₃) requires 880.8, found: 880.7.

**2.3 The general route for the synthesis of lipids represented by the structure of formula I, and more specifically IIIa is shown below.**

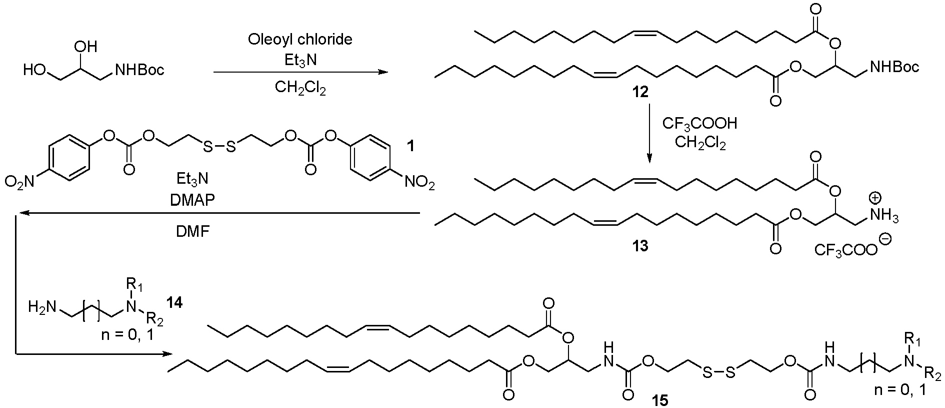

### Synthesis of compound 12

To a stirred solution of 3-(Boc-amino)-1,2-propanediol (2.43 g, 12.7 mmol, 1.0 equiv.) and oleoyl chloride (8.6 mL, 26.0 mmol, 2.05 equiv.) in CH₂Cl₂ (50 mL) at 0 °C was added dropwise Et₃N (5.3 mL, 38.1 mmol, 3.0 equiv.). The reaction mixture was then covered by aluminum foil and vigorously stirred at 0 °C for another 4 h. Then the reaction was quenched by sat. Na₂CO₃ (aq.). The organic phase was separated, washed by brine, dried over Na₂SO₄, filtered and concentrated. The resulting residue was purified by silica gel column chromatography (hexane/ethyl acetate = 15:1 to 10:1) to afford compound **12** as a colorless solid (4.06 g, 44%). The spectra data of **12** were in good agreement with data reported from the literature **(Luo et al., 2016).**

### Synthesis of compound 13

Compound **12** were then dissolved in the mixed solvent of CH₂Cl₂/CF₃COOH (10 mL/10 mL). After stirring at room temperature for 30 min, the solvent was removed under reduced pressure, and the crude product **13** was further dried under vacuum and used without further purification.

### Synthesis of compound 15

Compound **13** (1.0 equiv.), compound **1** (2.0 equiv.), DMAP (0.2 equiv.), and Et₃N (5.0 equiv.) were dissolved in DMF at room temperature. The resulting mixture was then covered by aluminum foil and further stirred at room temperature for 24 h before the amine **14** (2.5 equiv.) was added. After 5 h, the solvent DMF was removed under reduced pressure, and the crude residue was redissolved in ethyl acetate. The organic phase was first washed by sat. Na₂CO₃ (aq.) till its color turned to off-white, then washed by brine, dried over Na₂SO₄, filtered and concentrated. The resulting residue was purified by silica gel column chromatography (CH₂Cl₂ /CH₃OH = 30:1 to 15:1) to afford compound **15. The names of Compound 15 are given just below their structures.**

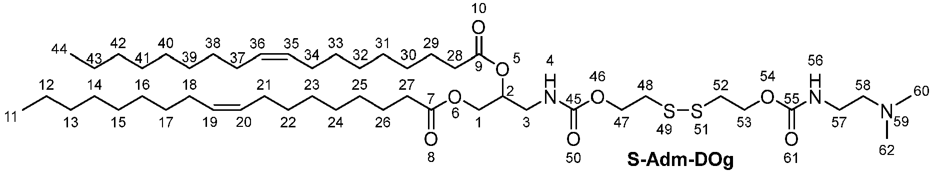

**Yield:** 30%. Colorless oil. **¹H NMR (400 MHz, Chloroform-*d*)** δ 5.50 (brd, 1H, **NH),** 5.37-5.32 (m, **5H,H19, H20, H35, H36 & NH),** 5.13-5.08 (m, 1H, **H2),** 4.33-4.26 (m, 5H, **H47, H53 & H1),** 4.13 (dd, *J =* 12.0, 5.7 Hz, 1H, **H1),** 3.48-3.34 (m, 2H, **H3),** 3.32-3.28 (m, 2H, **H57),** 2.92 (t, *J =* 6.4 Hz, 4H, **H48 & H52),** 2.50 (t, *J* = 5.9 Hz, 2H, **H58),** 2.33-2.27 (m, 10H, **H27, H28, H60 & H62),** 2.03-1.98 (m, 8H, **H18, H21, H34 & H37),** 1.63-1.58 (m, 4H, **H12 & H43),** 1.30-1.25 (m, 40H), 0.88 (t, *J* = 7.0 Hz, 6H, **H11 & H44)** ppm. **¹³C NMR (100 MHz, Chloroform-*d*)** δ 173.6, 173.2, 156.4, 156.3, 130.17, 130.16, 129.85, 129.83, 70.4, 62.8, 62.7, 58.3, 45.1, 41.4, 38.2, 38.0, 37.7, 34.4, 34.2, 32.0, 29.91, 29.86, 29.7, 29.47, 29.46, 29.35, 29.34, 29.3, 29.25, 29.23, 27.37, 27.32, 25.02, 24.99, 22.8, 14.3 **(C11 & C44)** ppm. **LRMS (ESI) (m/z):** calculated for [M+H]⁺ (C₄₉H₉₂O₈N₃S₂) requires 914.6, found: 914.5.

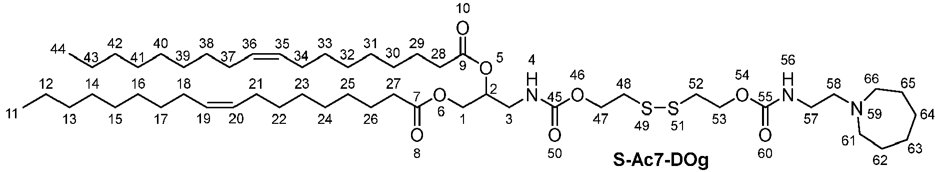

**Yield:** 30%. Colorless oil. **¹H NMR (400 MHz, Chloroform-*d*)** δ 5.79 (s, 1H, **NH),** 5.44-5.29 (m, 5H, **H19, H20, H35, H36 and NH),** 5.13-5.08 (m, 1H, **H2),** 4.33-4.26 (m, 5H, **H47, H53 and H1),** 4.13 (dd, *J* = 12.0, 5.7 Hz, 1H, **H1),** 3.48-3.34 (m, 4H, **H3, H57),** 2.94-2.91 (m, 4H, **H61, H66),** 2.81-2.66 (m, 6H, **including H48, H52, H58),** 2.31 (td, *J* = 7.6, 2.7 Hz, 4H, **H27, H28),** 2.05-1.96 (m, 8H, **H18, H21, H34, H37),** 1.73 (brd, 4H, **H26, H29),** 1.64-1.56 (m, 8H, **H62-65),** 1.34-1.25 (m, 40H), 0.87 (t, *J =* 7.0 Hz, 6H, **H11 & H44)** ppm. **¹³C NMR (100 MHz, Chloroform-d) δ** 173.6 **(ester C=O),** 173.2 **(ester C=O),** 156.4 **(carbamate C=O),** 156.3 **(carbamate C=O),** 130.2 **(alkene carbon),** 129.84 **(alkene carbon),** 129.83 **(alkene carbon),** 70.4 **(C2),** 63.0 **(C1),** 62.7, 56.8, 55.4 , 41.4 **(C3),** 38.0 **(C57),** 37.6 , 34.4, 34.2, 32.0, 29.90, 29.86, 29.66, 29.47, 29.45, 29.35, 29.27, 29.25, 29.23, 27.36, 27.32, 27.1, 25.02, 24.99, 22.82, 14.3 **(C11 & C44)** ppm. **LRMS (ESI) (m/z):** calculated for [M+H]⁺ (C₅₃H₉₈O₈N₃S₂) requires 968.7, found: 968.6.

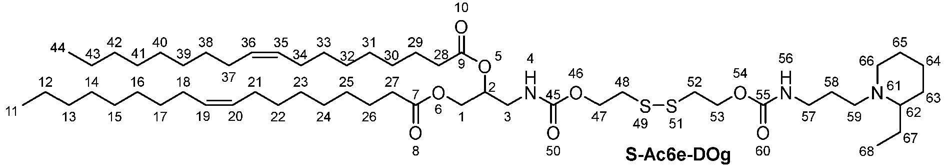

**Yield:** 24%. Colorless oil. **¹H NMR (400 MHz, Chloroform-d)** δ 6.02 (s, 1H, **NH),** 5.43-5.29 (m, 5H, **H19, H20, H35, H36 and NH),** 5.13-5.08 (m, 1H, **H2),** 4.40-4.26 (m, 5H, **H47, H53 and H1),** 4.13 (dd, *J* = 12.0, 5.7 Hz, 1H, **H1),** 3.48-3.35 (m, 2H, **H3),** 3.33-3.28 (m, 2H), 3.11 (brd, 2H), 2.96-2.88 (m, 6H), 2.33-2.29 (m, 4H, **H27, H28),** 2.05-1.96 (m, 8H, **H18, H21, H34, H37),** 1.85-1.51 (m, 14H), 1.32-1.25 (m, 40H), 0.98 (t, *J =* 7.4 Hz, 3H, **H68),** 0.88 (t, *J =* 7.0 Hz, 6H, **H11 & H44)** ppm. **¹³C NMR (100 MHz, Chloroform-d)** δ 173.6, 173.3, 156.7, 156.4, 130.2, 129.85, 129.83, 70.4 **(C2),** 62.9 **(C1),** 62.78, 62.74, 41.4 **(C3),** 37.9, 37.8, 34.4, 34.2, 32.0, 29.91, 29.87, 29.86, 29.7, 29.47, 29.46, 29.37, 29.35, 29.29, 29.28, 29.25, 29.23, 27.37, 27.32, 25.03, 25.00, 22.8, 14.3 **(C11, C44),** 10.4 **(C68). (C62 was not observed on ¹³C APT due to signal cancellation). LRMS (ESI) (m/z):** calculated for [M+H]⁺ (C₅₅H₁₀₂O₈N₃S₂) requires 996.7, found: 996.6.

**2.4 The general route for the synthesis of lipids represented by the structure of formula I, or more specifically IIIb is shown below.**

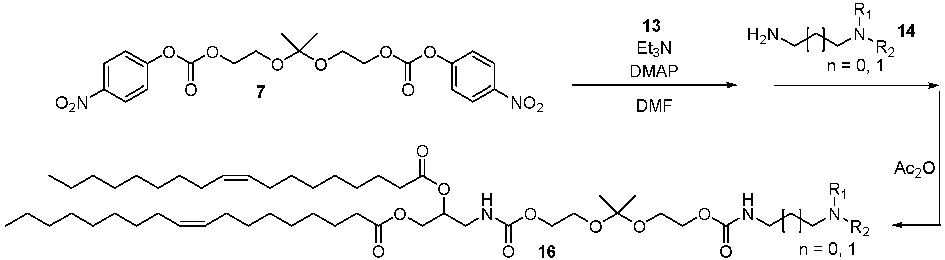

Compound **13** (1.0 equiv.), compound **7** (2.0 equiv.), DMAP (0.2 equiv.), and Et₃N (10.0 equiv.) were dissolved in DMF at room temperature. The resulting mixture was then covered by aluminum foil and further stirred at room temperature for 24 h before the amine **14** (2.5 equiv.) was added. After 5 h, Ac₂O (3.0 equiv.) was added, and the mixture was further stirred at room temperature for another 12 h. Note: Ac₂O was added to facilitate removing unidentified impurities from the final product. Then the solvent DMF was removed under reduced pressure, and the crude residue was redissolved in ethyl acetate. The organic phase was first washed by sat. Na₂CO₃ (aq.) till its color turned to off-white, then washed by brine, dried over Na₂SO₄, filtered and concentrated. The resulting residue was purified by silica gel column chromatography (CH₂Cl₂ /CH₃OH (+ 0.1% Et₃N) = 30:1 to 15:1) to afford compound **16. The names of Compound 16 are given just below their structures.**

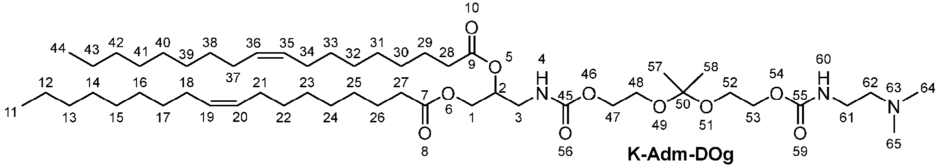

**Yield:** 20%. Colorless oil. **¹H NMR (400 MHz, Chloroform-d)** δ 5.72 (t, *J* = 5.4 Hz, 1H, **NH),** 5.47 (s, 1H, **NH),** 5.40-5.30 (m, 4H, **H19, H20, H35, H36),** 5.11-5.07 (m, 1H, **H2),** 4.32-4.08 (m, 6H, **Including H1, H47, H53),** 4.37-3.61 (m, 4H, **H48, H52),** 3.48-3.22 (m, 4H, **H3 & H61),** 2.41 (t, *J* = 5.9 Hz, 2H, **H62),** 2.33-2.23 (m, 10H, **H27, H28, H64, H65),** 2.04-1.96 (m, 8H, **H18, H21, H34, H37),** 1.62-1.58 (m, 4H, **H26 & H29),** 1.36 (s, 6H, **H57 & H58)** 1.34-1.25 (m, 40H), 0.88 (t, *J =* 7.0 Hz, 6H, **H11 & H44)** ppm. **¹³C NMR (100 MHz, Chloroform-d)** δ 173.5 **(ester C=O),** 173.2 **(ester C=O),** 156.89 **(carbamate C=O), 156.86(carbamate C=O),** 130.2 **(alkene carbon),** 129.86 **(alkene carbon),** 129.84 **(alkene carbon),** 100.0 **(C50),** 70.6 **(C2),** 67.4, 67.3, 64.5 **(C47 or C53), 64.2(C47 or C53),** 62.8 **(C1),** 59.5 **(C48 or C52),** 59.3 **(C48 or C52),** 58.3 **(C62),** 45.2 **(C64, C65),** 41.3 **(C3),** 38.4 **(C61),** 34.4 **(C27 or C28),** 34.2 **(C27 or C28),** 32.0, 29.92, 29.88, 29.87, 29.81, 29.7, 29.47, 29.46, 29.37, 29.35, 29.29, 29.26, 29.25, 27.37 **(allylic carbon),** 27.33 **(allylic carbon),** 25.00, 24.94 **(C58, C59),** 22.8, 14.3 **(C11, C44)** ppm. **LRMS (ESI) (m/z):** calculated for [M+H]⁺ (C₅₂H₉₈O₁₀N₃) requires 924.7, found: 924.6.

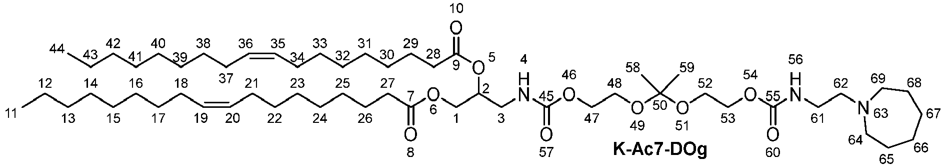

**Yield:** 18%. Colorless oil. **¹H NMR (400 MHz, Chloroform-*d*) δ** 5.79-5.77 (m, 1H, **NH),** 5.38-5.31 (m, 4H, **H19, H20, H35, H36),** 5.13-5.09 (m, 1H, **H2),** 4.32-4.10 (m, 6H, **Including H1, H47, H53),** 3.69-3.60 (m, 4H, **H48 & H52),** 3.52-3.43 (m, 1H, **H3),** 3.39-3.27 (m, 3H, **H3 & H61),** 2.77-2.70 (m, 6H, **H62, H64, H69),** 2.33-2.28 (m, 4H, **H27 & H28),** 2.04-1.96 (m, 8H, **H18, H21, H34, H37),** 1.70-1.58 (m, 12H, **H65-68 & H26, H29),** 1.36 (s, 6H, **H58 & H59),** 1.34-1.24 (m, 40H), 0.89-0.86 (m, 6H, **H11 & H44)** ppm. **¹³C NMR (100 MHz, Chloroform-*d*)** δ 173.5 **(ester C=O),173.2 (ester C=O),** 156.9 **(carbamate C=O**), 130.2 **(alkene carbon),** 129.85 **(alkene carbon),** 129.84 **(alkene carbon),** 100.0 **(C50),** 70.6 **(C2),** 67.6, 67.5, 67.4, 64.5, 64.2, 62.9 **(C1),** 59.5, 59.3, 56.5 **(C62),** 55.38, 55.35, 41.3 **(C3),** 39.6, 36.6, 34.4 **(C27 or C28),** 34.2 **(C27 or C28),** 32.0, 29.91, 29.88, 29.87, 29.85, 29.7, 29.47, 29.46, 29.37, 29.35, 29.33, 29.30, 29.28, 29.25, 27.37 **(allylic carbon),** 27.33 **(allylic carbon),** 27.1, 25.00, 24.94 **(C58 & C59),** 22.8, 14.3 **(C11 & C44)** ppm. **LRMS (ESI) (m/z):** calculated for [M+H]⁺ (C₅₆H₁₀₄O₁₀N₃) requires 978.8, found: 978.7.

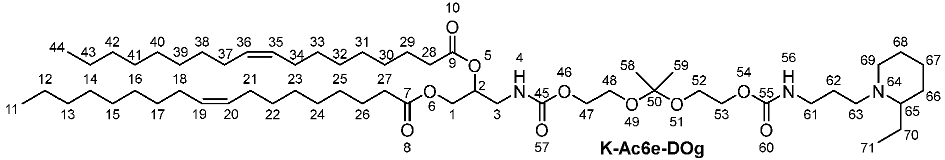

**Yield:** 15%. Colorless oil. **¹H NMR (400 MHz, Chloroform-*d*)** δ 6.05 (s, 1H, **NH),** 5.73 (s, 1H, **NH),** 5.38-5.31 (m, 4H, **H19, H20, H35, H36),** 5.11-5.08 (m, 1H, **H2),** 4.31-4.22 (m, 4H), 4.19-4.10 (m, 4H), 3.69-3.60 (m, 5H), 3.48-3.33 (m, 2H), 3.30-3.25 (m, 2H), 2.88-2.71 (m, 2H), 2.33-2.28 (m, 4H), 2.20-1.97 (m, 8H, **H18, H21, H34, H37),** 1.83-1.56 (m, 14H, **H26, H29, H62, H66, H67, H68, H70),** 1.35 (s, 6H, **H58, H59),** 1.29-1.26 (m, 40H), 0.96 (t, *J =* 7.4 Hz, 3H, **H71),** 0.87 (t, *J* = 7.0 Hz, 6H, **H11 & H44). ¹³C NMR (100 MHz, Chloroform-*d*)** δ 173.6, 173.3, 157.2, 156.9, 130.2, 129.84, 129.83, 100.3 **(C50),** 100.0 **(C50),** 70.5 **(C2),** 67.4, 64.6, 64.5, 64.2, 62.9, 62.8, 59.4, 59.2, 58.97, 58.92, 58.85, 58.80, 41.32 **(C3),** 41.27 **(C3),** 34.4 **(C27** or **C28),** 34.2 **(C27 or C28),** 32.0, 29.90, 29.86, 29.7, 29.46, 29.45, 29.37, 29.35, 29.28, 29.24, 27.36 **(allylic carbon),** 27.32 **(allylic carbon),** 25.01, 24.99, 24.92 **(C58, C59),** 24.89 **(C58, C59),** 22.8, 14.3 **(C11 & C44),** 10.4 **(C71). LRMS (ESI) (m/z):** calculated for [M+H]⁺ (C₅₈H₁₀₈O₁₀N₃) requires 1006.8, found: 1006.7.

**2.5 The general route for the synthesis of lipids represented by the structure of formula I or more specifically IIIc is shown below.**

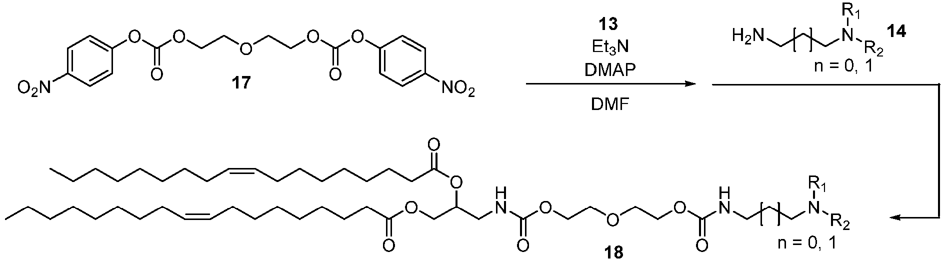

Compound **13** (1.0 equiv.), compound **17 (Yaeger et al., 2004)** (2.0 equiv.), DMAP (0.2 equiv.) and Et₃N (5.0 equiv.) were dissolved in DMF at room temperature. The resulting mixture was then covered by aluminum foil and further stirred at room temperature for 24 h before the amine **14** (2.5 equiv.) was added. Then the solvent DMF was removed under reduced pressure, and the crude residue was redissolved in ethyl acetate. The organic phase was first washed by sat. Na₂CO₃ (aq.) till its color turned to off-white, then washed by brine, dried over Na₂SO₄, filtered and concentrated. The resulting residue was purified by silica gel column chromatography (CH₂Cl₂ /CH₃OH = 30:1 to 15:1) to afford compound **18. The names of Compound 18 are given just below their structures.**

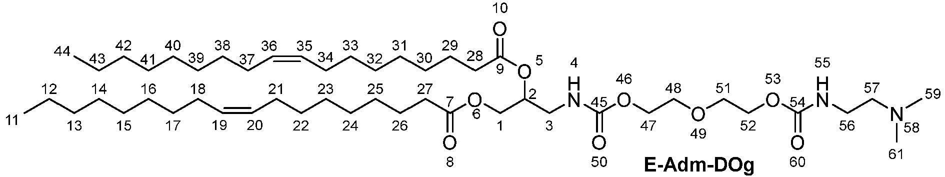

**Yield:** 24%. Colorless oil. **¹H NMR (400 MHz, Chloroform-*d*)** δ 5.41-5.32 (m, 5H, **H19, H20, H35, H36 & NH),** 5.17 (t, *J =* 6.2 Hz, 1H, NH), 5.12-5.07 (m, 1H, **H2),** 4.27 (dd, *J =* 12.0, 4.4 Hz, 1H, **H1),** 4.22 (t, *J* = 4.7 Hz, 4H, **H47 & H52),** 4.12 (dd, *J =* 12.0, 5.7 Hz, 1H, H1), 3.67 (t, *J* = 4.7 Hz, 4H, **H48 & H51),** 3.48-3.34 (m, 2H, **H3),** 3.30-3.25 (m, 2H, **H56),** 2.44 (t, *J* = 6.1 Hz, 2H, **H57),** 2.31 (td, *J =* 7.6, 2.3 Hz, 4H, **H27 & H28),** 2.25 (s, 6H, **H59 & H61),** 2.00 (q, *J =* 6.5 Hz, 8H, **H18, H21, H34 & H37),** 1.63-1.58 (m, 4H, **H12 & H43),** 1.32-1.25 (m, 40H), 0.88 (t, *J* = 7.0 Hz, 6H, **H11 & H44)** ppm. **¹³C NMR (100 MHz, Chloroform*-*d)** δ 173.6, 173.2, 156.6, 156.5, 130.2, 129.85, 129.84, 70.4, 69.7, 69.6, 64.3, 63.9, 62.7, 58.3, 45.2, 41.4, 38.4, 34.4, 34.2, 32.0, 29.91, 29.86, 29.7, 29.5, 29.35, 29.33, 29.27, 29.25, 29.23, 27.36, 27.32, 25.01, 24.99, 22.8, 14.3. **LRMS (ESI) (m/z):** calculated for [M+H]⁺ (C₄₉H₉₂O₉N₃) requires 866.7, found: 866.6.

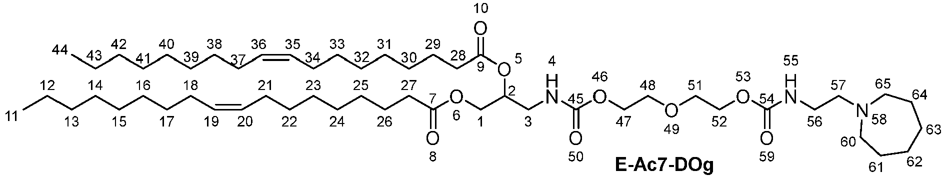

**Yield:** 22%. Colorless oil. **¹H NMR (400 MHz, Chloroform-*d*)** δ 5.66 (s, 1H, **NH**), 5.38-5.29 (m, 4H, **H19, H20, H35, H36**), 5.19 (s, 1H, **NH**), 5.12-5.07 (m, 1H, **H2**), 4.29-4.21 (m, 5H, **including H1, H47, H52**), 4.12 (dd, *J* = 12.0, 5.7 Hz, 1H, **H1**), 3.69-3.67 (m, 4H, **H48, H51**), 3.48-3.34 (m, 2H, **H3**), 3.33-3.29 (m, 2H, **H56**), 2.78-2.69 (m, 6H, **H57, H60, H65**), 2.31 (td, *J* = 7.6, 2.5 Hz, 4H, **H27, H28**), 2.03-1.98 (m, 8H, **H18, H21, H34, H37**), 1.69 (brd, 4H, **H26, H29**) 1.61 (brd, 8H, **H62-65**), 1.34-1.25 (m, **40H**), 0.87 (t, *J* = 7.0 Hz, 6H, **H11 & H44**) ppm. **¹³C NMR** (**100 MHz, Chloroform-*d***) δ 173.6 (**ester C=O**), 173.2 (**ester C=O**), 156.6 (**carbamate C=O**), 156.5 (**carbamate C=O**), 130.2 (**alkene carbon**), 129.85 (**alkene carbon**), 129.83 (**alkene carbon**), 70.4 (**C2**), 69.7 **(C48 or C51),** 69.6 **(C48 or C51),** 64.3 (**C47 or C52**), 64.0 (**C47 or C52**), 62.7 (**C1**), 56.7 (**C57**), 55.4 (**C60, C65**), 41.4 (**C3**), 38.2 (**C56**), 34.4 (**C27 or C28**), 34.2 (**C27 or C28**), 32.0, 29.90, 29.86, 29.7, 29.46, 29.45, 29.35, 29.33, 29.27, 29.24, 29.22, 27.36 (**allylic carbon**), 27.32 (**allylic carbon**), 27.1, 25.00, 24.98, 22.8, 14.3 (**C11 & C44**) ppm. **LRMS (ESI) (m/z):** calculated for [M+H]⁺ (C₅₃H₉₈O₉N₃) requires 920.7, found: 920.6.

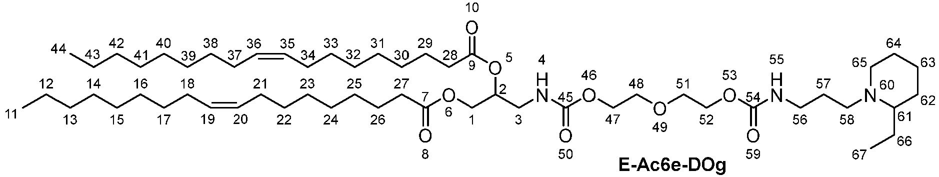

**Yield:** 16%. Colorless oil. **¹H NMR (400 MHz, Chloroform-*d*)** δ 5.94 (s, 1H, **NH**), 5.39-5.29 (m, 4H, **H19, H20, H35, H36**), 5.23 (s, 1H, **NH**), 5.10 (p, *J =* 5.2 Hz, 1H, **H2**), 4.29-4.19 (m, 5H, **including H1, H47, H52**), 4.12 (dd, *J =* 12.0, 5.7 Hz, 1H, **H1**), 3.68-3.65 (m, 4H, **H48, H51**), 3.48-3.34 (m, 2H, **H3**), 3.30-3.23 (m, 2H, **H56**), 2.95 (brd, 2H, **H58 or H65 or H61**), 2.58-2.47 (m, 3H, **H58 or H65 or H61**), 2.31 (td, *J* = 7.6, 2.7 Hz, 4H, **H27, H28**), 2.03-1.98 (m, 8H, **H18, H21, H34, H37**), 1.81-1.54 (m, 14H, **H26, H29, H57, H62, H63, H64, H66**), 1.29-1.25 (m, 40H), 0.93-0.86 (m, 9H, **H11, H44, H67**) ppm. **¹³C NMR (100 MHz, Chloroform-*d*)** δ 173.6 (**ester C=O**), 173.3 (**ester C=O**), 156.8 (**carbamate C=O**), 156.2 (**carbamate C=O**), 70.5 (**C2**), 69.66 (**C48 or C51**), 69.60 (**C48 or C51**), 64.3 (**C47 or C52**), 63.9 (**C47 or C52**), 62.7 (**C1**), 51.0 (**C58, C61, C65. The peak is weak due to the cancellation of the signals**), 41.4 (**C3**), 34.4 (**C27 or C28**), 34.2 (**C27 or C28**), 32.0, 29.90, 29.86, 29.7, 29.46, 29.45, 29.35, 29.33, 29.27, 29.24, 29.22, 27.36 (**allylic carbon**), 27.31 (**allylic carbon**), 25.01, 24.98, 22.8, 14.3 (**C11 & C44**), 10.3 (**C67**) ppm. **LRMS (ESI) (m/z):** calculated for [M+H]⁺ (C₅₅H₁₀₂O₉N₃) requires 948.8, found: 948.7.

**2.5 The general route for the synthesis of lipids represented by the structure of formula I or more specifically V is shown below.**

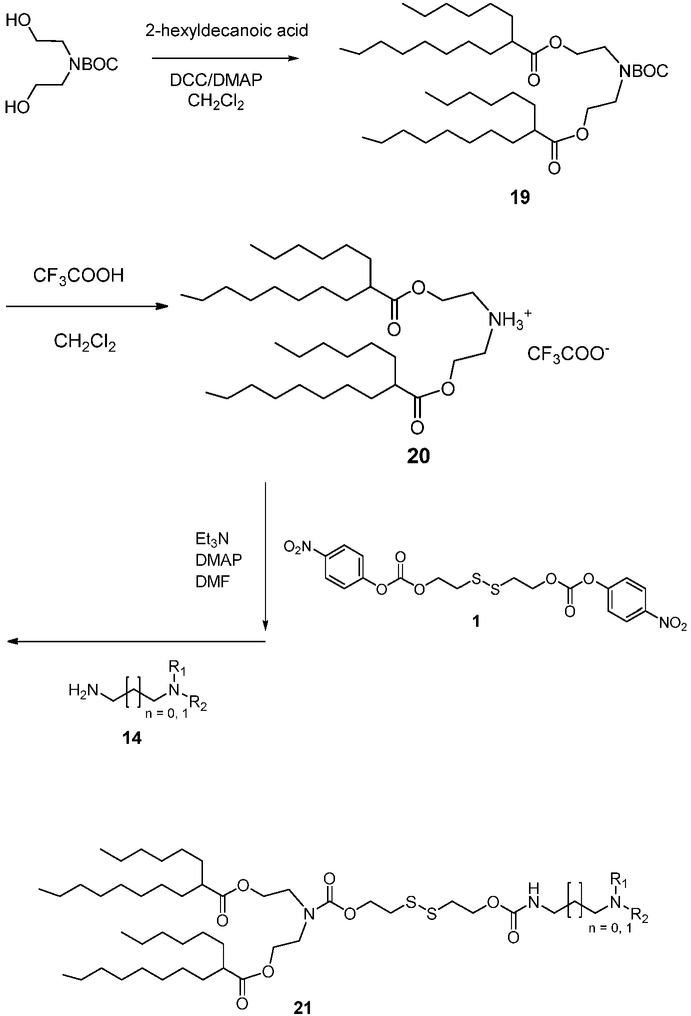

### Synthesis of compound 19

To a stirred solution of N-BOC-diethanolamine (5.43 g, 0.03 mol) and 2-hexyldecanoic acid (16.3 g, 0.06 mol) in CH₂Cl₂ (30 mL) at 0°C were added N-(3-dimethylaminopropyl)-N'-ethylcarbodiimide hydrochloride (13.1 g, 0.06 mol), TEA (6.42 g, 0.06 mol) and DMAP (0.66 mg, 0.005 mol). The reaction mixture was then covered by aluminum foil and vigorously stirred at 0 °C for 1h. The reaction was allowed to stir at room temperature for 48 hours. Then the reaction mixture was filtered and concentrated. The resulting residue was purified by silica gel column chromatography (hexane/ethyl acetate = 15:1) to afford **compound 19** as a brown oil (2.84 g, 17.00 %).

### Synthesis of compound 20

Compound **19** were then dissolved in the mixed solvent of CH₂Cl₂/CF₃COOH (10 mL/10 mL). After stirring at room temperature for 30 min, the solvent was removed under reduced pressure, and the crude product **20** was further dried under vacuum and used without further purification.

### Synthesis of compound 21

Compound **20** (1.0 equiv.), compound **1** (2.0 equiv.), DMAP (0.2 equiv.) and Et₃N (5.0 equiv.) were dissolved in DMF at room temperature. The resulting mixture was then covered by aluminum foil and further stirred at room temperature for 24 h before the amine **14** (2.5 equiv.) was added. After 5 h, the solvent DMF was removed under reduced pressure, and the crude residue was redissolved in ethyl acetate. The organic phase was first washed by sat. Na₂CO₃ (aq.) till its color turned to off-white, then washed by brine, dried over Na₂SO₄, filtered and concentrated. The resulting residue was purified by silica gel column chromatography (CH₂Cl₂ /CH₃OH = 30:1 to 10:1) to afford compound **21. The names of Compound 21 are given just below their structures.**

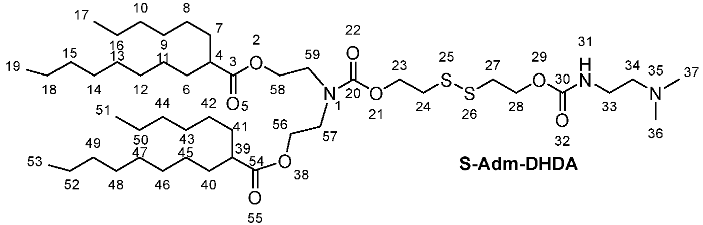

**Yield:** 16%. Colorless oil. **¹H NMR (400 MHz, Chloroform-*d***) 4.39-4.28 (m, 4H, **H56 & H58**), 4.23-4.16 (m, 4H, **H23 & H28**), 3.58-3.51 (m, 4H, **H57 & H59**), 3.38-3.29 (m, 2H, **H33**), 2.99-2.88 (m, 4H, **H24 & H27**), 2.61-2.51 (m, 2H, **H34**), 2.41-2.27 (m, 8H, **H4, H36, H37 & H39**), 1.64-1.38 (m, 8H, **H6, H7, H40 & H41**), 1.32-1.20 (brd, 40H), 0.87 (t, *J =* 6.8 Hz, 12H, **H17,H19,H51 & H53**) ppm. **LRMS (ESI) (m/z):** calculated for [M+H]⁺ (C₄₈H₈₉O₈N₃S₂) requires 876.6, found: 876.5.

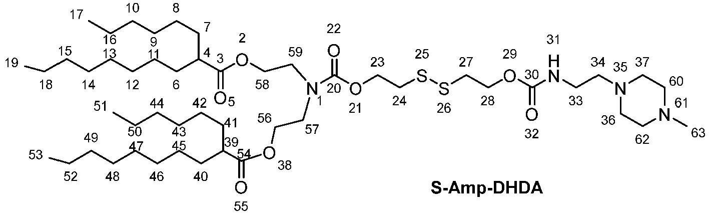

**Yield:** 28%. Colorless oil. **¹H NMR (400 MHz, Chloroform-*d*)** 4.39-4.28 (m, 4H, **H56 & H58**), 4.23-4.15 (m, 4H, **H23 & H28**), 3.55 (m, 4H, **H57 & H59**), 3.33-3.24 (m, 2H, **H33**), 2.98-2.89 (m, 4H, **H24 & H27**), 3.63- 2.54 (brd, 8H, **H36, H37, H60, H62**) 2.51 (t, 2H, *J* = 6.0 Hz, **H34**), 2.36 (s, 3H, **H63**) 2.34-2.28 (m, 2H, **H4 & H39**), 1.65-1.37 (m, 8H, **H6, H7, H40 & H41**), 1.33-1.20 (brd, 40H), 0.87 (t, *J* = 6.8 Hz, 12H, **H17,H19,H51 & H53**) ppm. **LRMS (ESI)** (m/z): calculated for [M+H]⁺ (C₄₉H₉₄O₈N₄S₂) requires 931.7, found: 931.5.

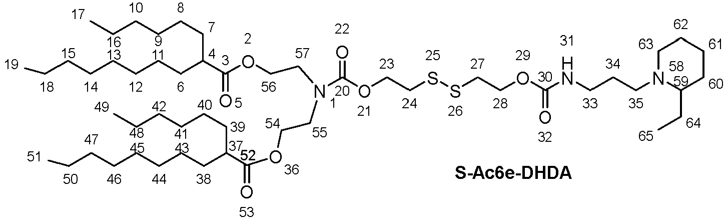

**Yield:** 20%. Colorless oil. **¹H NMR (400 MHz, Chloroform-*d*)** 4.40-4.27 (m, 4H, **H54 & H56**), 4.24-4.16 (m, 4H, **H23 & H28**), 3.55 (m, 4H, **H55 & H57**), 3.38-3.28 (m, 2H, **H33**), 3.03-2.89 (m, 6H, **H24, H27 & H63**), 3.03-2.28 (m, 2H, **H4 & H37**), 2.18-1.68 (m, 10H, **H34, H60, H61, H62 & H64**) 1.65-1.38 (m, 8H, **H6, H7, H40 & H41**), 1.25 (brd, 40H), 1.01 (t, *J* = 7.4 Hz, 3H, **H65**) 0.88 (t, *J* = 6.8 Hz, 12H, **H17, H19, H51 & H53**) ppm. **LRMS (ESI) (m/z):** calculated for [M+H]⁺ (C₅₂H₉₉O₈N₃S₂) requires 958,7, found: 958.7.

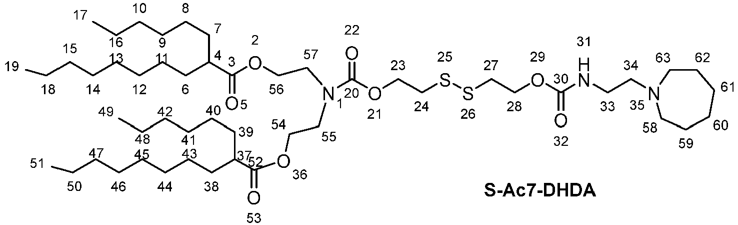

**Yield:** 68%. Colorless oil. **¹H NMR (400 MHz, Chloroform-*d*)** 4.39-4.25 (m, 4H, **H54,H56**), 4.25-4.15 (m, 4H, **H23,H28**), 3.58-3.51 (m, 6H, **H33,H55 & H57**), 2.99-2.88 (m, 8H, **H24, H27, H58 & H63**), 2.40-2.26 (m, 2H, **H4 & H37**), 2.00-1.64 (m, 10H, **H34, H59, H60, H61 & H62**), 1.63-1.38 (m, 8H, **H6, H7, H38 & H39**), 1.32-1.20 (brd, 40H), 0.87 (t, *J* = 6.8 Hz, 12H, **H17,H19, H49 & H51**) ppm. **LRMS (ESI) (m/z):** calculated for [M+H]⁺ (C₅₀H₉₅O₈N₃S₂) requires 930.7, found: 930.6

**2.6 The general route for the synthesis of lipids represented by the structure of formula I or more specifically VIa is shown below.**

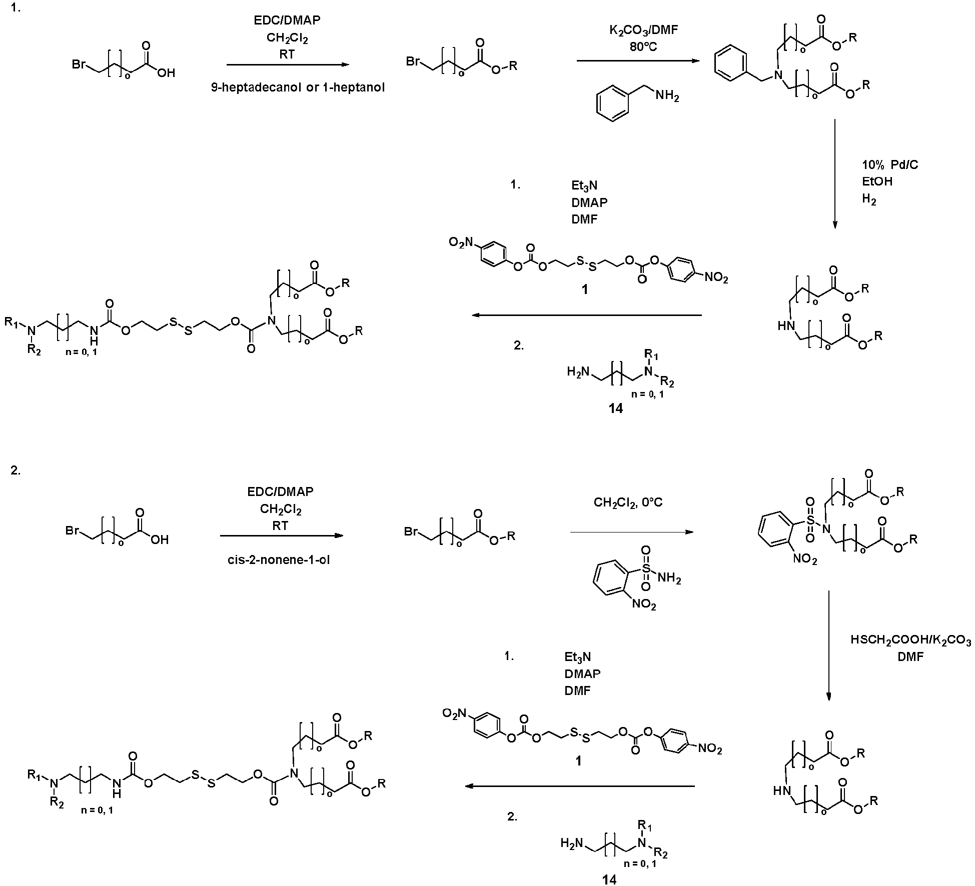

**2.7 The general route for the synthesis of lipids represented by the structure of formula I or more specifically VIb is shown below.**

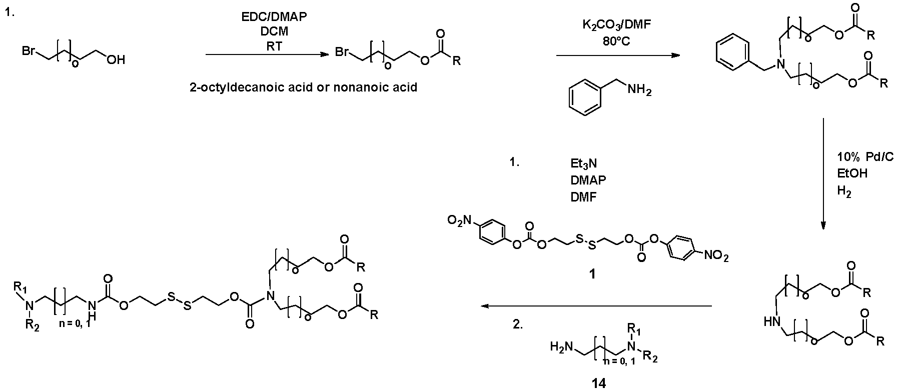

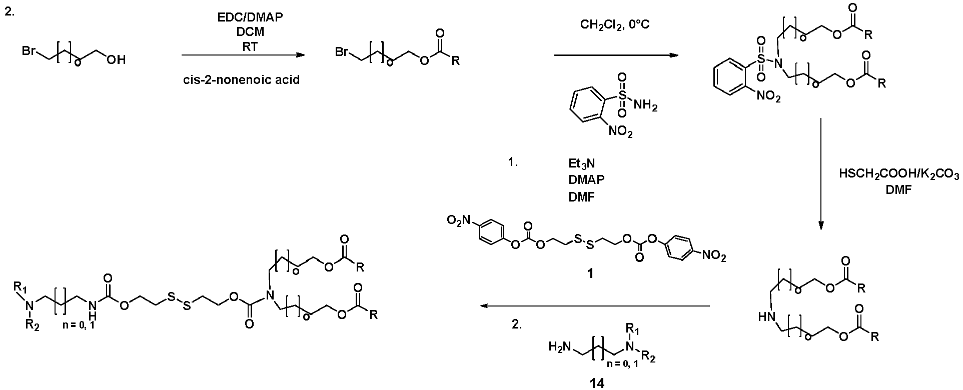

### EXAMPLE 2: IN VITRO EXPERIMENTS

### Materials and methods:

### mRNA synthesis:

mRNAs encoding eGFP and FireFly luciferase were prepared in vitro by T7-mediated transcription from linearized DNA templates (peTheRNAvs3 vector), which incorporates 5' and 3' UTRs and a polyA tail. The final mRNA utilizes Cap1 and 100% replacement of uridine with N1-methyl-pseudo-uridine.

### LNP synthesis:

Lipid based nanoparticles are produced by microfluidic mixing of an mRNA solution in sodium acetate buffer (100 mM, pH 4) and lipid solution in a 2:1 volume ratio at a speed of 9mL/min using the NanoAssemblr Benchtop (Precision Nanosystems). The lipid solution contained a mixture of the ionizable lipid of interest, DSPC, DOPC or DOPE (Avanti), Cholesterol (Sigma) and DMG-PEG2000 (Sunbright GM-020, NOF corporation). The 4 lipids were mixed at 6 different molar ratios. LNPs were dialyzed against TBS (10000 times more TBS volume than LNP volume) using slide-a-lyzer dialysis cassettes (20K MWCO, 3mL, ThermoFisher). Size, polydispersity and zeta potential were measured with a Zetasizer Nano (Malvern). mRNA encapsulation was measured by standard Ribogreen RNA assay (Invitrogen).

### Cell lines: HEK - TS/A - CT26 - B16

The most optimal culturing conditions per cell type including growth medium, subcultivation ratio, and medium renewal recommendations are summarized below. To harvest adherent cells, used-up growth medium was discarded and cells were rinsed twice with phosphate buffered saline (PBS) (Sigma) before addition of trypsine-EDTA (0.05%) (Gibco, Thermo Fisher Scientific) to loosen the cells. Medium renewal needs to occur every 2 to 3 days, whenever cells reached confluency of approximately 70%. Cell viability was determined using the Vi-Cell XR Cell Viability Analyzer (Beckman Coulter).

**Table 1: Cell type specific culturing conditions**

| Cell type | Description | Source | Growth medium | Subcultivation ratio |
|---|---|---|---|---|
| HEK | Adherent human embryonic kidney cells | ATCC | DMEM + 200U/mL L-glutamin + 2mM P/S (all from Sigma) + 10% FBS (PAN) | 1:6 to 1:10 |
| TSIA | Adherent mouse mammary adenocarcinoma | VIB | RPMI-1640 + 100U/mL L-glutamin + 2mM P/S (all from Sigma) + 10% FBS (PAN) | 1:5 to 1:10 |
| CT26 | Adherent mouse colon fibroblast | ATCC | RPMI-1640 + 100U/mL L-glutamin + 2mM PIS + 10mM HEPES + 1mM sodium pyruvate + 4.5 mg/ml glucose (all from Sigma) + 10% FBS (PAN) | 1:4 to 1:10 |
| B16F0 | Adherent mouse melanoma | VIB | DMEM + 100U/mL L-glutamin + 2mM PIS (all from Sigma) + 10% FBS (PAN) | 1:4 to 1:10 |

| | | | | |
|---|---|---|---|---|
| Abbreviations: DMEM: Dulbecco's Modified Eagle Medium; HEPES: 4-(2-hydroxyethyl)-&-piperazineethanesulfonic acid; RPMI: Roswell Park Memorial Institute; P/S: Penicillin/Streptomycin; FBS: Foetal Bovine Serum | | | | |

### Transfection

Cells were plated in a 96-well plate at a density of 20-30x10e4 cells/100µl complete growth medium (specific per cell type). Transfection was performed when cells reached 70-90% confluency. The positive control Lipofectamine (MessengerMAX, Invitrogen) was diluted in OptiMEM (serum reduced, Gibco) and incubated for 10 minutes. In the meantime, eGFP mRNA and LNPs encapsulating eGFP mRNA were diluted in OptiMEM to get to a concentration of the mRNA content of 200 and 50ng/well. mRNA : lipid complexes were incubated in a 1 : 1 ratio for 5 minutes and were added to each condition in quadruplicate. Cells were incubated for 24 hours at 37°C 5% CO2. Afterwards cells were harvested using 1 x TrypLE select enzyme (Gibco) and stained with a live/dead marker SYTOX blue (Life Technologies) in FACS buffer (PBS supplemented with 1% bovin serum albumin (BSA) and 0.09% azide (all from Sigma)). Cells were immediately acquired after addition of the live dead marker using the Attune Nxt Flow Cytometer (ThermoFisher Scientific). Lipofectamin only (lipofectamine treatment without addition of mRNA) and blanco or untreated (UT) cells were included as negative controls.

### Flow cytometry:

### E7-specific CD8 T cell response:

Blood was collected in heparin coated tubes on days 14 and 25 post tumor inoculation. Red blood cells were lysed and the remaining white blood cells were stained with viability dye. After incubation and washing, APC labelled E7_{(RAHYNIVTF)}-dextramer (Immudex) was added and incubated at RT for 30 minutes. Excess dextramer was washed away and an antibody mixture for surface molecules CD3 and CD8 was added to the cells and incubated for 30 minutes at 4°C.

| **Antibody/Dye** | **Fluorochrome** | **Clone** | **Supplier** |
|---|---|---|---|
| Viability dye | Zombie Aqua | n.a. | BioLegend |
| Dextramer | APC | n.a. | Immudex |
| CD3 | PerCPeF710 | 17A2 | eBioscience (Thermo Fisher) |
| CD8 | V450 | 53-6.7 | BD Horizon |

### eGFP expression

For assessment of eGFP expression, cells were stained with SYTOX blue. Within the gate of SYTOX blue negative cells, expression levels of eGFP determined. The relative mean fluorescence intensity (rel MFI) was calculated as the MFI value of the expression marker divided by that of untransfected cells.

Data were acquired on an Attune Nxt cytometer and analyzed with FlowJo Software. Flow cytometric data were analyzed using the Flowjo version 10 software.

### Tumor inoculation and intra-tumoral injection:

In case of CT26 tumor inoculation we used Balb/c mice. For B16F10 tumor inoculation C57/BL6 mice were used. To prepare for subcutaneous tumor inoculation, mice were anesthetized using 2.5% isoflurane and the injection site was shaved. The injection site is typically on the posterior/lateral aspect of the lower left flank. For inoculation purposes, cells need to be approximately 1 week in culture and between passage 3 and 5 after thawing. Cold tumor cell solution was injected subcutaneously at a dose of 0.5*10e6 cells/50µl PBS. Tumor growth was measured every 2-3 days using the Caliper device. The following formula was used to calculate tumor size: (tumor width * tumor width * tumor length)/2. When tumors reached a mean volume of 50-100 mm³, tumor were injected with LNP containing Firefly luciferase mRNA (10µg mRNA in 20µl TBS buffer) or with control buffer (TBS). After injections, mice were always monitored for 5-10 minutes until fully awake without showing any sings of pain, distress, or complications.

### In vivo bioluminescence:

Fluc mRNA expression in tumor and liver was assessed at 6 an 24 hours post injection. By injecting mice peritoneally with D-luciferin (Promega), bioluminescence can be measured through in vivo imaging (IVIS Spectrum System). Bioluminescence is generated through an oxidation reaction which occurs between the enzyme luciferase derived from the firefly mRNA encapsulated in the LNP and its substrate D-luciferin. The Living Image Software (PerkinElmer) was used to specify tumor and liver 'region of interests (ROIs) after which the average radiance (p/s/cm²/sr) was calculated within these ROIs. To monitor tolerability of different LNP types used, bodyweight at 6 and 24 hours post injection was compared to baseline bodyweight at day of randomization.

### Data analysis:

All raw data were analyzed using the GraphPad Prism version 7 software.

### Example 2.1: Expression levels of reporter eGFP mRNA upon in vitro transfection of HEK293T cells with the indicated LNP compositions.

LNPs were produced at a standard molar ratio ionizable lipid/DOPE/cholesterol/DMG-PEG2000 of about 50/10/38.5/1.5. MC-3 is the ionizable lipid used in Onpattro and is considered the state-of-the art. eGFP mRNA was encapsulated in all LNPs as reporter mRNA, at a mRNA/ionizable lipid molar ratio of 1/10.

**Table 2:**

| **ionizable lipid** | size | PDI | % encap |
|---|---|---|---|
| K-Adm-Dda | 65,56 | 0,197 | 100 |
| K-Adip-Dda | 78,59 | 0,292 | 97 |
| S-Adm-Dda | 115,3 | 0,44 | 100 |
| S-Adip-Dda | 86,95 | 0,197 | 98 |
| S-Ac7-Dda | 74,28 | 0,223 | 95 |
| MC3 | 73,14 | 0,103 | 100 |
| S-Adm-Dsa | 82,11 | 0,042 | 98 |
| S-Adip-Dsa | 70,9 | 0,153 | 101 |
| S-Ac7-Dsa | 71,05 | 0,188 | 99 |

As detailed in Table 2: all LNPs showed a high encapsulation efficiency, as measured by the RiboGreen assay. Size and Polydispersity Index were assessed by Dynamic Light Scattering (DLS) on a Malvern Zetasizer. MC-3 is the ionizable lipid used in Onpattro and is considered the state-of-the art.

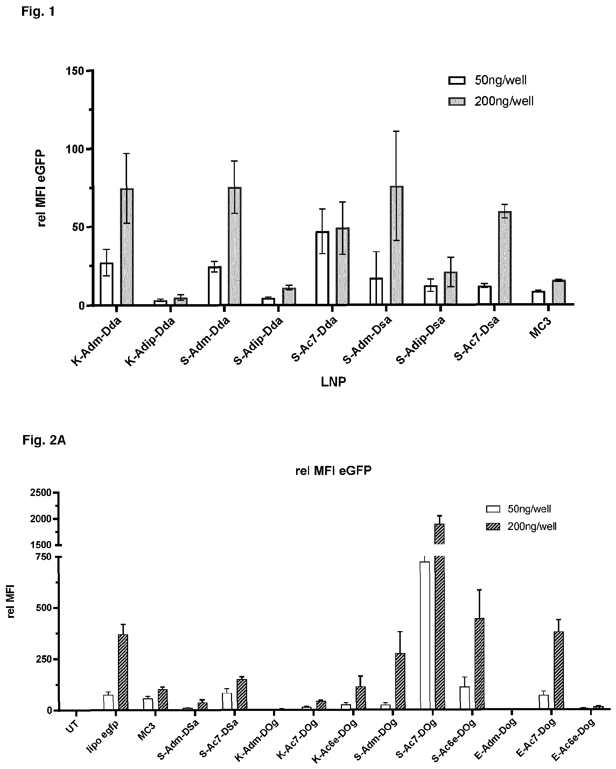

Figure 1 shows the Relative Mean Fluorescence Intensity (measured as the fold-increase in eGFP MFI compared to untreated cells) of eGFP expression in HEK293T cells upon incubation with the indicated LNPs at mRNA conc. of 50 ng and 200 ng/well or MC3 as positive control. As evident from this figure, overall the LNPs of the present invention perform equally well or better compared to the positive control samples.

### Example 2.2: Expression levels of reporter eGFP mRNA upon in vitro transfection of HEK293T and of cancer cell lines (CT26-B16F10-TS/A) with the indicated LNP compositions

LNPs were produced at a standard molar ratio ionizable lipid/phospholipid/cholesterol/DMG-PEG2000 of about 50/10/38.5/1.5. MC-3 is the ionizable lipid used in Onpattro and is considered the state-of-the art. All LNPs were formulated with DOPE, except from the MC-3 based LNP, which was formulated with DSPC as phospholipid. eGFP mRNA was encapsulated in all LNPs as reporter mRNA, at a mRNA/ionizable lipid molar ratio of 1/10.

**Table 3:**

| **ionizable lipid** | size | PDI | % encap |
|---|---|---|---|
| MC3 (DSPC) | 73,33 | 0,08 | 94 |
| S-Adm-DSa | 91,15 | 0,137 | 100 |
| S-Ac7-DSa | 89,44 | 0,158 | 93 |
| K-Adm-DOg | 91,9 | 0,157 | 94 |
| K-Ac7-DOg | 62,26 | 0,108 | 93 |
| K-Ac6e-DOg | 75,8 | 0,268 | 95 |
| S-Adm-DOg | 68,08 | 0,3 | 98 |
| S-Ac7-DOg | 87,46 | 0,21 | 90 |
| S-Ac6e-DOg | 63,86 | 0,311 | 95 |
| E-Adm-Dog | 59,56 | 0,217 | 100 |
| E-Ac7-Dog | 93,05 | 0,258 | 100 |
| E-Ac6e-DOg | 67,3 | 0,241 | 100 |

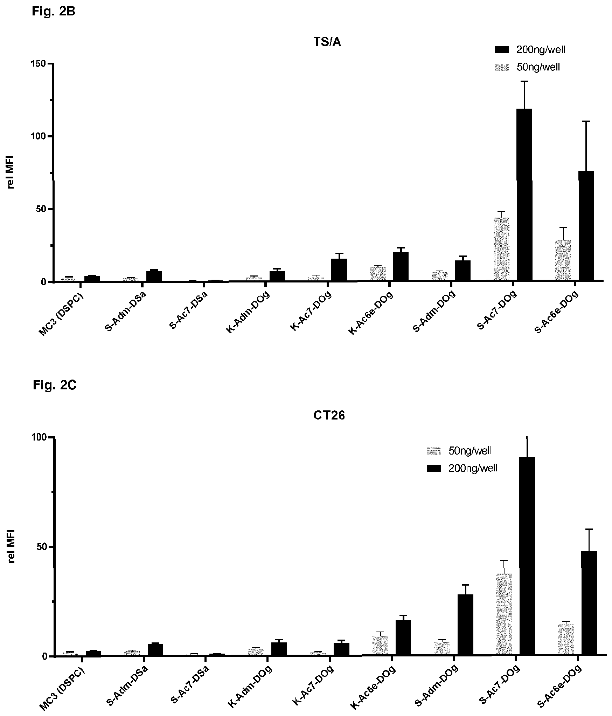

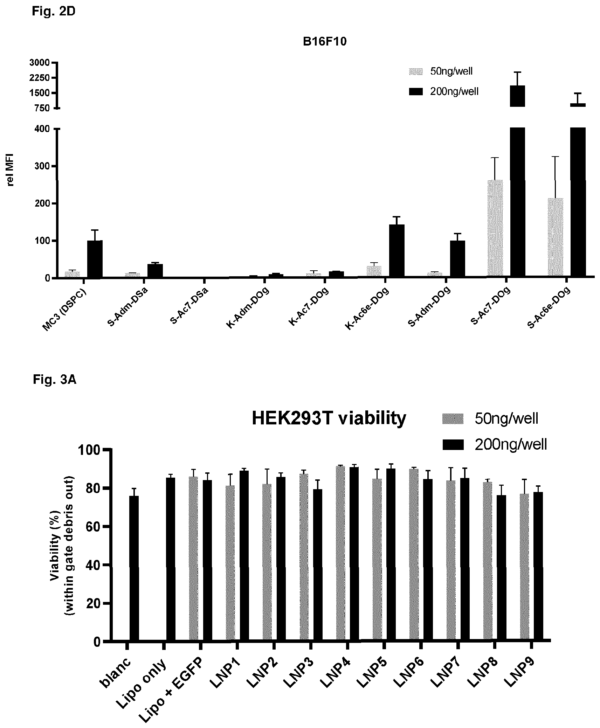

Figure 2 shows again that LNPs perform equally well or better compared to the positive control MC3, in multiple cell lines. Specifically, LNPs with an ionizable lipid combining the S-S linker motif with DOg acyl chains were most efficient in transfecting cells. In terms of amine group, Ac7 was superior to Ac6e and to Adm.

### Example 2.3: Assessing relevance of mol% PEG lipid and Nitrogen/Phosphate (N:P) ratio

To assess the impact of the %DMG-PEG2000 and of the molar ratio ionizable lipid:mRNA on the LNPs' physicochemical properties and capacity to transfect cells, 9 mRNA LNPs were generated at 3 N:P ratios and at 3 mol% DMG-PEG2000. All LNPs were based on S-Ac7-Dog as ionizable lipid. eGFP mRNA was encapsulated to enable assessment of transfection efficiency in vitro.

**Table 4. Size and PDI of the indicated LNP compositions as measured by DLS.**

| LNP number | ionizable lipid | % PEG-lipid | N:P | size | PDI |
|---|---|---|---|---|---|
| 1 | S-Ac7-DOg | 0,5 | 10 | 230,2 | 0,136 |
| 2 | S-Ac-DOg | 1,5 | 10 | 124,3 | 0,102 |
| 3 | S-Ac-DOg | 3,0 | 10 | 96,64 | 0,1 |
| 4 | S-Ac-DOg | 0,5 | 5 | 198,4 | 0,171 |
| 5 | S-Ac-DOg | 1,5 | 5 | 159,9 | 0,149 |
| 6 | S-Ac-DOg | 3,0 | 5 | 96,04 | 0,087 |
| 7 | S-Ac-DOg | 0,5 | 20 | 242,2 | 0,077 |
| 8 | S-Ac-DOg | 1,5 | 20 | 111,1 | 0,165 |
| 9 | S-Ac7-DOg | 3,0 | 20 | 84,54 | 0,117 |

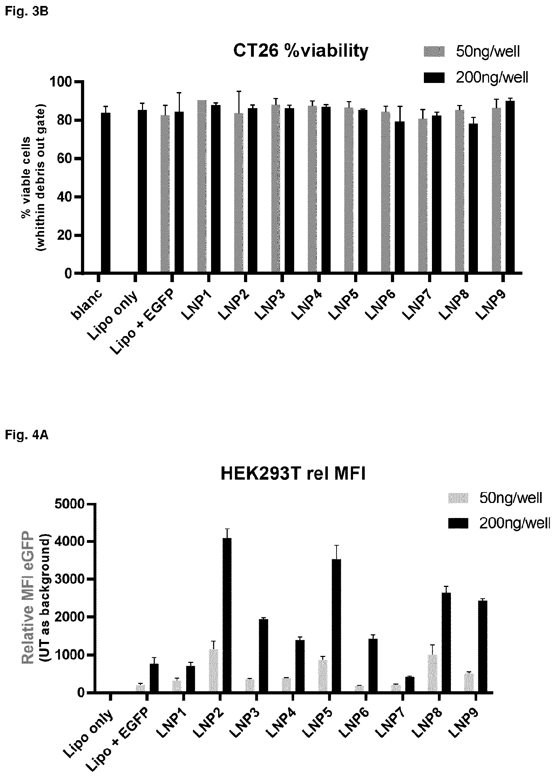

Figure 3A and 3B reveal that none of the LNPs have a significant impact on the viability of the transfected HEK293T cells and CT26 cells respectively.

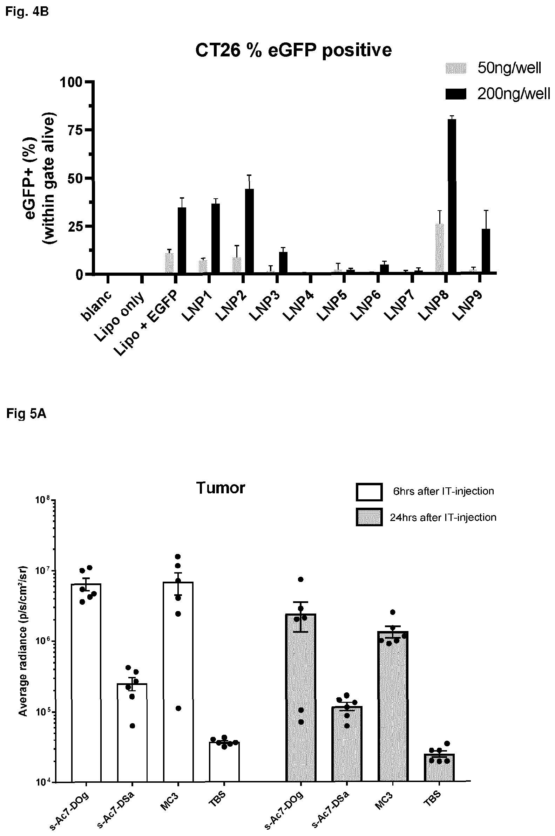

Decreasing the % DMG-PEG2000 led to increases in LNP size. LNPs with 1.5% DMG-PEG2000 showed improved transfection efficiency, across the N:P ratios tested. To transfect HEK293T cells, N:P 10 was most efficient (Figure 4A). To transfect CT26 tumor cells, N:P 20 was superior to the N:P 10 and N:P 5 ratio (Figure 4B).

### Example 2.4: In vivo mRNA expression upon intratumoral mRNA LNP injection of subcutaneously growing CT26 tumors.

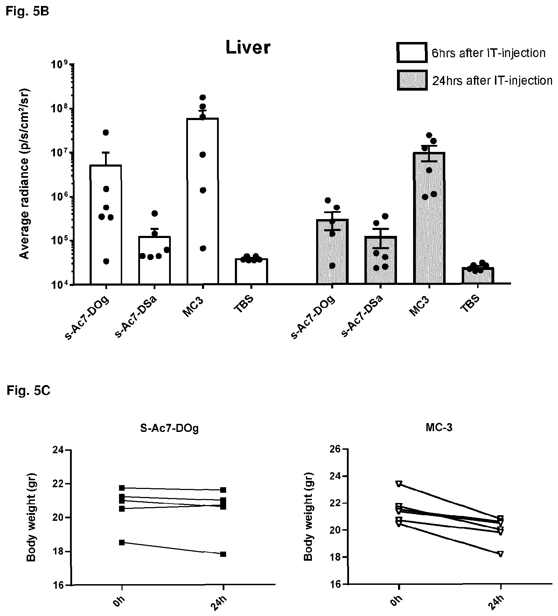

Balb/c mice were subcutaneously inoculated with CT26 tumor cells. When tumors reached a mean volume of 50-100 mm³, tumors were injected with the respective mRNA LNPs (10 µg mRNA; 20 µl volume; TBS buffer) or with control buffer. Fluc mRNA expression in tumors (Fig. 5A) and liver (Fig. 5B) was assessed via in vivo bioluminescence measurement at 6 hours and 24 hours post injection. mRNA delivery by the S-Ac7-DOg based LNP resulted in similar Fluc expression levels in the tumor compared to the MC-3 based benchmark LNP, but show strongly reduced off-target expression in the liver. No weight loss (Fig. 5C) was observed upon mRNA delivery by S-Ac-DOg, whereas delivery by MC-3 resulted in a significant body weight loss.

### Example 2.5: In vivo mRNA expression upon intratumoral mRNA LNP injection of subcutaneously growing B16F10 tumors.

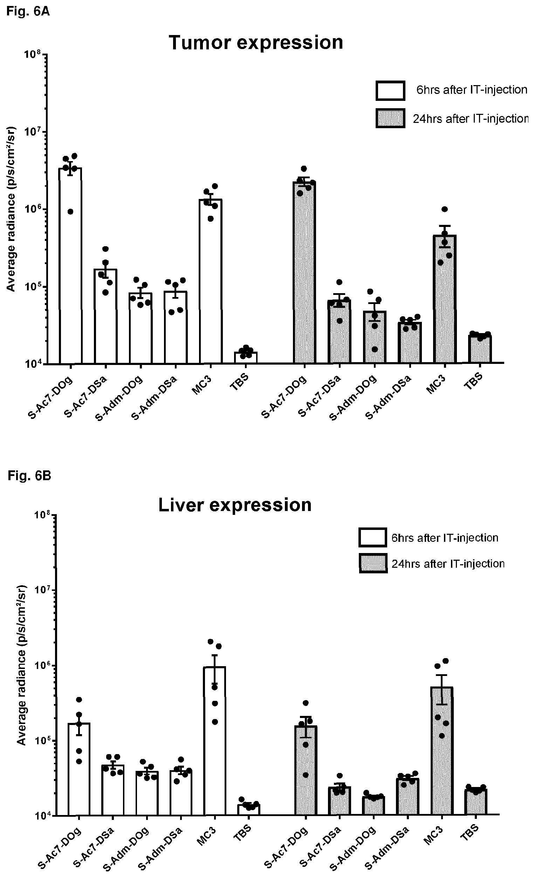

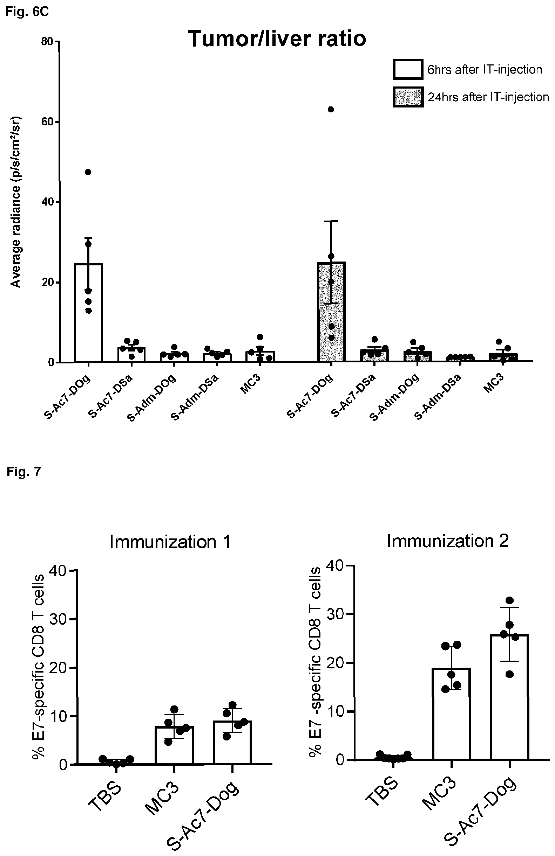

Balb/c mice were subcutaneously inoculated with CT26 tumor cells. When tumors reached a mean volume of 100 mm³, tumors were injected with the respective mRNA LNPs (10 µg mRNA; 20 µl volume; TBS buffer) or with control buffer. Fluc mRNA expression in tumors (Fig. 6A) and liver (Fig. 6B) was assessed via in vivo bioluminescence measurement at 6 hours and 24 hours post injection. mRNA delivery by the S-Ac7-DOg based LNP resulted in increased Fluc expression levels in the tumor compared to the MC-3 based benchmark LNP, but strongly shows strongly reduced off-target expression in the liver (Fig. 6C). No weight loss was observed upon mRNA delivery by S-Ac-DOg, whereas delivery by MC-3 resulted in a significant body weight loss.

### Example 2.6: Induction of T cell responses upon intramuscular vaccination

C57BL/6 mice were vaccinated intramuscular with 10 µg of E7 mRNA encapsulated in LNPs with S-Ac7-DOg or MC-3 as ionizable lipid at days 1 and 8. LNPs were formulated at a molar ratio S-Ac7-DOg/mRNA ratio of 10:1. The E7-specific CD8 T cell response was measured by flow cytometry 6 days after each vaccination (Fig. 7).

### Example 3: Induction of antigen-specific CD8 T cells upon intramuscular mRNA LNP vaccination

### Materials and methods:

### Intramuscular injections and muscle thickness assessment:

All mice were housed under specific pathogen-free conditions, and animal studies were conducted under protocols and guidelines approved by the Ghent University animal care and use committee (ECD20/100). Mice were injected in biceps femoris with mRNA LNPs in TBS (50 µl volume, 5 ug of mRNA). The thickness of the muscle at the injection site was measured with an electronic external measuring gauge (K220T, Kroeplin) at d1 and d4 after injection.

### Flow cytometry (assessment of E7-specific CD8 T cell response): Anna

100ul of whole blood for flow cytometry staining was collected at d7 after prime immunization and at d14 after boost immunization. After red blood cells lysis (RBC lysis buffer, 420302 Biolegend), the whole blood cells were incubated with 0.5 ug/test of Mouse BD Fc Block^{™} (BD, 553142) and Zombie Aqua viability dye (Biolegend, 423102). After incubation and washing, 5ul/test of APC labelled E7_{(RAHYNIVTF)}-dextramer (Immudex, JA2195) was added and incubated at room temperature (RT) for 30 minutes. After wash step cells underwent surface staining during 30 min incubation with an antibody mix in the FACS buffer (PBS, 2mM EDTA, 1%BSA) at the following amounts per test: CD3 PerCP-eFluor710 (Invitrogen, 46-0032-82), CD8-V450 (BD Biosciences, 560469), KLRG1 (Biolegend, 138408) and CD127-BV605 (Biolegend, 135041). After next wash stem samples were acquired on a 3-laser AtuneNxt flow cytometer (ThermoFisher, A29003) and data was analysed with FLowJo v10.7.1 software (BD Biosciences, FlowJo portal account).

### LNP production

LNP formulations were prepared using a modified procedure of a method previously described for siRNA LNP synthesis. All formulations were prepared in a sterile manner with a N/P ratio of 10. All lipid components were dissolved in ethanol at molar ratios of 50:10:38.5:1.5 mol % (ionizable lipid / DSPC / Cholesterol / DMG-PEG2000). DSPC, Cholesterol and DMG-PEG2000 were purchased from Avanti Polar Lipids (Alabaster, Alabama, USA), while the Ionizable lipids are synthesized in-house. The final lipid concentration in ethanol was fixed at 10 mg/mL. E7 mRNA was dissolved in 100 mM acetate buffer (Sigma Aldrich, Saint-Louis, Missouri, USA) pH 4. The ethanol and aqueous phase were combined using a microfluidic mixer (NanoAssembIr^{®} BenchTop (Precision Nanosystems, Vancouver, BC) in a 2:1 (aqueous:ethanol) ratio. Formulations were dialysed afterwards against 1X Sterile TBS pH 7.4 (Sigma Aldrich, Saint-Louis, Missouri, USA) using Slide-A-Lyzer^{®} dialysis cassettes with a MWCO of 20.000 Da (Thermo Scientific, Massachusetts, USA) for 18 hours. Afterwards, the purified formulations were concentrated using Amicon ultra centrifugal filters (EMD Millipore, Massachusetts, USA). Finally, the exact concentration and E.E. % was determined through a Ribogreen Assay prior to final dilution with TBS (100 µg/mL E7 mRNA per formulation. All formulations were characterized for size and PDI using Dynamic Light Scattering Zetasizer Nano-ZS (Malvern Panalytical Ltd., Malvern, UK) and stored afterwards at 4°C. The physico-chemical characteristics of each formulation can be found in Table 5.

**Table 5: List of relevant physico-chemical properties of different LNP formulations. These include the type of ionizable lipid, the composition, the mol fraction of each component, the size and PDI as determined by dynamic light scattering and the encapsulation efficiency. Encapsulation efficiency has been measured by Ribogreen assay.**

| **Ionizable Lipid** | **Composition** | **Ratio [mol %]** | **Size [nm]** | **PDI** | **E. E. [%]** |
|---|---|---|---|---|---|
| S-Ac7-DOg | S-Ac7-DOg / DSPC / Chol / DMG-PEG2000 | 50 / 10 / 38.5 / 1.5 | 97.51 | 0.062 | 96 |
| S-Ac6e-DOg | S-Ac6e-DOg / DSPC / Chol / DMG-PEG2000 | 50 / 10 / 38.5 / 1.5 | 178.1 | 0.266 | 92 |
| S-Ac7-DLg | S-Ac7-DLg / DSPC / Chol / DMG-PEG2000 | 50 / 10 / 38.5 / 1.5 | 150.2 | 0.155 | 90 |
| K-Ac7-DOg | K-Ac7-DOg / DSPC / Chol / DMG-PEG2000 | 50 / 10 / 38.5 / 1.5 | 164.8 | 0.130 | 49 |
| MC3 | MC3 / DSPC / Chol / DMG-PEG2000 | 50 / 10 / 38.5 / 1.5 | 67.57 | 0.106 | 99 |

| | | | | | |
|---|---|---|---|---|---|
| Note: E.E. % is defined as the encapsulation efficiency of the mRNA inside the LNP nanoparticle. | | | | | |

### Results:

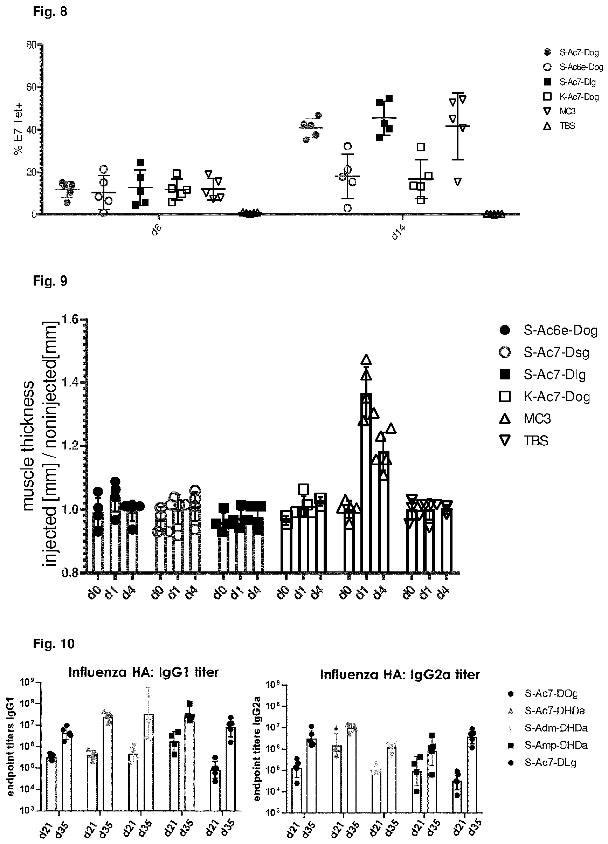

The respective mRNA LNP vaccines induced robust E7-specific CD8 T cell responses upon intramuscular immunization, which were clearly affected by the chemistry of the ionizable lipid (Fig. 8). In contrast to LNPs formulated with MC-3 - the ionizable lipid used to deliver Onpattro - LNPs formulated with the ionizable lipids of interest did not evoke significant edema (as measured by relative increase in muscle thickness) at the injection site (Fig. 9).

### Example 4: induction of anti-HA (hemagglutinin) immune responses upon intramuscular mRNA vaccination

### Materials and methods:

### Flow cytometry (ICS):

2*10e6 splenocytes collected were stimulated with a peptide library containing 139 peptides from HA/Puerto Rico/8/1934 H1N1(PepMix^{™} Influenza A, JPT, PM-INFA-HAPR) at the concentration 1 ug/peptide/ml. 20 ng/ml PMA (79346-1MG, Sigma) and 1 µg/ml lonomycin (I0634-1MG, Sigma) treated splenocytes were used as a positive control. 0.065 µg/sample CD107a - BV711 (564348, BD) antibodies were added together with activation stimuli. Cells were stimulated for 5 h. After 1h from the beginning of stimulation 1X GolgiPlug (555028, BD) was added to halt cytokine secretion. Cells were subsequently incubated with a cocktail of the antibodies for surface staining: 0.125 ug/test CD4-FITC (Biolegend, 100509), 0.02 ug/test Thy1.2-Alexa700 (Biolegend, 140323), 0.05 ug/test CD8-eFluor450 (eBioscience, 48-0081-82). Cells were fixed and permeabilized using BD Cytofix/Cytoperm Plus Fixation/ Permeabilization Solution Kit (555028,BD) according to the guidelines of the manufacturer. Cells were stained in permeabilization buffer containing mAb in the following concentrations: 0.03 ug/test IFNg -PE (BD, 554412), 0.065 ug/test CD154-PerCP-eFluor710 (ebioscience, 46-1541-80), 0.62 ul/test Granz-AF647 ( Biolegend, 515406), 0.125 ug/test IL2-BV605 (BD , 563911), 0.065 ug/ml TNFa-BV785 (Biolegend, 506341) .Cells were analyzed on AtuneNxt flow cytometer (ThermoFisher, A29003). Forward and side light scattering were used to gate mononuclear cells, then duplets were discriminated using FCS-A and FCS-H scattering, then dead cells were gated out based on fixable dead cell stain fluorescence. All additional gates are indicated and were based on fluorescence minus one controls where applicable. Data acquisition and analysis was done using FlowJo v10.7.1 software (BD Biosciences).

### Assessment of mouse endpoint Immunoglobulin titers

100 ul of mouse whole blood was collected on d21 and d35 in serum gel tubes (SarsTedt). Serum was separated from the blood clot by centrifugation at 10 000 g for 10 min at 4C.

Black flat bottom maxisorp 96-well plates (437111, Life Technologies) were coated overnight at 4C with 100 µl 1µg/ml of recombinant H1N1 (A/Puerto Rico/8/1934) HA protein (Sino Biological, 11684-V08H) in carbonate/bicarbonate buffer (0.1 M, pH 9.6). Plates were subsequently blocked with 100 µl of 3% BSA (05479-250g, Sigma) in PBS (w/v) for 2h. Subsequently, plates were washed 3 times with PBS/0.1% Tween (10113103, Fisher Scientific). A serial dilution of serum samples was added to the plates (initial 100X dilution of serum for d21 and initial 1000x serum dilution for d35; 5X dilution steps). After 2h incubation at RT plates were washed 5 times and solutions of rabbit anti-mouse IgG1 conjugated with HRP (1:15 000, Biorad, OBT1508P) or goat anti-mouse IgG2a conjugated with HRP (1:8000, STAR133P Biorad) were added for another 1h. After a final wash, the fluorescent Amplex UltraRed Reagent (A36006, Invitrogen) was used to develop plates according to the manufacturer's instructions. Plates were read on a Tecan Infinite 200 Pro with λₑₓ=540nm, λₑₘ=590nm. The dilutions of serum of TBS treated mice served for cut-off determination, being the average fluorescence measured in the TBS samples plus 3 standard deviations. All points beyond cut-off were considered to be below quantification limit. The 5PL curves were fitted to the dilution data then the endpoint titer was calculated at cross point of the modeled curve with the cut off.

### LNP production:

LNP formulations were prepared as indicated above. All formulations were prepared in a sterile manner with a N/P ratio of 10. All formulations were characterized for size and PDI using Dynamic Light Scattering Zetasizer Nano-ZS (Malvern Pananlytical Ltd., Malvern, UK) and stored afterwards at 4°C. The physico-chemical characteristics of each formulation can be found in Table 6.

**Table 6: List of relevant physico-chemical properties of different LNP formulations. These include the type of ionizable lipid, the lipid composition and the mol fraction of each component, the size and PDI (as determined by dynamic light scattering) and the encapsulation efficiency. Encapsulation efficiency has been measured by Ribogreen assay.**

| **Ionizable Lipid** | **Composition** | **Ratio [mol %]** | **Size [nm]** | **PDI** | **E. E. [%]** |
|---|---|---|---|---|---|
| S-Ac7-DOg | S-Ac7-DOg / DSPC / Chol / DMG-PEG2000 | 50 / 10 / 38.5 / 1.5 | 93,62 | 0,077 | 98 |
| S-Ac7-DHDa | S-Ac7-DHDa / DSPC / Chol / DMG-PEG2000 | 50 / 10 / 38.5 / 1.5 | 105,1 | 0,083 | 97 |
| S-Adm-DHDa | S-Adm-DHDa / DSPC / Chol / DMG-PEG2000 | 50 / 10 / 38.5 / 1.5 | 79,075 | 0,184 | 98 |
| S-Amp-DHDa | S-Amp-DHDa / DSPC / Chol / DMG-PEG2000 | 50 / 10 / 38.5 / 1.5 | 106,3 | 0,1515 | 99 |
| S-Ac7-DLg | S-Ac7-DLg / DSPC / Chol / DMG-PEG2000 | 50 / 10 / 38.5 / 1.5 | 180,15 | 0,163 | 75 |

| | | | | | |
|---|---|---|---|---|---|
| Note: E.E. % is defined as the encapsulation efficiency of the mRNA inside the LNP nanoparticle. | | | | | |

### Results:

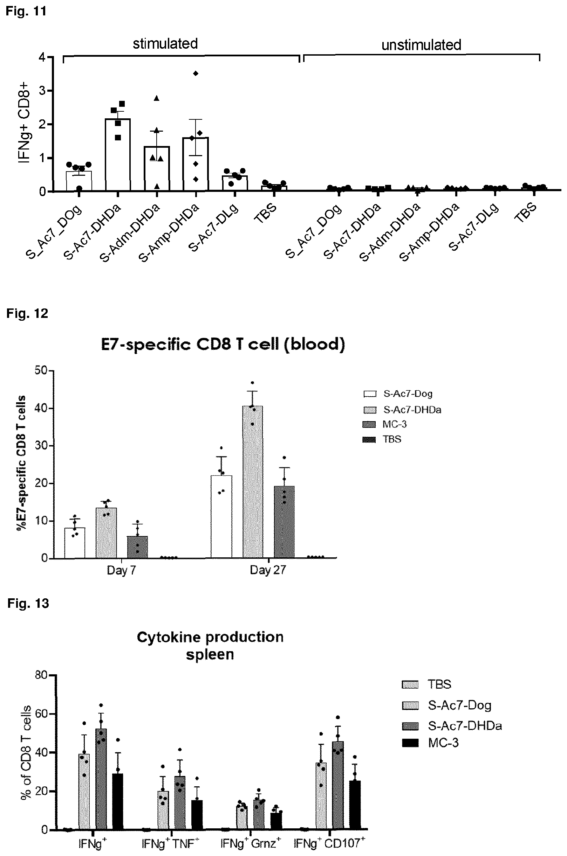

The respective mRNA LNP vaccines induced anti-HA antibody titers. A clear increase in titers was observed after boosting (Fig. 10). In addition, the mRNA LNP vaccines also elicited IFNg+ CD8 T cell responses against HA, which were influenced by the chemistry of the ionizable lipid used (Fig. 11).

### Example 5: Induction of E7-specific CD8 T cell responses upon intramuscular vaccination

### Materials and methods:

### Flow cytometry (assessment of E7-specific CD8 T cell response):

100 µl of whole blood for flow cytometry staining was collected at d7 after prime immunization and at d14 after boost immunization. After red blood cells lysis (RBC lysis buffer, 420302 Biolegend), the whole blood cells were incubated with 0.5 ug/sample of Mouse BD Fc Block^{™} (BD, 553142) and Zombie Aqua viability dye (Biolegend, 423102). After incubation and washing, 5µl/sample of APC labelled E7_{(RAHYNIVTF)}-dextramer (Immudex, JA2195) was added and incubated at room temperature (RT) for 30 minutes. After wash step cells underwent surface staining during 30 min incubation with an antibody mix in the FACS buffer (PBS, 2mM EDTA, 1%BSA) at the following amounts per test: CD3 PerCP-eFluor710 (Invitrogen, 46-0032-82), CD8-V450 (BD Biosciences, 560469), KLRG1 (Biolegend, 138408) and CD127-BV605 (Biolegend, 135041). Samples were acquired on a 3-laser Atune Nxt flow cytometer (ThermoFisher, A29003) and data was analysed with FLowJo software (FlowJo_v10.7.1, FlowJo portal account).

### LNP production:

LNP formulations were prepared as indicated above. All formulations were prepared in a sterile manner with a N/P ratio of 10. All formulations were characterized for size and PDI using Dynamic Light Scattering Zetasizer Nano-ZS (Malvern Pananlytical Ltd., Malvern, UK) and stored afterwards at 4°C. The physico-chemical characteristics of each formulation can be found in Table 7.

**Table 7: List of relevant physico-chemical properties of different LNP formulations. These include the type of ionizable lipid, the lipid composition together and the mol fraction of each component, the size and PDI (as determined by dynamic light scattering) and the encapsulation efficiency. Encapsulation efficiency has been measured by Ribogreen assay.**

| **Ratio** | **Ionizable Lipid** | **Composition** | **Ratio [mol %]** | **Size [nm]** | **PDI** | **E. E. [%]** |
|---|---|---|---|---|---|---|
| 1 | S-Ac7-DHDa | S-Ac7-DHDa / DSPC / Chol / DMG-PEG2000 | 50 / 10 / 38.5 / 1.5 | 90 | 0.095 | 97 |
| 2 | S-Ac7-DHDa | S-Ac7-DHDa / DSPC / Chol / DMG-PEG2000 | 56.71 / 14.43 / 27.58 / 1.28 | 88 | 0.081 | 95 |
| 1 | S-Ac7-DOg | S-Ac7-DOg / DSPC / Chol / DMG-PEG2000 | 50 / 10 / 38.5 / 1.5 | 109 | 0.094 | 97 |
| 2 | S-Ac7-DOg | S-Ac7-DOg / DSPC / Chol / DMG-PEG2000 | 56.71 / 14.43 / 27.58 / 1.28 | 97 | 0.106 | 96 |
| 1 | SS-EC | SS-EC / DOPE / Chol / DMG-PEG2000 | 50 / 10 / 38.5 / 1.5 | 85 | 0.177 | 97 |
| 1 | MC-3 | MC-3 / DSPC / Chol / DMG-PEG2000 | 50 / 10 / 38.5 / 1.5 | 74 | 0.117 | 96 |

| | | | | | | |
|---|---|---|---|---|---|---|
| Note: E.E. % is defined as the encapsulation efficiency of the mRNA inside the LNP nanoparticle. | | | | | | |

### Results:

The respective mRNA LNP vaccines induced robust E7-specific CD8 T cell responses upon intramuscular immunization, which were clearly affected by the chemistry of the ionizable lipid (Fig. 12). LNPs based on S-Ac7-DHDa induced superior T cell responses compared to MC-3 (Fig. 13).

### Example 6: intravenous immunization with LNP co-encapsulating peptide antigen and imidazoquinoline TLR7/8 agonist

### Materials and methods

### Lipid nanoparticle (LNP) formulation

The minimal epitope amino acid sequence of E7 (RAHYNIVTF) was extended with ten glutamic acid residues and a flanking amino acid sequence (QAEPD) from the native E7 protein amino acid sequence and two serine residues (SS). The resulting peptide (EEEEEEEEEESSQAEPDRAHYNIVTF) is further referred to as GLU10-E7. As unformulated control, SSQAEPDRAHYNIVTF is used and is further referred to as E7. The TLR7/8 agonist 1-(4-(aminomethyl)benzyl)-2-butyl-1*H*-imidazo[4,5-c]quinolin-4-amine (IMDQ) was conjugated a peptide containing ten glutamic acid residues. This conjugated is further referred to as GLU10-IMDQ. For fluorescence-based tracking, a Cy5-labeled peptide containing ten glutamic acid residues, further referred to as GLU10-Cy5, was used.

For LNP formulation, an aqueous phase containing GLU10-E7 (or GLU10-Cy5 for fluorescence-based tracking experiments) and GLU10-IMDQ was prepared in a 25 mM acetate buffer (pH 5.2) GLU10-E7 or GLU10-IMDQ. An organic phase was prepared by dissolving S-Ac7-DOG, DOPE, cholesterol and DMG-PEG at a molar ratio of 50 : 10 : 38.5 : 1.5. The ratio of peptide to ionizable lipid was fixed at an N:C ratio 5:1 ( N: ionisable amine from the ionisable lipid, C: carboxylic acid from glutamic acid residues). Organic and aqueous solutions were mixed at a 1 : 3 ratio by dropwise addition of the organic phase into a vigorously stirring aqueous solution. Subsequently, the formed LNPs are dialyzed against PBS for 12 h using a 3.5 kDa cut-off dialysis membrane to remove ethanol.

### Results and methods

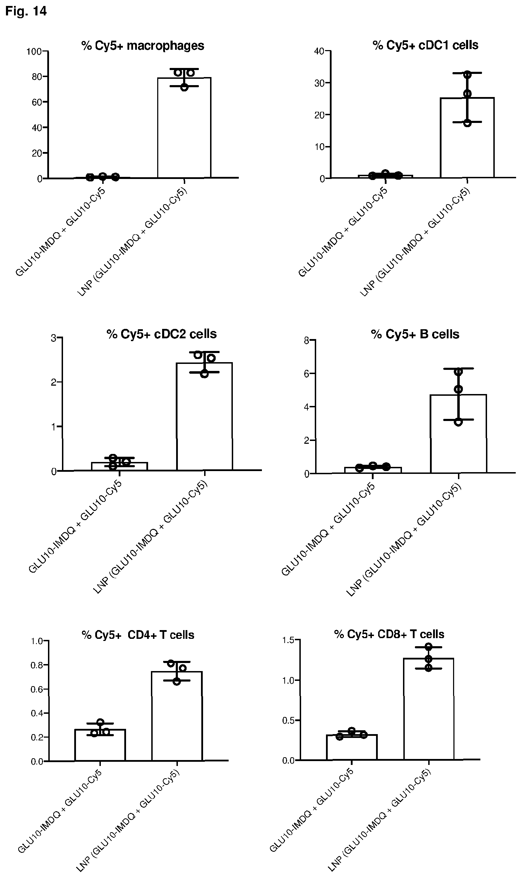

Relative to unformulated soluble peptide (GLU10-Cy5), LNP-formulated peptide (LNP (GLU10)) leads to a strong increase in peptide uptake by macrophages and dendritic cells (cDC1 and cDC2 subsets) in the spleen after intravenous administration (Figure 14). Also, B cells and to a slight extent T cells become associated with peptide. Co-formulation of GLU10-IMDQ in LNP further increases splenic uptake of peptide by macrophages, cDC1 dendritic cells, B cells and T cells (CD4+ and CD8+ T cells subsets).

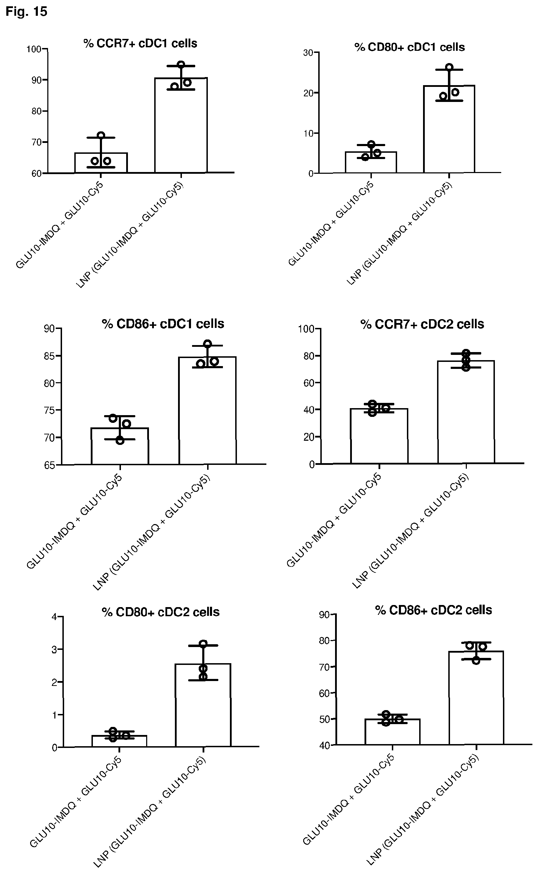

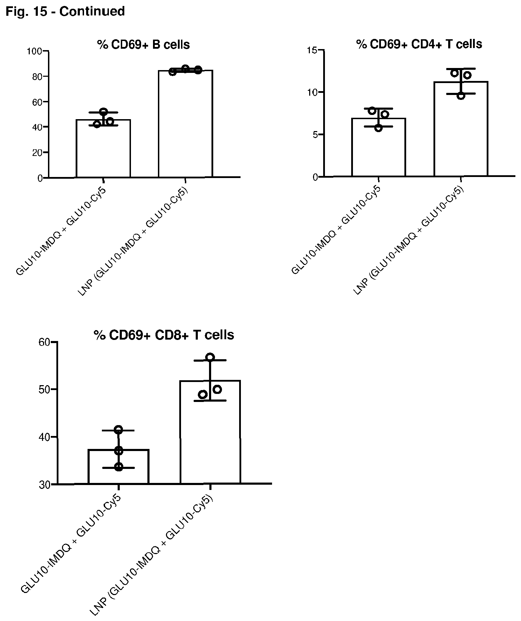

LNP containing the TLR7/8 agonist GLU10-IMDQ can activate dendritic cells (cDC1 and cDC2 subsets), B cells and T cells (CD4+ and CD8+ T cells subsets) in the spleen after intravenous administration (Figure 15).

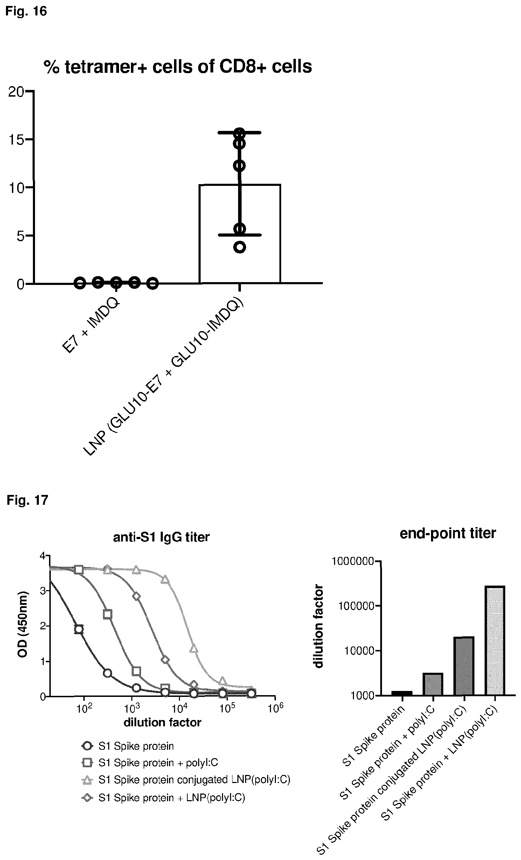

Relative to unformulated soluble peptide E7 antigen, and soluble peptide E7 antigen adjuvanted with the TLR7/8 agonist IMDQ, LNP containing GLU10-E7 peptide antigen and GLU10-IMDQ induce a strong increase in tetramer-positive CD8 T cells in the blood of immunized mice after 2 doses with a 2-week interval. Administration of antigen and TLR7/8 agonist within the same LNP induces a higher response that separate populations of LNP containing antigen or TLR7/8 agonist respectively (Figure 16).

### Example 7: intramuscular immunization with polvl:C LNP and protein antigen

### Materials and methods

### Lipid nanoparticle (LNP) formulation

LNP formulations were prepared with a N/P ratio of 10. All lipid components were dissolved in ethanol at molar ratios of 50:10:38.5:1.5 mol % (S-Ac7-DOg / DOPE / Cholesterol / DSG-PEG2000). Low molecular weight polyl:C was dissolved in 100 mM acetate buffer pH 4. The ethanol and aqueous phase were mixed at a 1 : 3 ratio by dropwise addition of the organic phase into a vigorously stirring aqueous solution. Subsequently, the formed LNP are dialyzed against PBS for 12 h using a 3.5 kDa cut-off dialysis membrane to remove ethanol.

### Results and methods

Relative to naïve polyl:C, formulation of polyl:C in LNP increases the anti-S1 Spike protein IgG antibody titers. These titers are further increased when S1 Spike protein is conjugated to the LNP surface (Figure 17).

### Example 8: intramuscular immunization with CpG LNP and protein antigen

### Materials and methods

### Lipid nanoparticle (LNP) formulation

LNP formulations were prepared with a N/P ratio of 10. All lipid components were dissolved in ethanol at molar ratios of 50:10:38.5:1.5 mol % (S-Ac7-DOg / DOPE / Cholesterol / DSG-PEG2000). CpG was dissolved in 100 mM acetate buffer pH 4. The ethanol and aqueous phase were were mixed at a 1 : 3 ratio by dropwise addition of the organic phase into a vigorously stirring aqueous solution. Subsequently, the formed LNP are dialyzed against PBS for 12 h using a 3.5 kDa cut-off dialysis membrane to remove ethanol.

### Results and methods

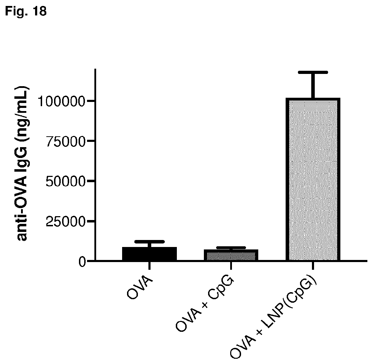

Relative to native CpG, formulation of CpG in LNP increases the anti-ovalbumin (OVA) IgG antibody titers (Figure 18).

### REFERENCES

1. Amano, Y.; Umezawa, N.; Sato, S.; Watanabe, H.; Umehara, T.; Higuchi, T. Activation of lysine-specific demethylase 1 inhibitor peptide by redox-controlled cleavage of a traceless linker. Bioorg. Med. Chem. 2017, 25, 1227-1234.
2. Shenoi, R. A.; Lai, B. F. L.; Kizhakkedathu, J. N. Synthesis, characterization, and biocompatibility of biodegradable hyperbranched polyglycerols from acid-cleavable ketal group functionalized initiators. Biomacromolecules 2012, 13, 3018-3030.
3. Luo, C.; Miao, L.; Zhao, Y.; Musetti, S.; Wang, Y.; Shi, K.; Huang, L. A novel cationic lipid with intrinsic antitumor activity to facilitate gene therapy of TRAIL DNA. Biomaterials 2016, 102, 239-248.
4. Yeager, A. R.; Finney, N. S. The first direct evaluation of the two-active site mechanism for chitin synthase. J. Org. Chem. 2004, 69, 613-618.

## Claims

1. An ionizable lipid represented by formula (I) 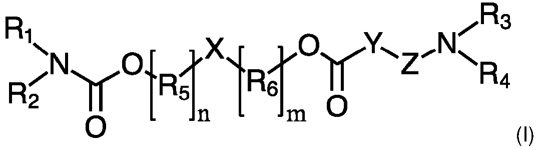 wherein
R₁ and R₂ are each independently selected from -H, -C₁₋₂₀alkyl, -C₂₋₂₀alkenyl, and -C₂₋₂₀alkynyl; wherein each of said -C₁₋₂₀alkyl, -C₂₋₂₀alkenyl, and -C₂₋₂₀alkynyl may optionally be substituted with from 1 to 3 -O-(C=O)-R₇, -(C=O)-O-R₇, -C₁₋₂₀alkyl, -C₂₋₂₀alkenyl, and -C₂₋₂₀alkynyl; and wherein the total number of C atoms in R₁ and R₂ together is at least 8;
R₃ and R₄ are each independently a -C₁₋₆alkyl; or R₃ and R₄ taken together with the N atom to which they are attached form a 5-10 membered aromatic or non-aromatic heterocycle; said heterocycle may further optionally comprise one or more additional N atoms, and/or may optionally be substituted with from 1-3 substituents selected from: -C₁₋₆alkyl; and
each R₅ and R₆ is independently -CH₂-;
each R₇ is independently selected from -C₁₋₂₀alkyl, -C₂₋₂₀alkenyl, and -C₂₋₂₀alkynyl; wherein each of said -C₁₋₂₀alkyl, -C₂₋₂₀alkenyl, and -C₂₋₂₀alkynyl may optionally be substituted with from 1 to 3 -O-(C=O)-R₇, -(C=O)-O-R₇, -C₁₋₂₀alkyl, -C₂₋₂₀alkenyl, and -C₂₋₂₀alkynyl;
m and n are each independently an integer selected from 1, 2, 3, and 4;
X is selected from -O-, -S-, -S-S-, -O-(C=O)-, -O-(C=O)-O-, -(C=N-NH₂)-, -O-CR₈R₉-O-, and -S-CR₈R₉-S-;
each R₈ and R₉ is independently selected from -H, -C₁₋₆alkyl and -C₃₋₆cycloalkyl;
Y is selected from -NH- and -O-; and
Z is -C₁₋₆alkylene-.

2. An ionizable lipid as defined in claim 1, and being represented by formula (II) 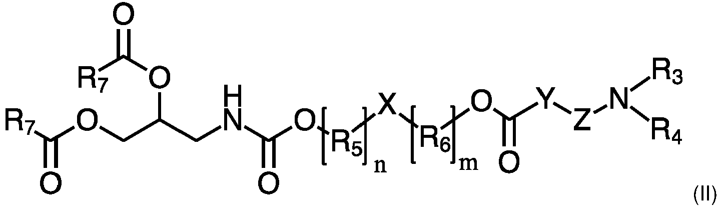 wherein
R₃ and R₄ are each independently a -C₁₋₆alkyl; or R₃ and R₄ taken together with the N atom to which they are attached form a 5-10 membered aromatic or non-aromatic heterocycle; said heterocycle may further optionally comprise one or more additional N atoms, and/or may optionally be substituted with from 1-3 substituents selected from: -C₁₋₆alkyl;
each R₅ and R₆ is independently -CH₂-;
each R₇ is independently selected from -C₁₋₂₀alkyl, -C₂₋₂₀alkenyl, and -C₂₋₂₀alkynyl; wherein each of said -C₁₋₂₀alkyl, -C₂₋₂₀alkenyl, and -C₂₋₂₀alkynyl may optionally be substituted with from 1 to 3 -O-(C=O)-R₇, -(C=O)-O-R₇, -C₁₋₂₀alkyl, -C₂₋₂₀alkenyl, and -C₂₋₂₀alkynyl; and the total number of C atoms in both R₇ moieties together is at least 5;
m and n are each independently an integer selected from 1, 2, 3, and 4;
X is selected from -O-, -S-, -S-S-, -O-(C=O)-, -O-(C=O)-O-, -(C=N-NH₂)-, -O-CR₈R₉-O-, and -S-CR₈R₉-S-;
each R₈ and R₉ is independently selected from -H, -C₁₋₆alkyl and -C₃₋₆cycloalkyl;
Y is selected from -NH- and -O-; and
Z is -C₁₋₆alkylene-.

3. An ionizable lipid as defined in anyone of claims 1 or 2 and being represented by anyone of formula (Illa), (IIIb) or (IIIc) 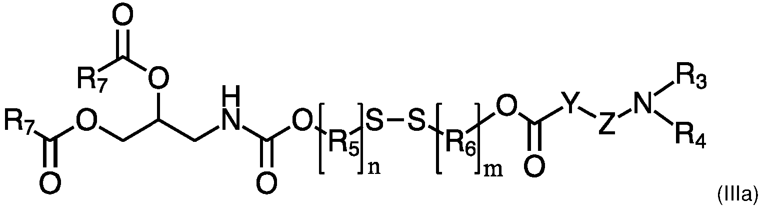 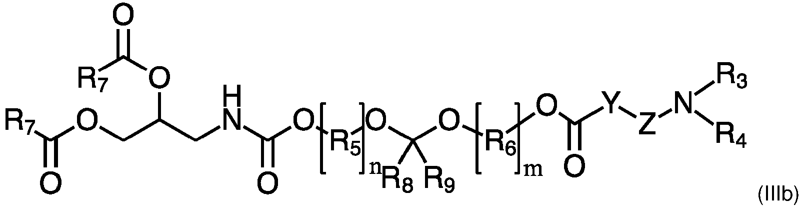 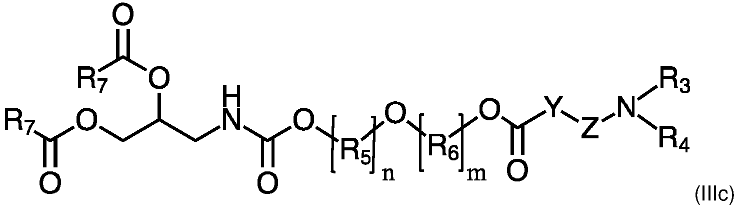 wherein
R₃ and R₄ are each independently a -C₁₋₆alkyl; or R₃ and R₄ taken together with the N atom to which they are attached form a 5-10 membered aromatic or non-aromatic heterocycle; said heterocycle may further optionally comprise one or more additional N atoms, and/or may optionally be substituted with from 1-3 substituents selected from: -C₁₋₆alkyl; and
each R₈ and R₆ is -CH₂-;
each R₇ is independently selected from -C₁₋₂₀alkyl, -C₂₋₂₀alkenyl, and -C₂₋₂₀alkynyl; wherein each of said -C₁₋₂₀alkyl, -C₂₋₂₀alkenyl, and -C₂₋₂₀alkynyl may optionally be substituted with from 1 to 3 -O-(C=O)-R₇, -(C=O)-O-R₇, -C₁₋₂₀alkyl, -C₂₋₂₀alkenyl, and -C₂₋₂₀alkynyl; and the total number of C atoms in both R₇ moieties together is at least 5;
m and n are each independently an integer selected from 1, 2, 3, and 4;
each R₈ and R₉ is independently selected from -H, -C₁₋₆alkyl and -C₃₋₆cycloalkyl;
Y is selected from -NH- and -O-; and
Z is -C₁₋₆alkylene-.

4. An ionizable lipid as defined in claim 1 and being represented by anyone of formula (IVa), (IVb), and (IVc) 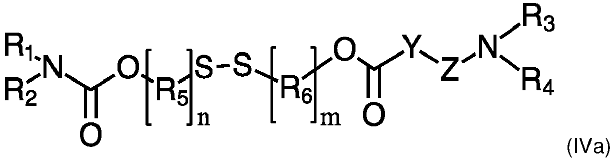 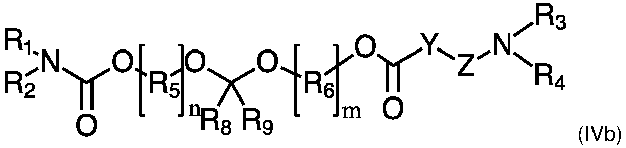 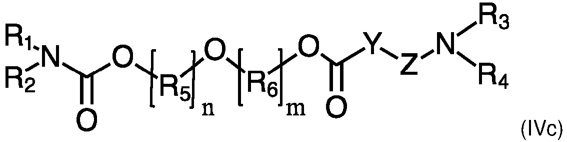 wherein
R₁ and R₂ are each independently selected from -H, -C₁₋₂₀alkyl, -C₂₋₂₀alkenyl, and -C₂₋₂₀alkynyl; wherein each of said -C₁₋₂₀alkyl, -C₂₋₂₀alkenyl, and -C₂₋₂₀alkynyl may optionally be substituted with from 1 to 3 -O-(C=O)-R₇, -(C=O)-O-R₇, -C₁₋₂₀alkyl, -C₂₋₂₀alkenyl, and -C₂₋₂₀alkynyl; and wherein the total number of C atoms in R₁ and R₂ together is at least 8;
R₃ and R₄ are each independently a -C₁₋₆alkyl; or R₃ and R₄ taken together with the N atom to which they are attached form a 5-10 membered aromatic or non-aromatic heterocycle; said heterocycle may further optionally comprise one or more additional N atoms, and/or may optionally be substituted with from 1-3 substituents selected from: -C₁₋₆alkyl;
each R₇ is independently selected from -C₁₋₂₀alkyl, -C₂₋₂₀alkenyl, and -C₂₋₂₀alkynyl; wherein each of said -C₁₋₂₀alkyl, -C₂₋₂₀alkenyl, and -C₂₋₂₀alkynyl may optionally be substituted with from 1 to 3 -O-(C=O)-R₇, -(C=O)-O-R₇, -C₁₋₂₀alkyl, -C₂₋₂₀alkenyl, and -C₂₋₂₀alkynyl;
each R₅ and R₆ is independently selected from -CH₂-, and -O-CH₂-;
m and n are each independently an integer selected from 1, 2, 3, and 4;
each R₈ and R₉ is independently selected from -H, -C₁₋₆alkyl, and -C₃₋₆cycloalkyl;
Y is selected from -NH- and -O-; and
Z is -C₁₋₆alkylene-.

5. An ionizable lipid as defined in claim 1 and being represented by formula (V) 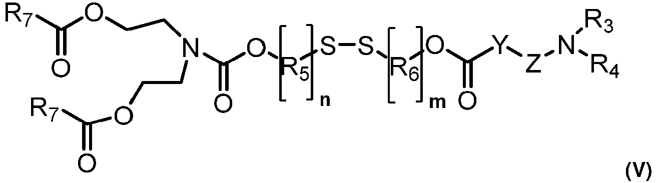 wherein
R₃ and R₄ are each independently a -C₁₋₆alkyl; or R₃ and R₄ taken together with the N atom to which they are attached form a 5-10 membered aromatic or non-aromatic heterocycle; said heterocycle may further optionally comprise one or more additional N atoms, and/or may optionally be substituted with from 1-3 substituents selected from: -C₁₋₆alkyl; and
each R₅ and R₆ is independently selected from -CH₂-, and -O-CH₂-;
each R₇ is independently selected from -C₁₋₂₀alkyl, -C₂₋₂₀alkenyl, and -C₂₋₂₀alkynyl; wherein each of said -C₁₋₂₀alkyl, -C₂₋₂₀alkenyl, and -C₂₋₂₀alkynyl may optionally be substituted with from 1 to 3 -O-(C=O)-R₇, -C₁₋₂₀alkyl, -C₂₋₂₀alkenyl, and -C₂₋₂₀alkynyl; and the total number of C atoms in both R₇ moieties together is at least 5;
m and n are each independently an integer selected from 1, 2, 3, and 4;
Y is selected from -NH- and -O-; and
Z is -C₁₋₆alkylene-.

6. An ionizable lipid as defined in any one of claims 1 to 5, and being selected from the list comprising:

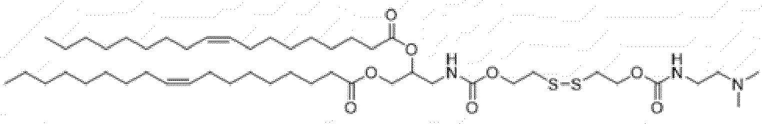

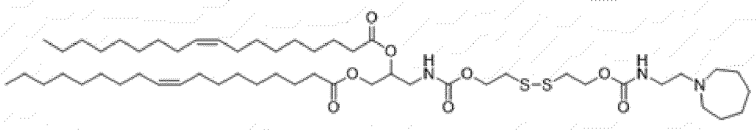

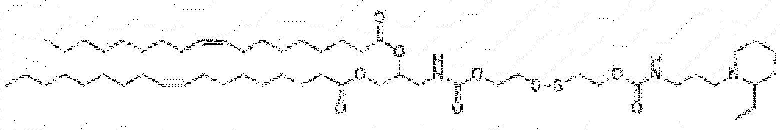

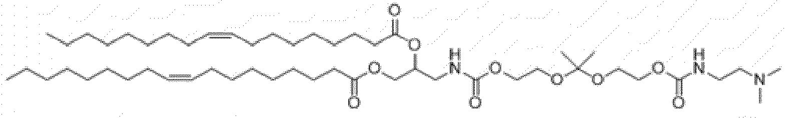

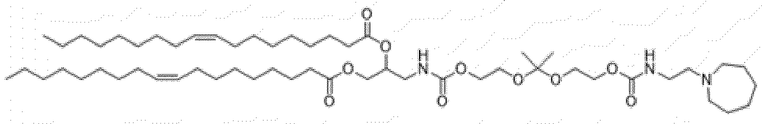

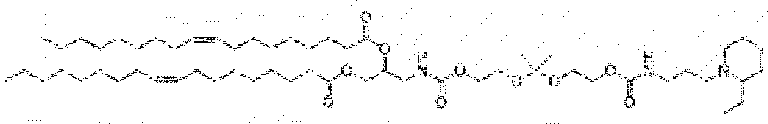

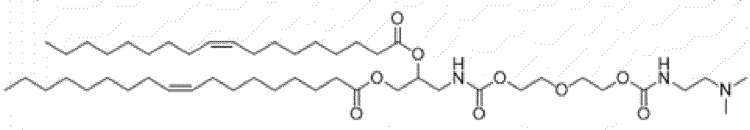

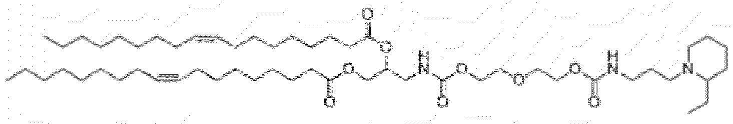

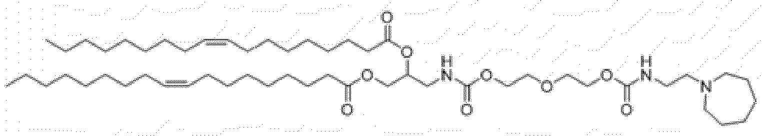

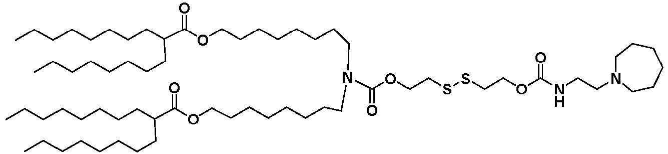

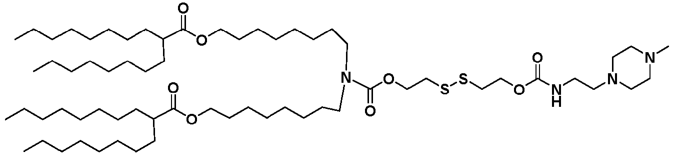

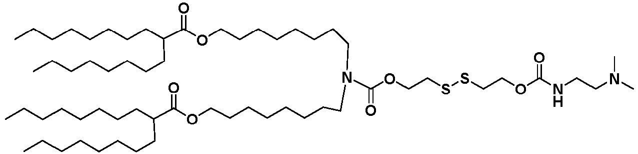

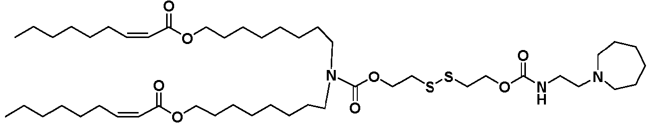

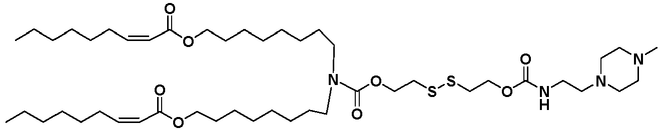

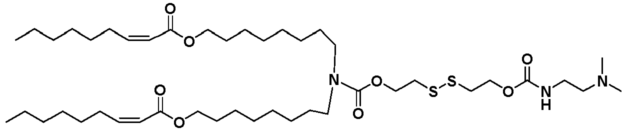

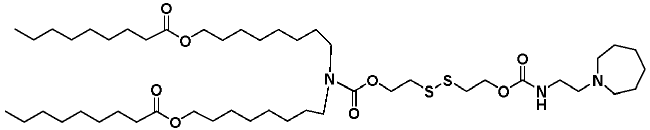

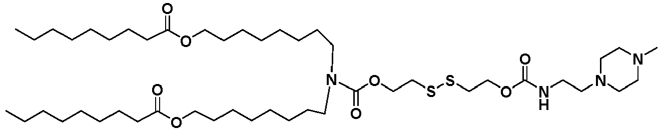

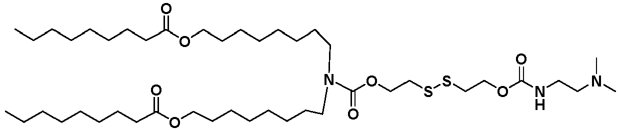

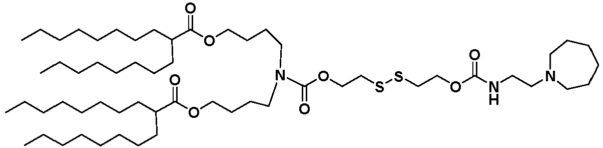

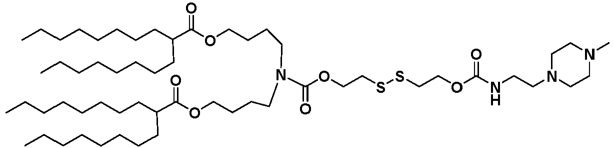

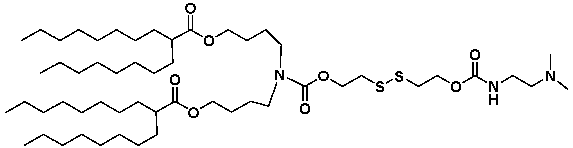

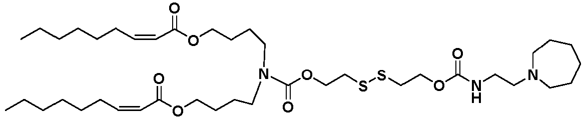

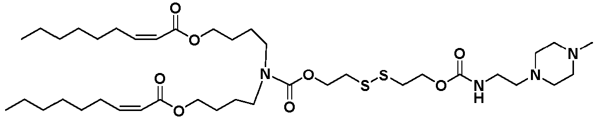

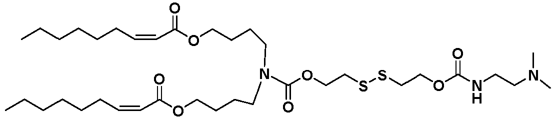

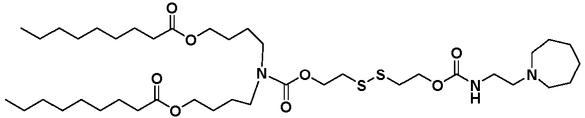

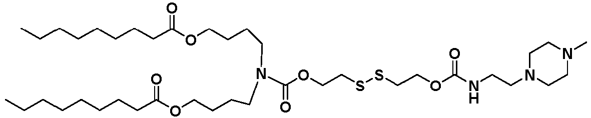

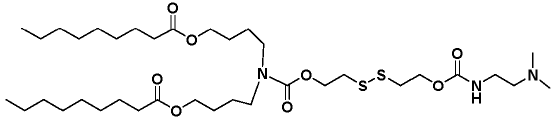

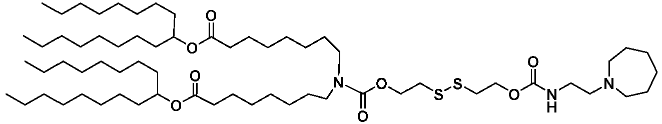

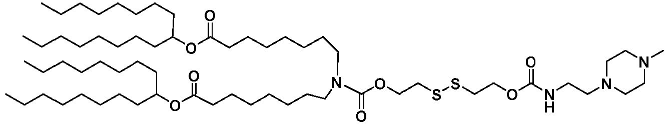

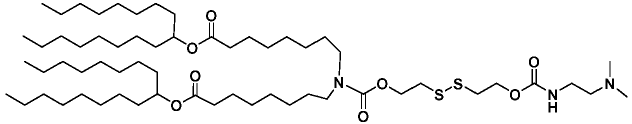

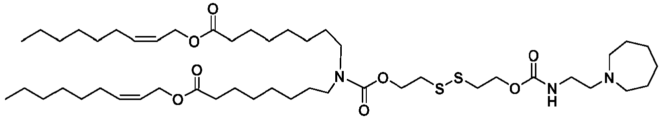

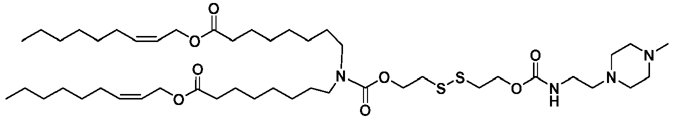

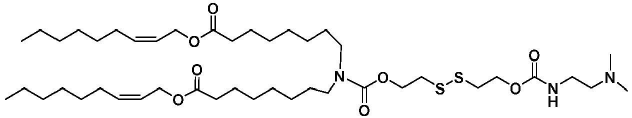

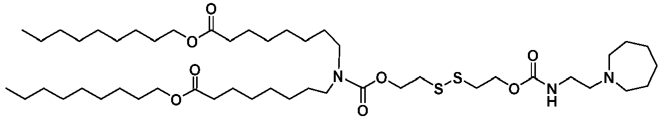

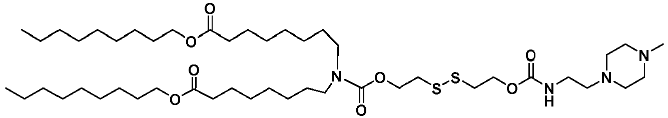

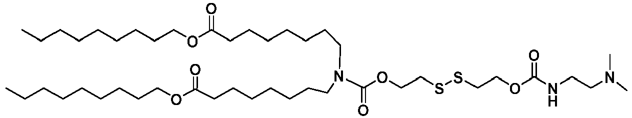

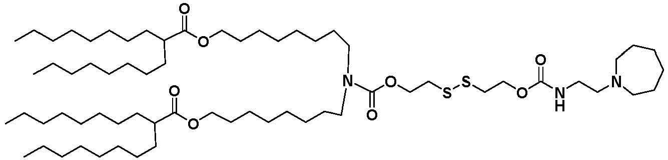

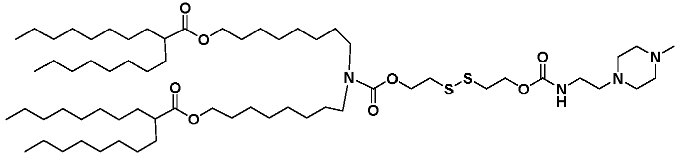

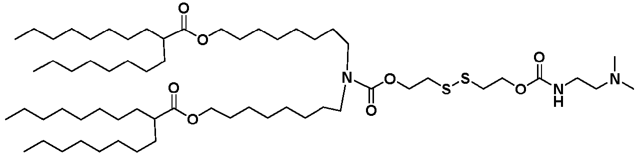

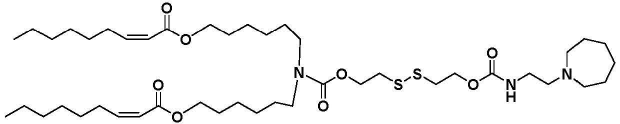

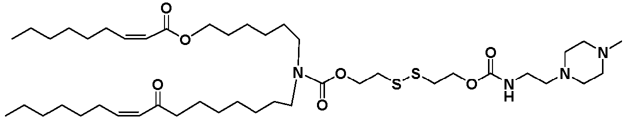

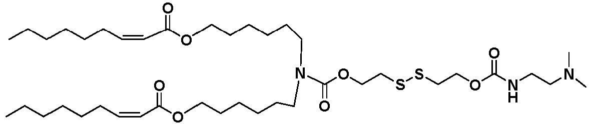

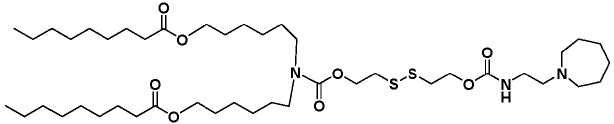

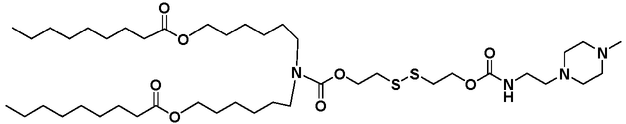

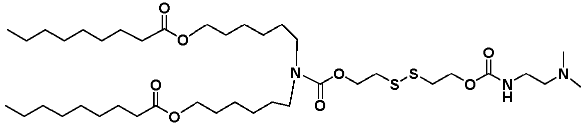

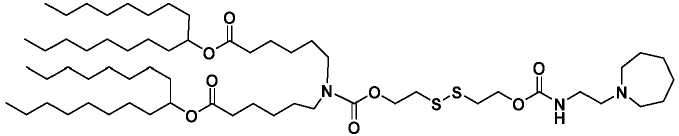

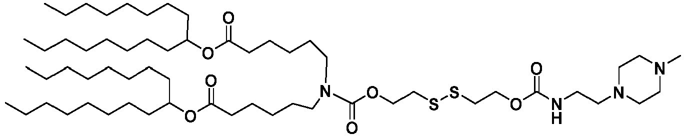

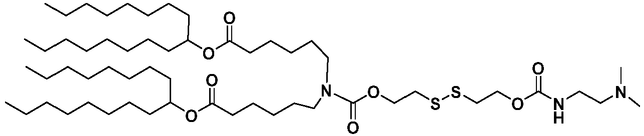

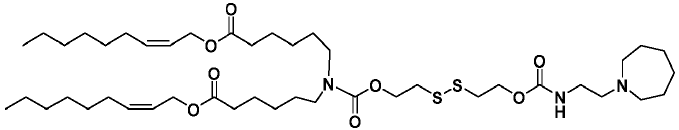

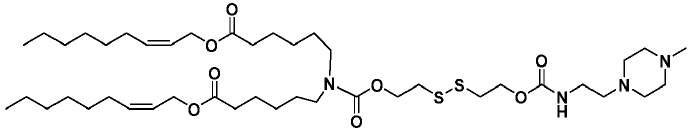

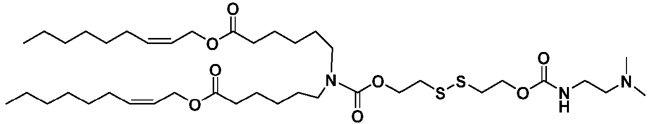

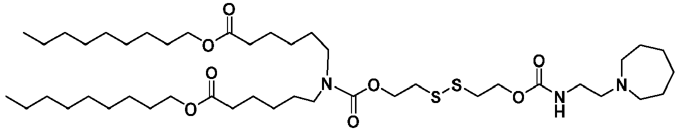

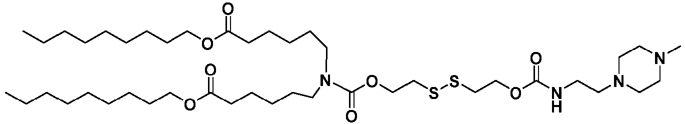

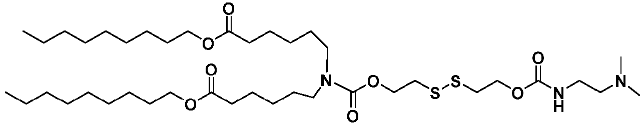

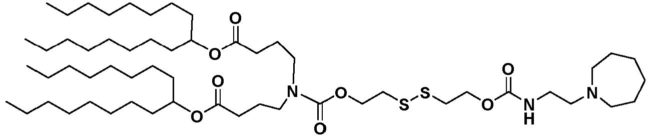

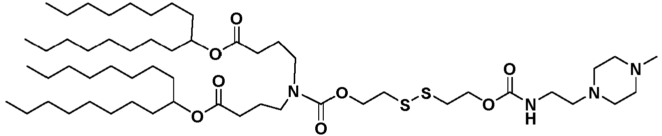

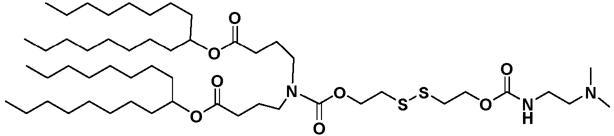

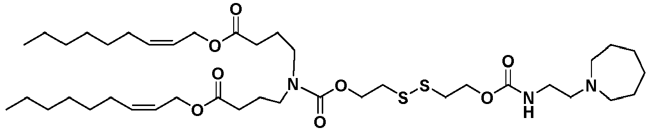

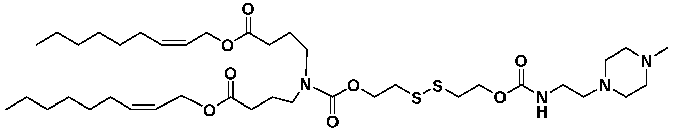

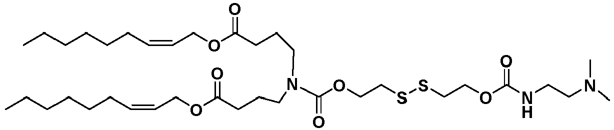

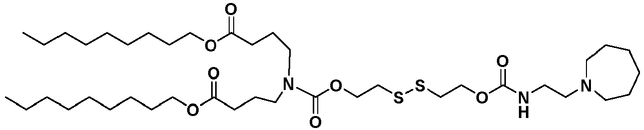

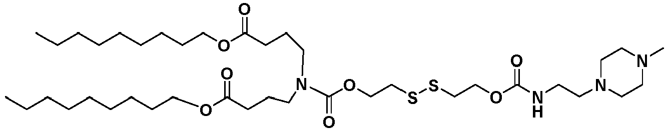

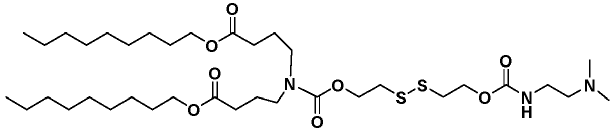

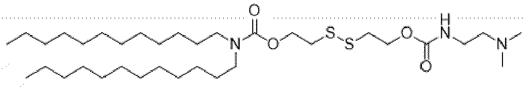

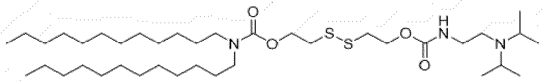

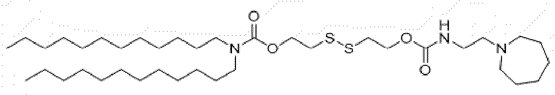

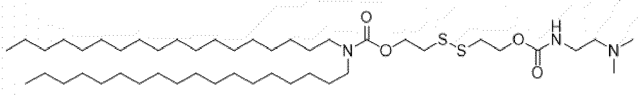

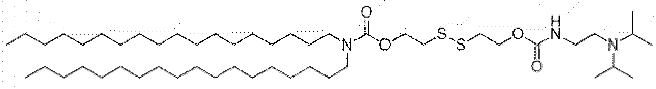

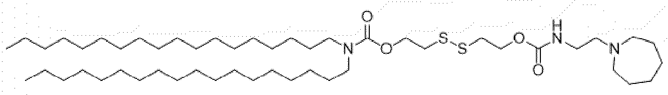

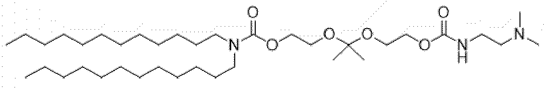

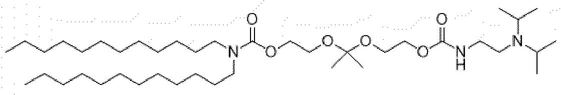

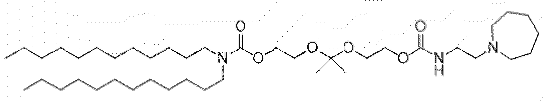

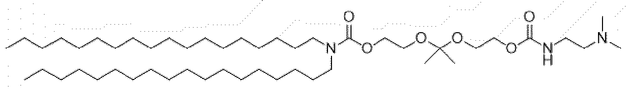

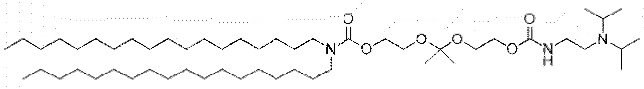

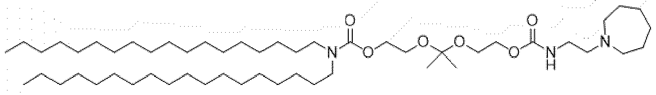

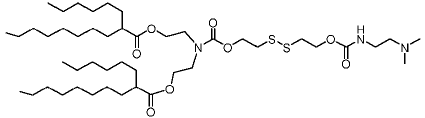

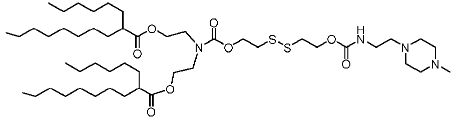

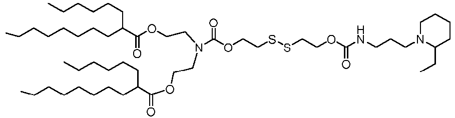

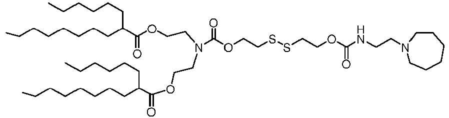

7. An ionizable lipid as defined in anyone of claims 1 to 3 or 5; wherein the total number of C atoms in R₁ and R₂ together is at least 14.

8. An ionizable lipid as defined in anyone of claims 1 to 3 or 5; wherein m and n are the same, being an integer selected from 1, 2, 3, and 4; preferably 2.

9. An ionizable lipid as defined in anyone of claims 1 to 3 or 5; wherein Y is -NH-.

10. A lipid nanoparticle or lipid nanoparticle composition comprising an ionizable lipid as defined in anyone of claims 1 to 9.

11. The lipid nanoparticle or lipid nanoparticle composition according to claim 10, further comprising a phospholipid, a sterol and/or a PEG lipid.

12. The lipid nanoparticle or lipid nanoparticle composition according to anyone of claims 10 to 11, further comprising an active agent, in particular a nucleic acid, preferably mRNA.

13. Use of an ionizable lipid as defined in anyone of claims 1 to 9 in the manufacture of a lipid nanoparticle or lipid nanoparticle composition.

14. A pharmaceutical composition comprising a lipid nanoparticle or lipid nanoparticle composition as defined in anyone of claims 10 to 12, and a pharmaceutically acceptable agent.

15. A pharmaceutical composition as defined in claim 14 for use in medicine.

## Patentansprüche

1. Ionisierbares Lipid, dargestellt durch Formel (I) 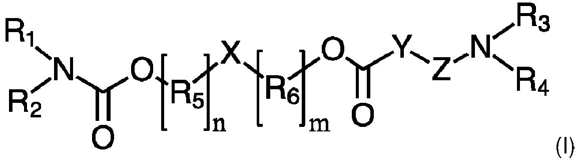 wobei
R₁ und R₂ jeweils unabhängig voneinander ausgewählt sind aus -H, -C₁₋₂₀-Alkyl, -C₂₋₂₀-Alkenyl und -C₂₋₂₀-Alkinyl;
wobei -C₁₋₂₀-Alkyl, -C₂₋₂₀-Alkenyl und -C₂₋₂₀-Alkinyl jeweils gegebenenfalls mit 1 bis 3 -O-(C=O)-R₇, -(C=O)-O-R₇, -C₁₋₂₀-Alkyl, -C₂₋₂₀-Alkenyl und -C₂₋₂₀-Alkinyl substituiert sein kann; und wobei die Gesamtzahl der C-Atome in R₁ und R₂ zusammen mindestens 8 beträgt;
R₃ und R₄ jeweils unabhängig voneinander ein -C₁₋₆-Alkyl sind; oder R₃ und R₄ zusammen mit dem N-Atom, an das sie gebunden sind, einen 5- bis 10-gliedrigen aromatischen oder nicht-aromatischen Heterozyklus bilden; der Heterozyklus ferner gegebenenfalls ein oder mehrere zusätzliche N-Atome umfassen kann und/oder gegebenenfalls mit 1-3 Substituenten substituiert sein kann, ausgewählt aus: -C₁₋₆-Alkyl; und
R₅ und R₆ jeweils unabhängig voneinander -CH₂- ist;
R₇ jeweils unabhängig voneinander ausgewählt ist aus -C₁₋₂₀-Alkyl, -C₂₋₂₀-Alkenyl und -C₂₋₂₀-Alkinyl; wobei -C₁₋₂₀-Alkyl, -C₂₋₂₀-Alkenyl und -C₂₋₂₀-Alkinyl jeweils gegebenenfalls mit 1 bis 3 -O-(C=O)-R₇, -(C=O)-O-R₇, -C₁₋₂₀-Alkyl, -C₂₋₂₀-Alkenyl und -C₂₋₂₀-Alkinyl substituiert sein kann;
m und n jeweils unabhängig voneinander eine ganze Zahl, ausgewählt aus 1, 2, 3 und 4, sind;
X ausgewählt ist aus -O-, -S-, -S-S-, -O-(C=O)-, -O-(C=O)-O-, -(C=N-NH₂)-, -O-CR₈R₉-O- und -S- CR₈R₉-S-,
R₈ und R₉ jeweils unabhängig voneinander ausgewählt ist aus -H, -C₁₋₆-Alkyl und -C₃₋₆-Cycloalkyl;
Y ausgewählt ist aus -NH- und -O-; und Z -C₁₋₆-Alkylen- ist.

2. Ionisierbares Lipid, wie in Anspruch 1 definiert, und dargestellt durch Formel (II) 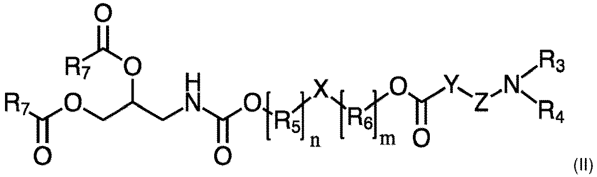 wobei
R₃ und R₄ jeweils unabhängig voneinander ein -C₁₋₆-Alkyl sind; oder R₃ und R₄ zusammen mit dem N-Atom, an das sie gebunden sind, einen 5- bis 10-gliedrigen aromatischen oder nicht-aromatischen Heterozyklus bilden; der Heterozyklus ferner gegebenenfalls ein oder mehrere zusätzliche N-Atome umfassen kann und/oder gegebenenfalls mit 1-3 Substituenten substituiert sein kann, ausgewählt aus: -C₁₋₆-Alkyl;
R₅ und R₆ jeweils unabhängig voneinander -CH₂- ist;
R₇ jeweils unabhängig voneinander ausgewählt ist aus -C₁₋₂₀-Alkyl, -C₂₋₂₀-Alkenyl und -C₂₋₂₀-Alkinyl; wobei -C₁₋₂₀-Alkyl, -C₂₋₂₀-Alkenyl und -C₂₋₂₀-Alkinyl jeweils gegebenenfalls mit 1 bis 3 -O-(C=O)-R₇, -(C=O)-O-R₇, -C₁₋₂₀-Alkyl, -C₂₋₂₀-Alkenyl und -C₂₋₂₀-Alkinyl substituiert sein kann; und die Gesamtzahl der C-Atome in beiden R₇-Resten zusammen mindestens 5 beträgt;
m und n jeweils unabhängig voneinander eine ganze Zahl, ausgewählt aus 1, 2, 3 und 4, sind;
X ausgewählt ist aus -O-, -S-, -S-S-, -O-(C=O)-, -O-(C=O)-O-, -(C=N-NH₂)-, -O-CR₈R₉-O- und -S- CR₈R₉-S-,
R₈ und R₉ jeweils unabhängig voneinander aus -H, -C₁₋₆-Alkyl und -C₃₋₆-Cycloalkyl ausgewählt ist;
Y ausgewählt ist aus -NH- und -O-; und
Z -C₁₋₆-Alkylen- ist.

3. Ionisierbares Lipid, wie in einem der Ansprüche 1 oder 2 definiert und dargestellt durch eine der Formeln (Illa), (Illb) oder (Illc) 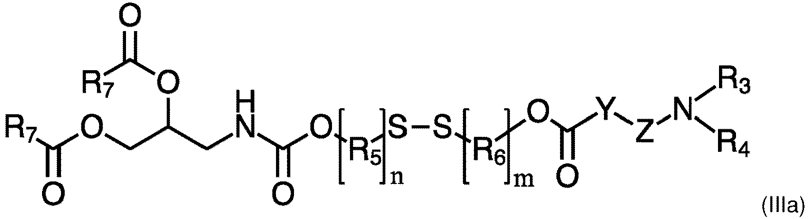 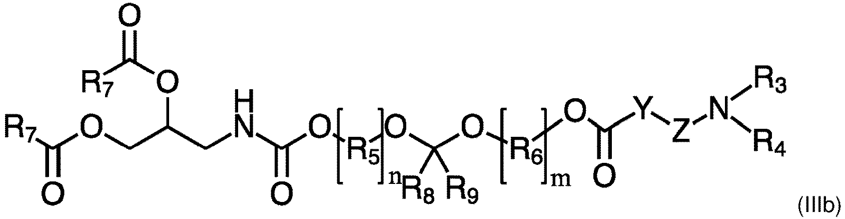 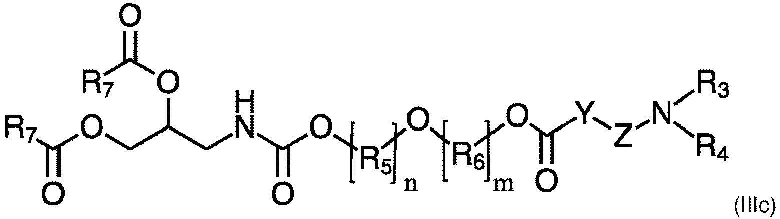 wobei
R₃ und R₄ jeweils unabhängig voneinander ein -C₁₋₆-Alkyl sind; oder R₃ und R₄ zusammen mit dem N-Atom, an das sie gebunden sind, einen 5- bis 10-gliedrigen aromatischen oder nicht-aromatischen Heterozyklus bilden; der Heterozyklus ferner gegebenenfalls ein oder mehrere zusätzliche N-Atome umfassen kann und/oder gegebenenfalls mit 1-3 Substituenten substituiert sein kann, ausgewählt aus: -C₁₋₆-Alkyl; und
R₅ und R₆ jeweils -CH₂- ist;
R₇ jeweils unabhängig voneinander ausgewählt ist aus -C₁₋₂₀-Alkyl, -C₂₋₂₀-Alkenyl und -C₂₋₂₀-Alkinyl; wobei -C₁₋₂₀-Alkyl, -C₂₋₂₀-Alkenyl und -C₂₋₂₀-Alkinyl jeweils gegebenenfalls mit 1 bis 3 -O-(C=O)-R₇, -(C=O)-O-R₇, -C₁₋₂₀-Alkyl, -C₂₋₂₀-Alkenyl und -C₂₋₂₀-Alkinyl substituiert sein kann; und die Gesamtzahl der C-Atome in beiden R₇-Resten zusammen mindestens 5 beträgt;
m und n jeweils unabhängig voneinander eine ganze Zahl, ausgewählt aus 1, 2, 3 und 4, sind;
R₈ und R₉ jeweils unabhängig voneinander aus -H, -C₁₋₆-Alkyl und -C₃₋₆-Cycloalkyl ausgewählt ist;
Y ausgewählt ist aus -NH- und -O-; und
Z -C₁₋₆-Alkylen- ist.

4. Ionisierbares Lipid, wie in Anspruch 1 definiert und dargestellt durch eine der Formeln (IVa), (IVb) und (IVc) 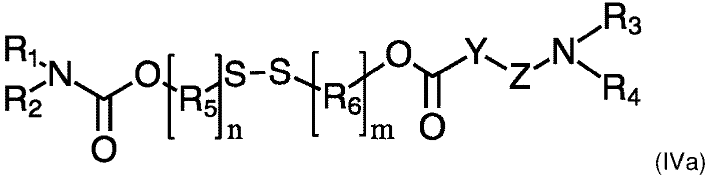 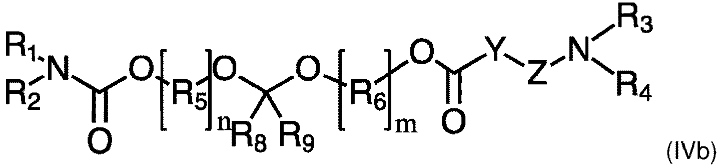 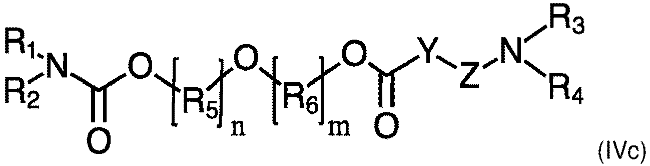 wobei
R₁ und R₂ jeweils unabhängig voneinander ausgewählt sind aus -H, -C₁₋₂₀-Alkyl, -C₂₋₂₀-Alkenyl und -C₂₋₂₀-Alkinyl; wobei -C₁₋₂₀-Alkyl, -C₂₋₂₀-Alkenyl und -C₂₋₂₀-Alkinyl jeweils gegebenenfalls mit 1 bis 3 -O-(C=O)-R₇, -(C=O)-O-R₇, -C₁₋₂₀-Alkyl, - C₂₋₂₀-Alkenyl und -C₂₋₂₀-Alkinyl substituiert sein kann; und wobei die Gesamtzahl der C-Atome in R₁ und R₂ zusammen mindestens 8 beträgt;
R₃ und R₄ jeweils unabhängig voneinander ein -C₁₋₆-Alkyl sind; oder R₃ und R₄ zusammen mit dem N-Atom, an das sie gebunden sind, einen 5- bis 10-gliedrigen aromatischen oder nicht-aromatischen Heterozyklus bilden; der Heterozyklus ferner gegebenenfalls ein oder mehrere zusätzliche N-Atome umfassen kann und/oder gegebenenfalls mit 1-3 Substituenten substituiert sein kann, ausgewählt aus: -C₁₋₆-Alkyl;
R₇ jeweils unabhängig voneinander ausgewählt ist aus -C₁₋₂₀-Alkyl, -C₂₋₂₀-Alkenyl und -C₂₋₂₀-Alkinyl; wobei -C₁₋₂₀-Alkyl, -C₂₋₂₀-Alkenyl und -C₂₋₂₀-Alkinyl jeweils gegebenenfalls mit 1 bis 3 -O-(C=O)-R₇, -(C=O)-O-R₇, -C₁₋₂₀-Alkyl, -C₂₋₂₀-Alkenyl und -C₂₋₂₀-Alkinyl substituiert sein kann;
R₅ und R₆ jeweils unabhängig aus -CH₂-, und-O-CH₂- ausgewählt ist;
m und n jeweils unabhängig voneinander eine ganze Zahl, ausgewählt aus 1, 2, 3 und 4, sind;
R₈ und R₉ jeweils unabhängig voneinander aus -H, -C₁₋₆-Alkyl und -C₃₋₆-Cycloalkyl ausgewählt ist;
Y ausgewählt ist aus -NH- und -O-; und
Z -C₁₋₆-Alkylen- ist.

5. Ionisierbares Lipid, wie in Anspruch 1 definiert und dargestellt durch Formel (V) 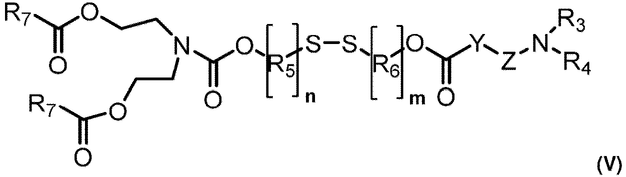 wobei
R₃ und R₄ jeweils unabhängig voneinander ein -C₁₋₆-Alkyl sind; oder R₃ und R₄ zusammen mit dem N-Atom, an das sie gebunden sind, einen 5- bis 10-gliedrigen aromatischen oder nicht-aromatischen Heterozyklus bilden; der Heterozyklus ferner gegebenenfalls ein oder mehrere zusätzliche N-Atome umfassen kann und/oder gegebenenfalls mit 1-3 Substituenten substituiert sein kann, ausgewählt aus: -C₁₋₆-Alkyl; und
R₅ und R₆ jeweils unabhängig aus -CH₂-, und-O-CH₂- ausgewählt ist;
R₇ jeweils unabhängig voneinander ausgewählt ist aus -C₁₋₂₀-Alkyl, -C₂₋₂₀-Alkenyl und -C₂₋₂₀-Alkinyl; wobei -C₁₋₂₀Alkyl, -C₂₋₂₀-Alkenyl und -C₂₋₂₀-Alkinyl jeweils gegebenenfalls mit 1 bis 3 -O-(C=O)-R₇, -C₁₋₂₀Alkyl, -C₂₋₂₀-Alkenyl und -C₂₋₂₀-Alkinyl substituiert sein kann; und die Gesamtzahl der C-Atome in beiden R₇-Resten zusammen mindestens 5 beträgt;
m und n jeweils unabhängig voneinander eine ganze Zahl, ausgewählt aus 1, 2, 3 und 4, sind;
Y ausgewählt ist aus -NH- und -O-; und
Z -C₁₋₆-Alkylen- ist.

6. Ionisierbares Lipid, wie in einem der Ansprüche 1 bis 5 definiert, und ausgewählt aus der Liste, umfassend:

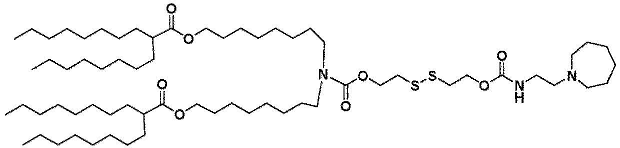

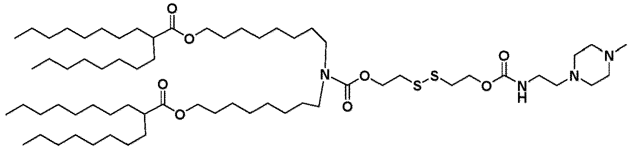

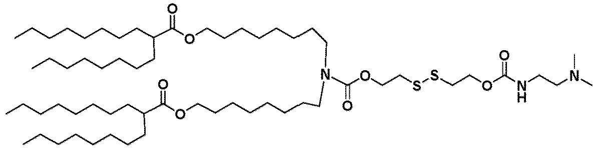

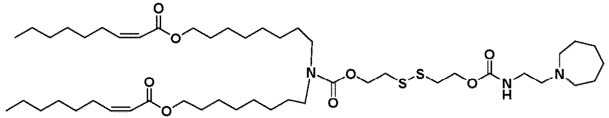

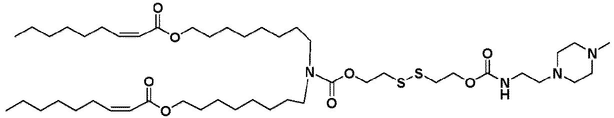

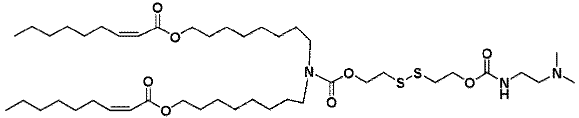

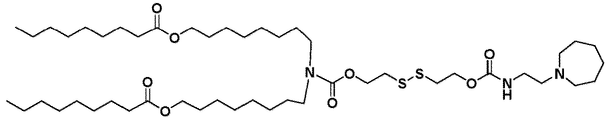

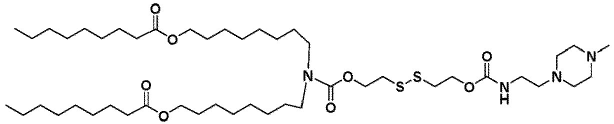

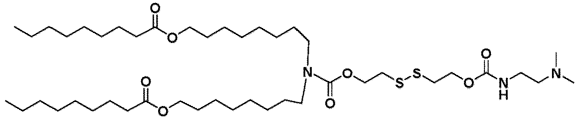

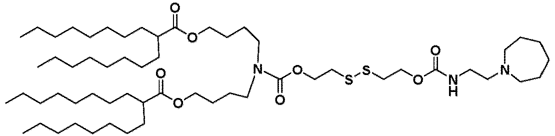

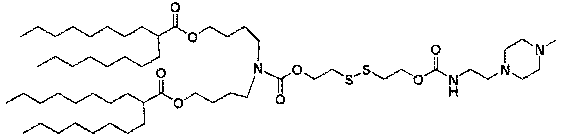

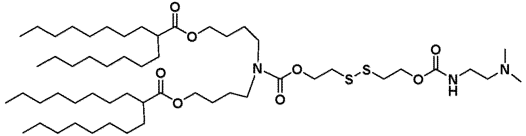

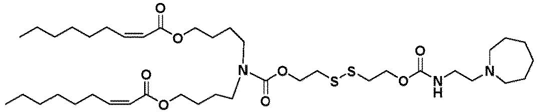

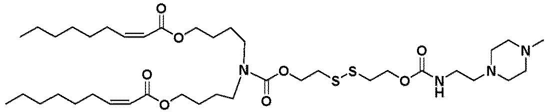

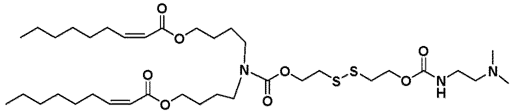

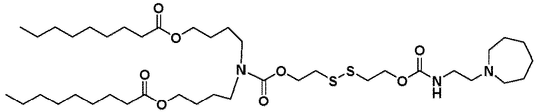

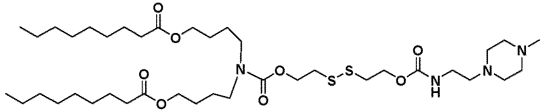

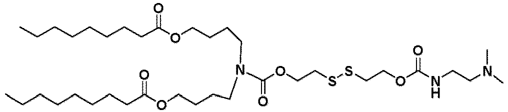

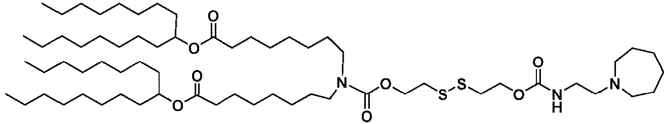

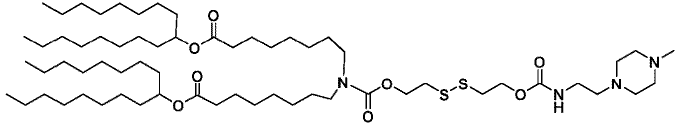

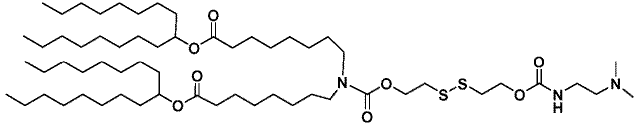

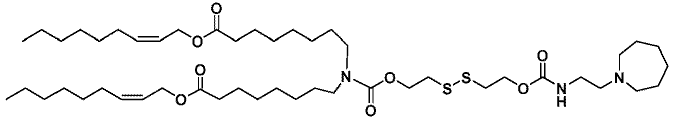

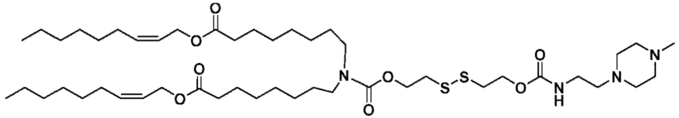

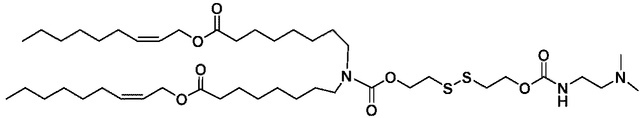

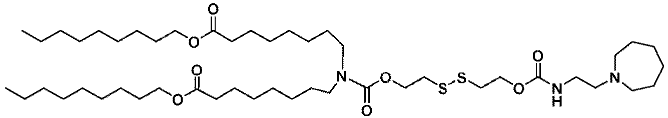

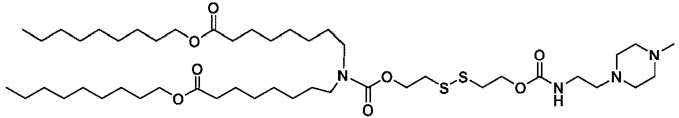

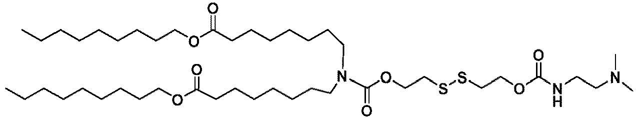

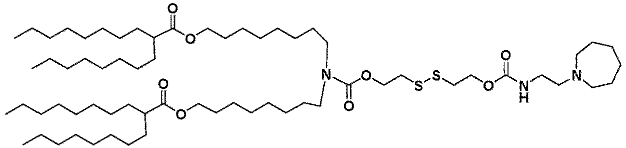

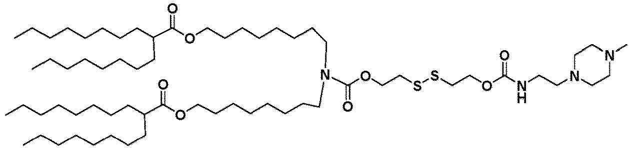

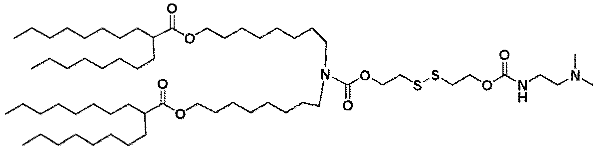

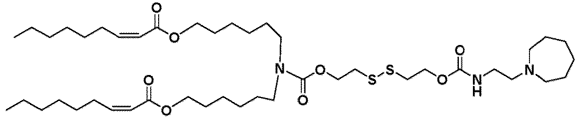

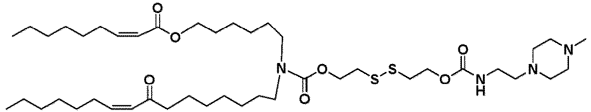

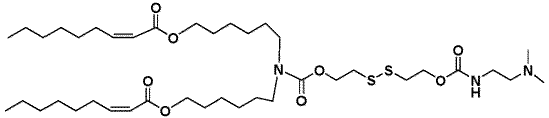

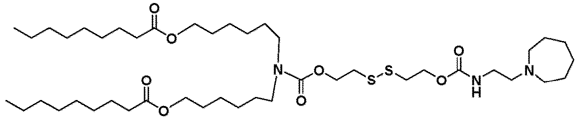

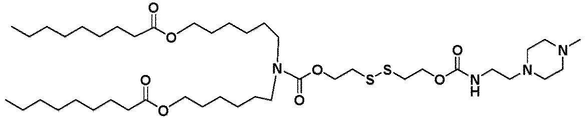

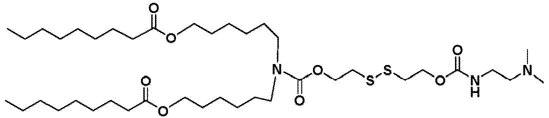

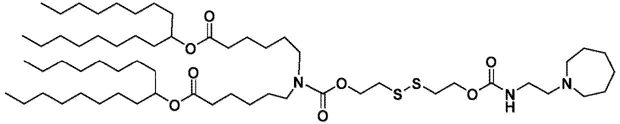

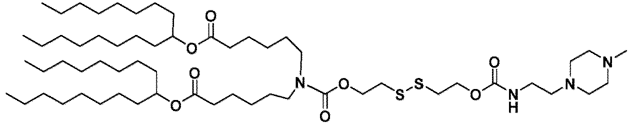

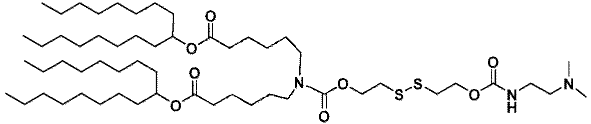

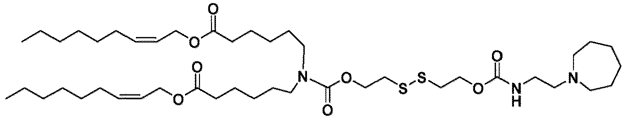

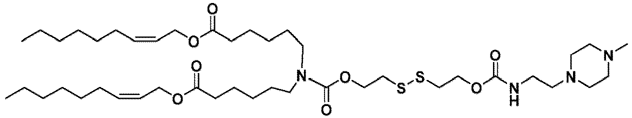

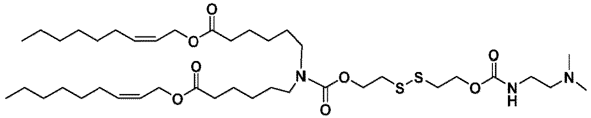

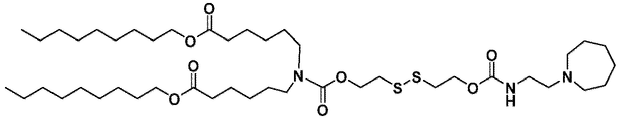

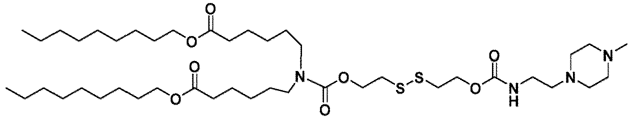

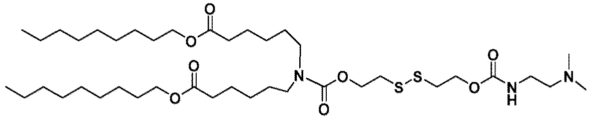

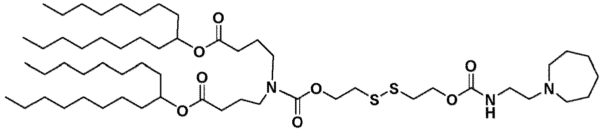

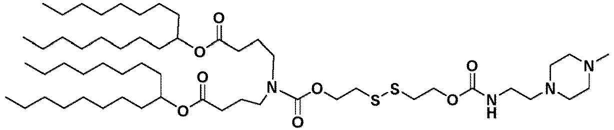

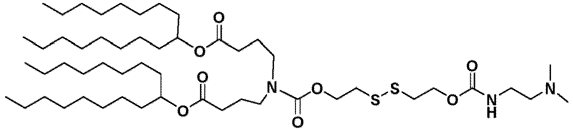

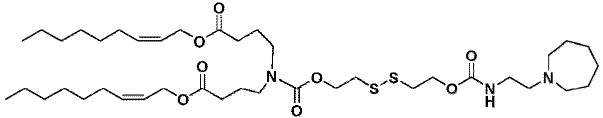

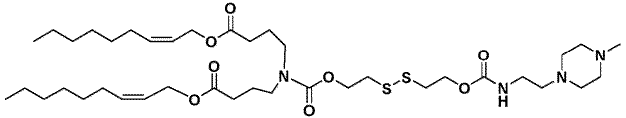

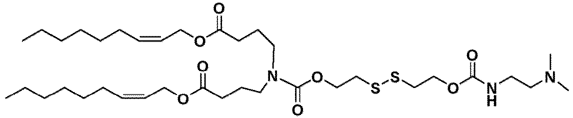

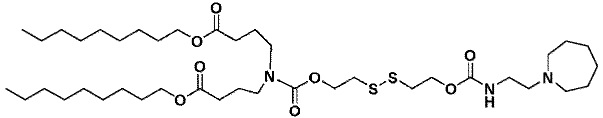

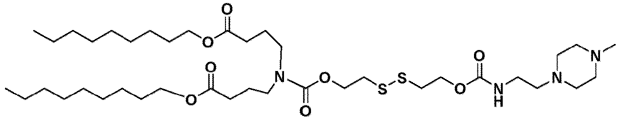

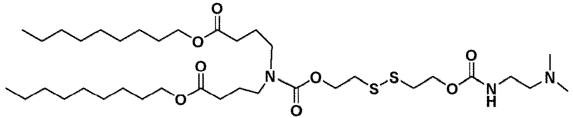

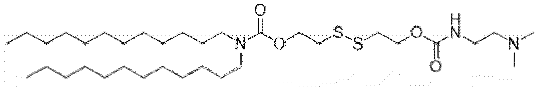

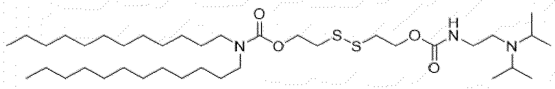

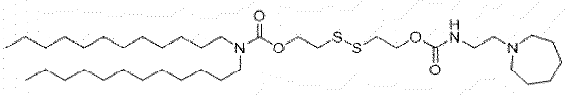

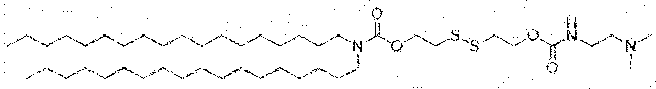

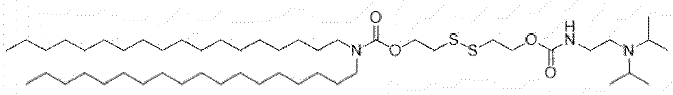

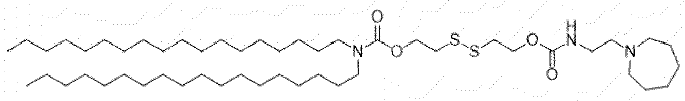

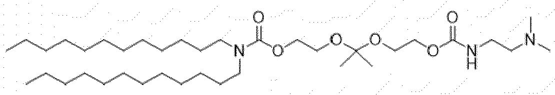

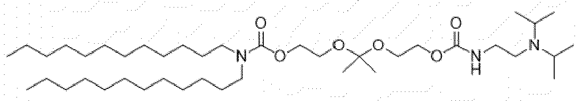

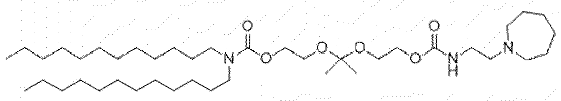

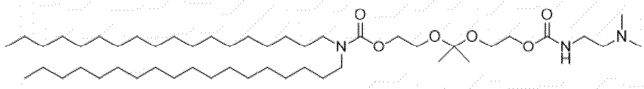

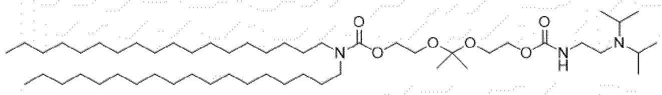

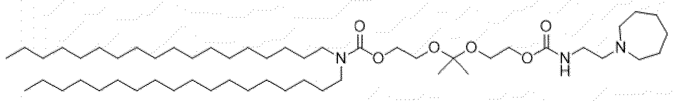

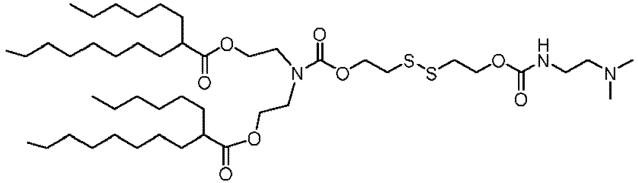

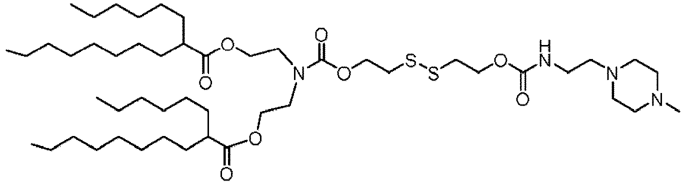

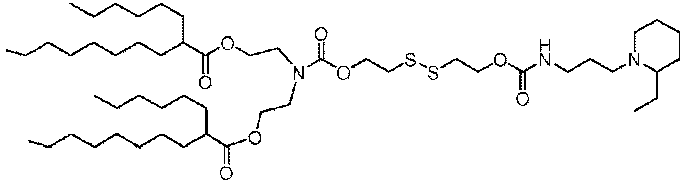

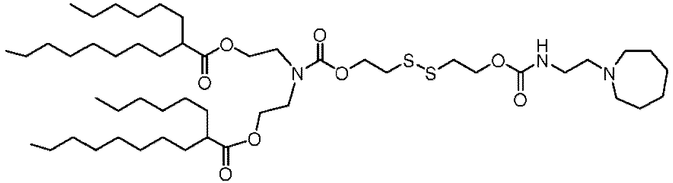

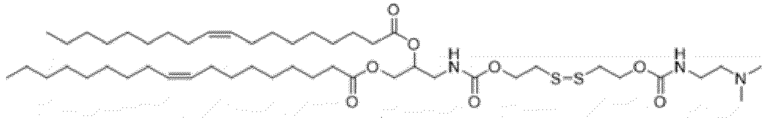

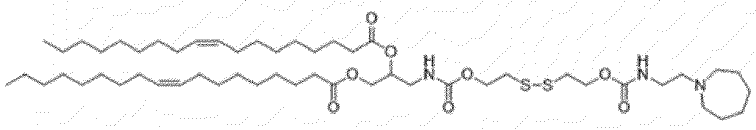

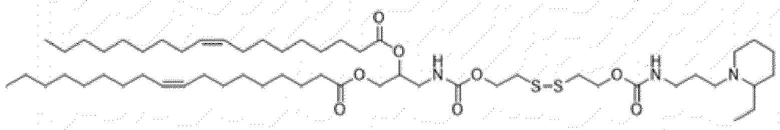

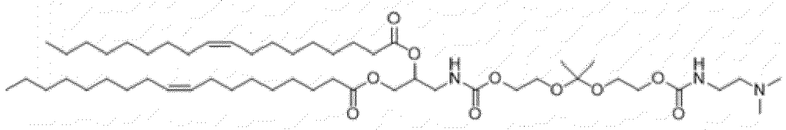

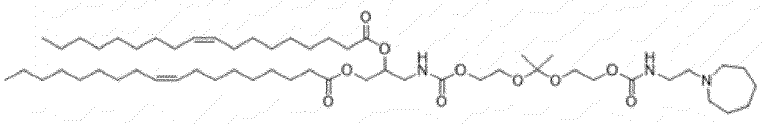

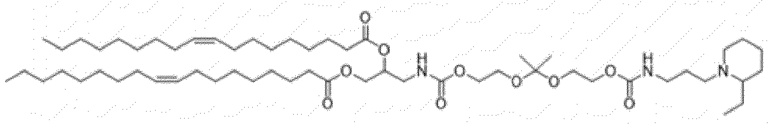

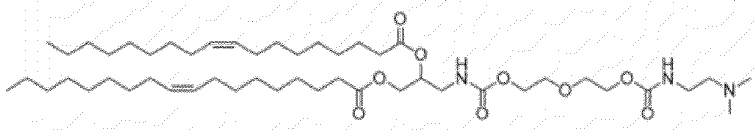

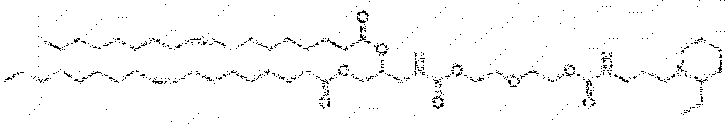

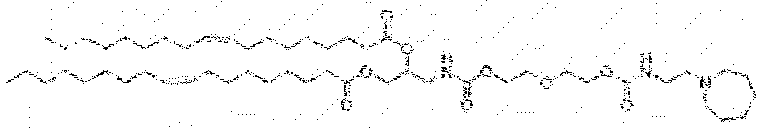

7. Ionisierbares Lipid, wie nach einem der Ansprüche 1 bis 3 oder 5 definiert; wobei die Gesamtzahl der C-Atome in R₁ und R₂ zusammen mindestens 14 beträgt.

8. Ionisierbares Lipid, wie nach einem der Ansprüche 1 bis 3 oder 5 definiert; wobei m und n gleich sind, wobei es sich um eine ganze Zahl, ausgewählt aus 1, 2, 3 und 4, handelt; vorzugsweise 2.

9. Ionisierbares Lipid, wie nach einem der Ansprüche 1 bis 3 oder 5 definiert; wobei Y -NH- ist.

10. Lipid-Nanopartikel oder Lipid-Nanopartikel-Zusammensetzung, umfassend ein ionisierbares Lipid, wie in einem der Ansprüche 1 bis 9 definiert.

11. Lipid-Nanopartikel oder Lipid-Nanopartikel-Zusammensetzung nach Anspruch 10, ferner umfassend ein Phospholipid, ein Sterol und/oder ein PEG-Lipid.

12. Lipidnanopartikel oder Lipid-Nanopartikel-Zusammensetzung nach einem der Ansprüche 10 bis 11, ferner umfassend einen Wirkstoff, insbesondere eine Nukleinsäure, vorzugsweise mRNA.

13. Verwendung eines ionisierbaren Lipids, wie in einem der Ansprüche 1 bis 9 definiert, bei der Herstellung eines Lipid-Nanopartikels oder einer Lipid-Nanopartikel-Zusammensetzung.

14. Pharmazeutische Zusammensetzung, umfassend ein Lipid-Nanopartikel oder eine Lipid-Nanopartikel-Zusammensetzung, wie in einem der Ansprüche 10 bis 12 definiert, und ein pharmazeutisch verträgliches Mittel.

15. Pharmazeutische Zusammensetzung, wie in Anspruch 14 definiert, zur Verwendung in der Medizin.

## Revendications

1. Lipide ionisable représenté par la formule (I) 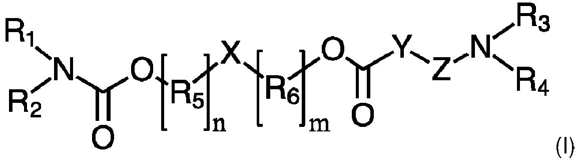 où
R₁ et R₂ sont chacun indépendamment choisis parmi -H, alkyle en -C_{1 à 20}, alcényle en -C_{2 à 20} et -alcynyle en C_{2 à 20} ;
dans lequel chacun desdits alkyle en -C₁ à ₂₀, alcényle en -C_{2 à 20} et alcynyle en -C_{2 à 20} peut éventuellement être substitué par de 1 à 3 -O-(C=O)-R₇, - (C=O)-O-R₇, alkyle en -C₁ à ₂₀, alcényle en -C_{2 à 20} et alcynyle en -C₂ à ₂₀ ; et dans lequel le nombre total d'atomes de C dans R₁ et R₂ ensemble est d'au moins 8 ;
R₃ et R₄ sont chacun indépendamment un alkyle en -C₁ à ₆ ; ou R₃ et R₄ pris conjointement avec l'atome de N auquel ils sont liés forment un hétérocycle aromatique ou non aromatique à 5 à 10 chaînons ; ledit hétérocycle peut en outre éventuellement comprendre un ou plusieurs atomes de N supplémentaires, et/ou peut éventuellement être substitué par 1 à 3 substituants choisis parmi : alkyle en -C_{1 à 6} ; et
chaque R₅ et R₆ est indépendamment -CH₂- ;
chaque R₇ est indépendamment choisi parmi alkyle en -C_{1 à 20}, alcényle en - C_{2 à 20} et alcynyle en -C_{2 à 20} ; dans lequel chacun desdits alkyle en -C₁ à ₂₀, alcényle en -C_{2 à 20} et alcynyle en -C_{2 à 20} peut éventuellement être substitué par de 1 à 3 -O-(C=O)-R₇, -(C=O)-O-R₇, alkyle en -C₁ à ₂₀, alcényle en -C_{2 à 20} et alcynyle en -C_{2 à 20} ;
m et n sont chacun indépendamment un nombre entier choisi parmi 1, 2, 3 et 4 ;
X est choisi parmi -O-, -S-, -S-S-, -O-(C=O)-, -O-(C=O)-O-, -(C=N-NH₂)-, -O-CR₈R₉-O- et -S- CR₈R₉-S- ;
chaque R₈ et R₉ est indépendamment choisi parmi -H, alkyle en -C_{1 à 6} et cycloalkyle en -C_{3 à 6} ;
Y est choisi parmi -NH- et -O- ; et Z est alkylène en -C₁ à ₆-.

2. Lipide ionisable tel que défini dans la revendication 1, et étant représenté par la formule (II) 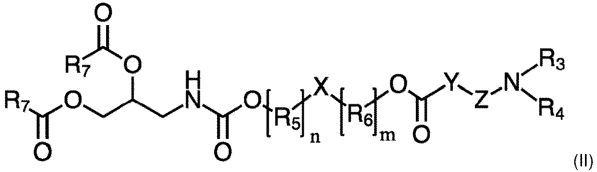 où
R₃ et R₄ sont chacun indépendamment un alkyle en -C₁ à ₆ ; ou R₃ et R₄ pris conjointement avec l'atome de N auquel ils sont liés forment un hétérocycle aromatique ou non aromatique à 5 à 10 chaînons ; ledit hétérocycle peut en outre éventuellement comprendre un ou plusieurs atomes de N supplémentaires, et/ou peut éventuellement être substitué par 1 à 3 substituants choisis parmi : alkyle en -C_{1 à 6} ;
chaque R₅ et R₆ est indépendamment -CH₂- ;
chaque R₇ est indépendamment choisi parmi alkyle en -C_{1 à 20}, alcényle en -C_{2 à 20} et alcynyle en -C_{2 à 20} ; dans lequel chacun desdits alkyle -C₁ à ₂₀, alcényle en -C_{2 à 20} et alcynyle en -C_{2 à 20} peut éventuellement être substitué par de 1 à 3 -O-(C=O)-R₇, -(C=O)-O-R₇, alkyle en -C₁ à ₂₀, alcényle en -C₂ à ₂₀ et alcynyle en -C_{2 à 20} ; et le nombre total d'atomes de C dans les deux fragments R₇ pris ensemble est d'au moins 5 ;
m et n sont chacun indépendamment un nombre entier choisi parmi 1, 2, 3 et 4 ;
X est choisi parmi -O-, -S-, -S-S-, -O-(C=O)-, -O-(C=O)-O-, -(C=N-NH₂)-, -O-CR₈R₉-O- et -S- CR₈R₉-S- ;
chaque R₈ et R₉ est indépendamment choisi parmi -H, alkyle en -C_{1 à 6} et cycloalkyle en -C_{3 à 6} ;
Y est choisi parmi -NH- et -O- ; et
Z est alkylène en -C₁ à ₆-.

3. Lipide ionisable tel que défini dans l'une quelconque des revendications 1 ou 2 et étant représenté par l'une quelconque des formules (IIIa), (IIIb) ou (IIIc) 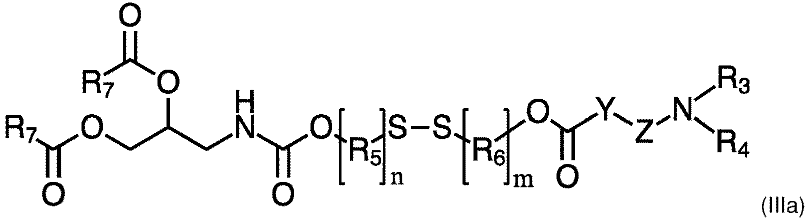 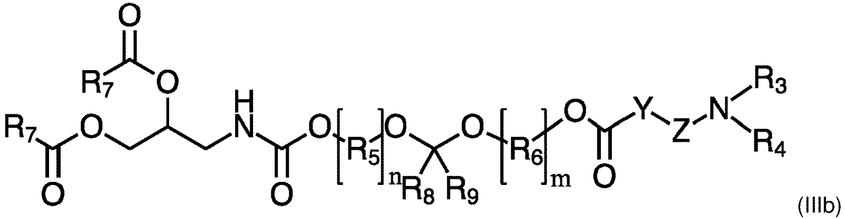 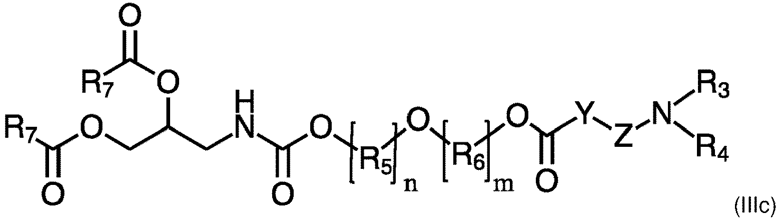 où
R₃ et R₄ sont chacun indépendamment un alkyle en -C_{1 à 6} ; ou R₃ et R₄ pris conjointement avec l'atome de N auquel ils sont liés forment un hétérocycle aromatique ou non aromatique à 5 à 10 chaînons ; ledit hétérocycle peut en outre éventuellement comprendre un ou plusieurs atomes de N supplémentaires, et/ou peut éventuellement être substitué par 1 à 3 substituants choisis parmi : alkyle en -C_{1 à 6} ; et
chaque R₅ et R₆ est -CH₂- ;
chaque R₇ est indépendamment choisi parmi alkyle en -C_{1 à 20}, alcényle en -C_{2 à 20} et alcynyle en -C_{2 à 20} ; dans lequel chacun desdits alkyle en -C₁ à ₂₀, alcényle en -C_{2 à 20} et alcynyle en -C_{2 à 20} peut éventuellement être substitué par de 1 à 3 -O-(C=O)-R₇, -(C=O)-O-R₇, alkyle en -C₁ à ₂₀, alcényle en -C₂ à ₂₀ et alcynyle en -C_{2 à 20} ; et le nombre total d'atomes de C dans les deux fragments R₇ pris ensemble est d'au moins 5 ;
m et n sont chacun indépendamment un nombre entier choisi parmi 1, 2, 3 et 4 ;
chaque R₈ et R₉ est indépendamment choisi parmi -H, alkyle en -C_{1 à 6} et cycloalkyle en -C_{3 à 6} ;
Y est choisi parmi -NH- et -O- ; et
Z est alkylène en -C_{1 à 6}-.

4. Lipide ionisable tel que défini dans la revendication 1 et étant représenté par l'une quelconque des formules (IVa), (IVb) et (IVc) 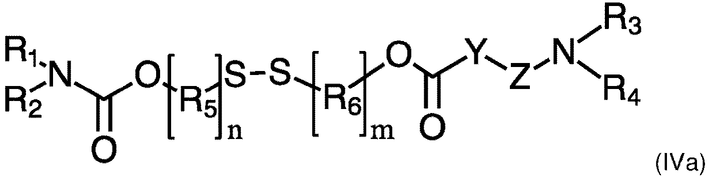 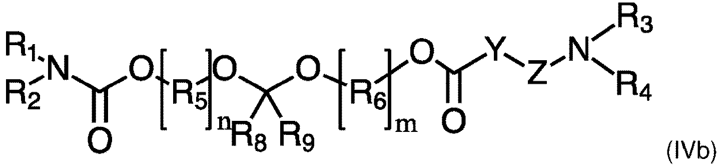 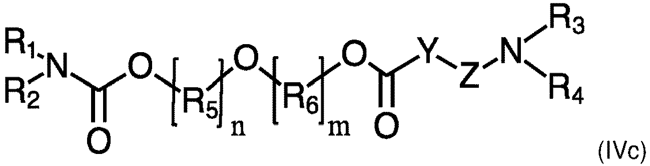 dans laquelle
R₁ et R₂ sont chacun indépendamment choisis parmi -H, alkyle en -C₁ à ₂₀, alcényle en -C_{2 à 20}, et alcynyle en - C_{2 à 20} ; dans lequel chacun desdits alkyle en -C_{1 à 20}, alcényle en -C_{2 à 20} et alcynyle en -C₂ à ₂₀ peut éventuellement être substitué par de 1 à 3 -O-(C=O)-R₇, -(C=O)-O-R₇, alkyle en -C₁ à ₂₀, alcényle en - C₂ à ₂₀ et alcynyle en -C₂ à ₂₀ ; et dans lequel le nombre total d'atomes de C dans R₁ et R₂ ensemble est d'au moins 8 ;
R₃ et R₄ sont chacun indépendamment un alkyle en -C₁ à ₆ ; ou R₃ et R₄ pris conjointement avec l'atome de N auquel ils sont liés forment un hétérocycle aromatique ou non aromatique à 5 à 10 chaînons ; ledit hétérocycle peut en outre éventuellement comprendre un ou plusieurs atomes de N supplémentaires, et/ou peut éventuellement être substitué par 1 à 3 substituants choisis parmi : alkyle en -C_{1 à 6} ;
chaque R₇ est indépendamment choisi parmi alkyle en -C_{1 à 20}, alcényle en - C_{2 à 20} et alcynyle en -C_{2 à 20} ; dans lequel chacun desdits alkyle en -C₁ à ₂₀, alcényle en -C_{2 à 20} et alcynyle en -C_{2 à 20} peut éventuellement être substitué par de 1 à 3 -O-(C=O)-R₇, -(C=O)-O-R₇, alkyle en -C₁ à ₂₀, alcényle en -C_{2 à 20} et alcynyle en -C_{2 à 20} ;
R₅ et R₆ sont chacun indépendamment choisis parmi -CH₂-, et -O-CH₂- ;
m et n sont chacun indépendamment un nombre entier choisi parmi 1, 2, 3 et 4 ;
R₈ et R₉ sont chacun indépendamment choisis parmi -H, alkyle en -C₁ à ₆, et cycloalkyle en -C_{3 à 6} ;
Y est choisi parmi -NH- et -O- ; et
Z est alkylène en -C₁ à ₆-.

5. Lipide ionisable tel que défini dans la revendication 1 et étant représenté par la formule (V) 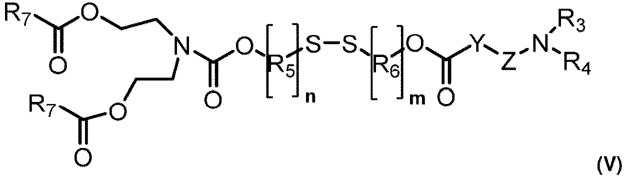 où
R₃ et R₄ sont chacun indépendamment un alkyle en -C_{1 à 6} ; ou R₃ et R₄ pris conjointement avec l'atome de N auquel ils sont liés forment un hétérocycle aromatique ou non aromatique à 5 à 10 chaînons ; ledit hétérocycle peut en outre éventuellement comprendre un ou plusieurs atomes de N supplémentaires, et/ou peut éventuellement être substitué par 1 à 3 substituants choisis parmi : alkyle en -C_{1 à 6} ; et
R₅ et R₆ sont chacun indépendamment choisis parmi -CH₂-, et -O-CH₂- ;
chaque R₇ est indépendamment choisi parmi alkyle en -C_{1 à 20}, alcényle en - C_{2 à 20} et alcynyle en -C_{2 à 20} ; dans lequel chacun desdits alkyle en -C₁ à ₂₀, alcényle en -C_{2 à 20} et alcynyle en -C_{2 à 20} peut éventuellement être substitué par de 1 à 3 -O-(C=O)-R₇, alkyle en -C_{1 à 20}, alcényle en -C_{2 à 20} et alcynyle en -C_{2 à 20} ; et le nombre total d'atomes de C dans les deux fragments R₇ pris ensemble est d'au moins 5 ;
m et n sont chacun indépendamment un nombre entier choisi parmi 1, 2, 3 et 4 ;
Y est choisi parmi -NH- et -O- ; et
Z est alkylène en -C_{1 à 6}-.

6. Lipide ionisable tel que défini dans l'une quelconque des revendications 1 à 5, et étant choisi dans la liste comprenant :

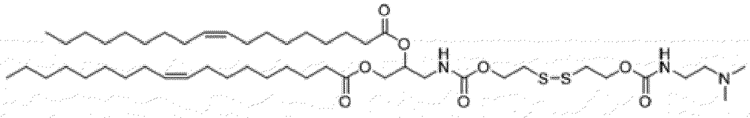

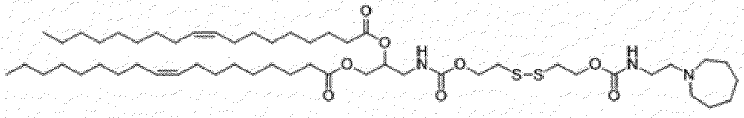

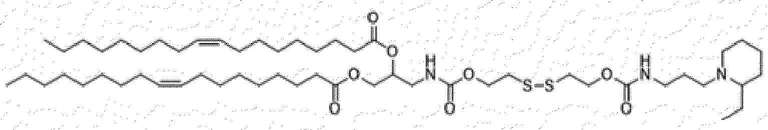

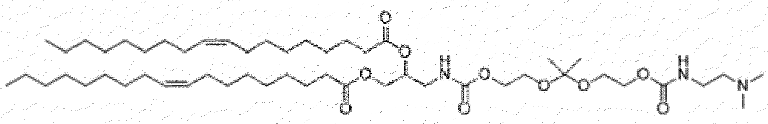

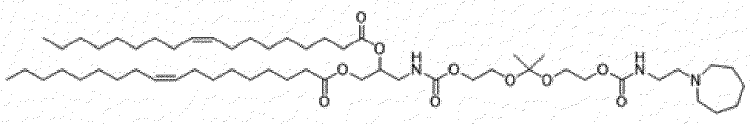

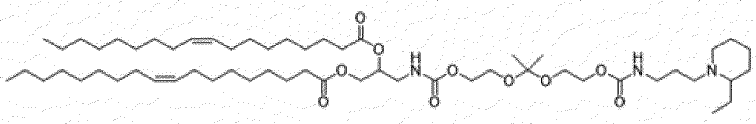

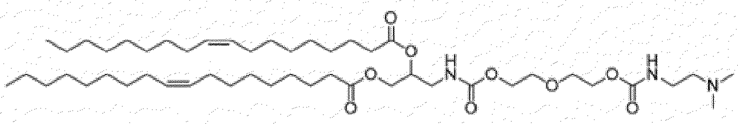

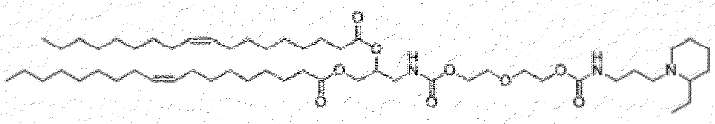

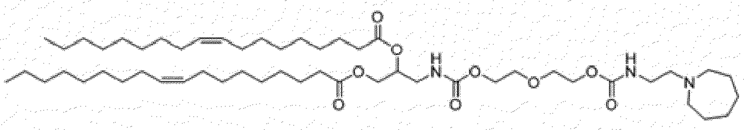

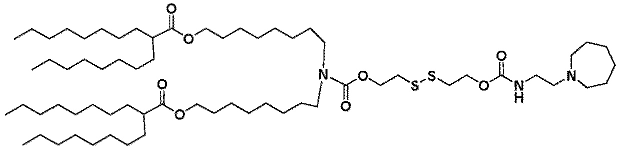

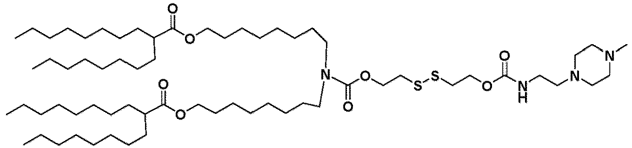

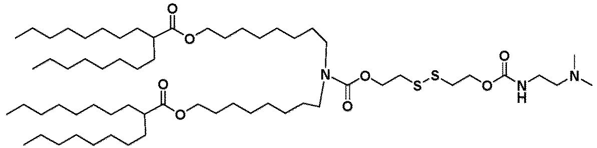

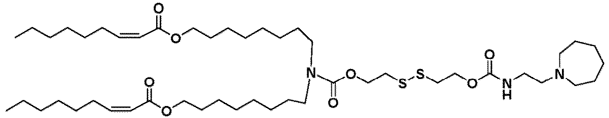

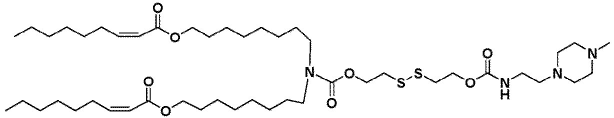

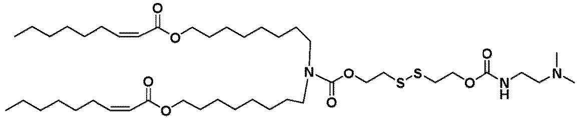

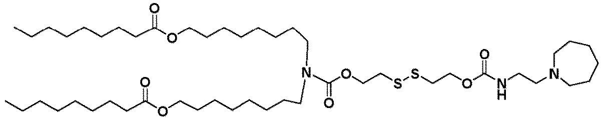

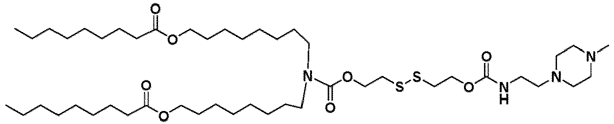

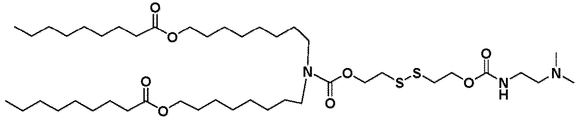

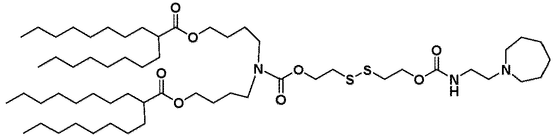

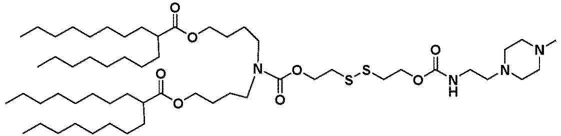

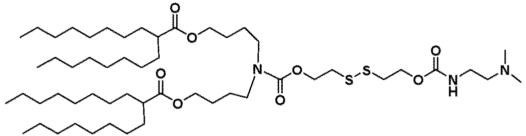

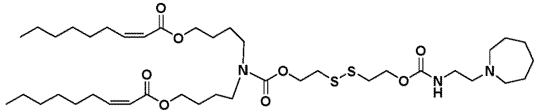

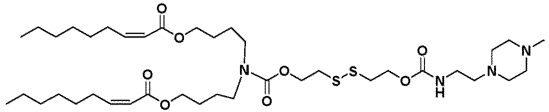

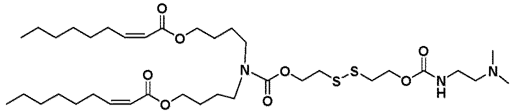

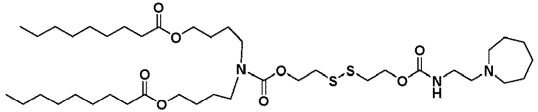

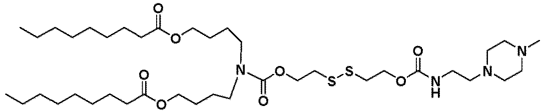

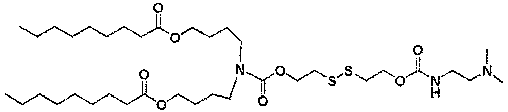

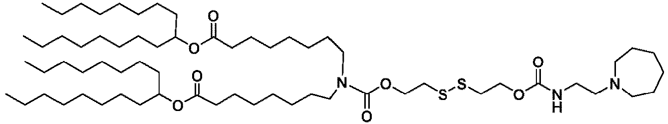

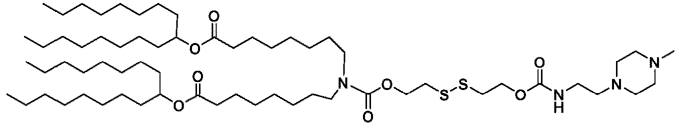

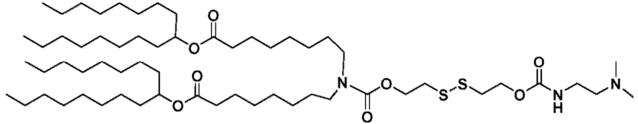

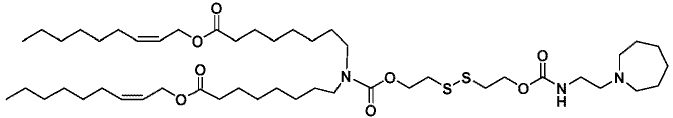

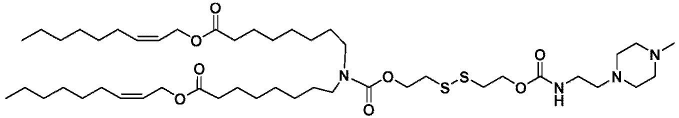

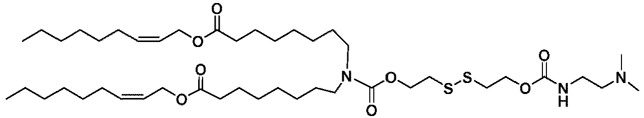

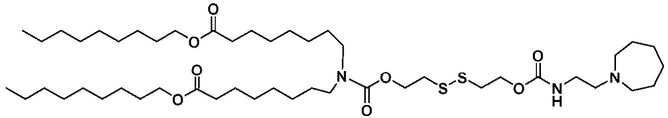

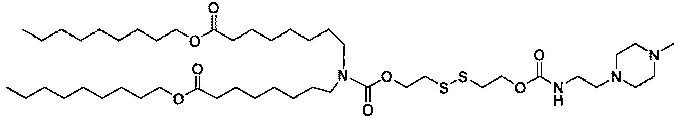

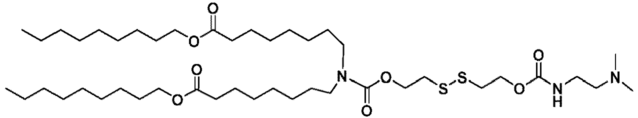

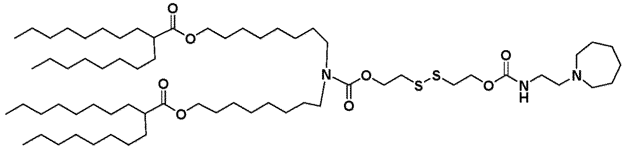

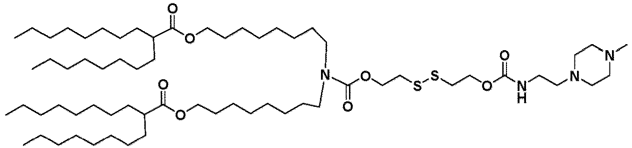

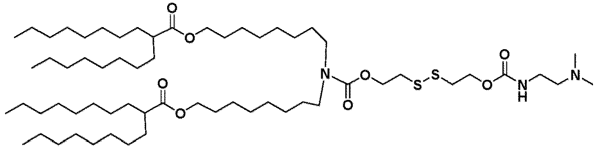

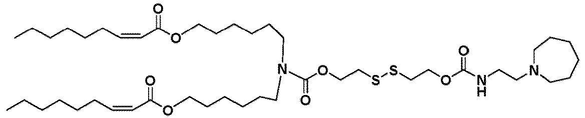

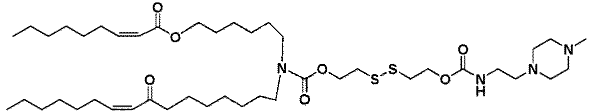

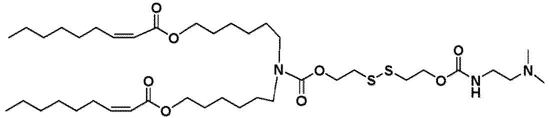

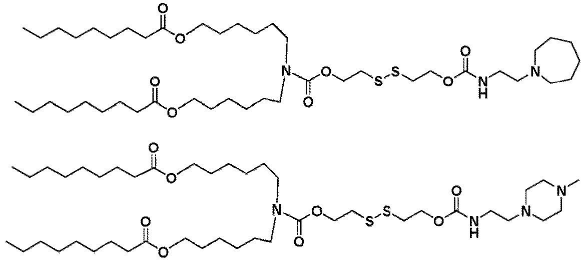

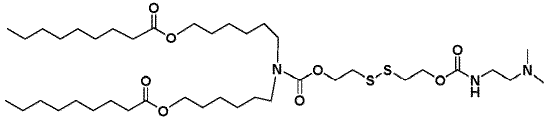

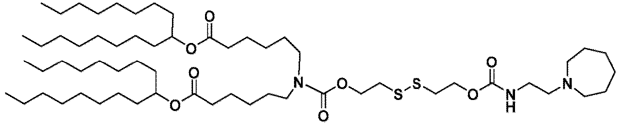

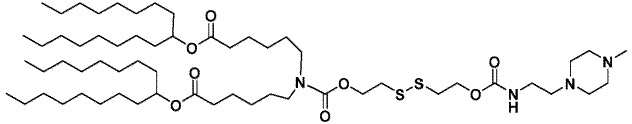

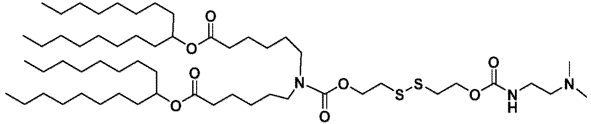

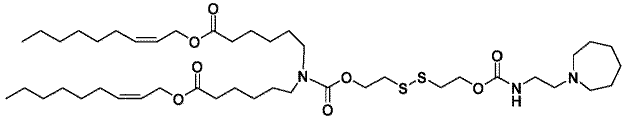

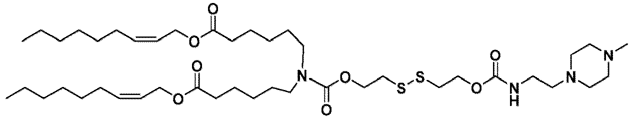

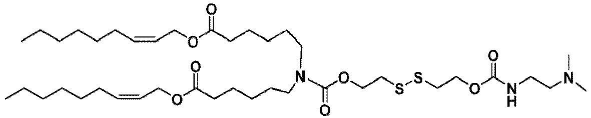

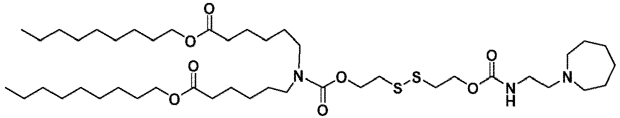

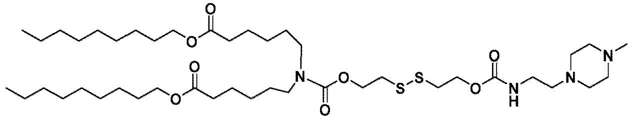

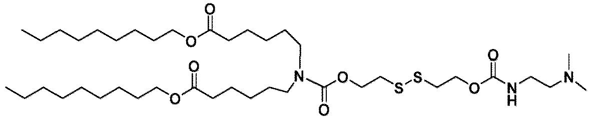

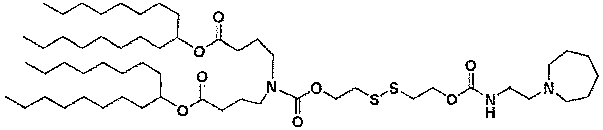

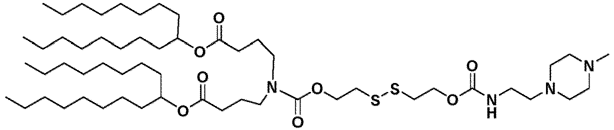

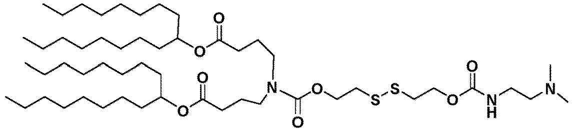

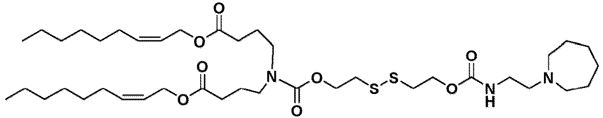

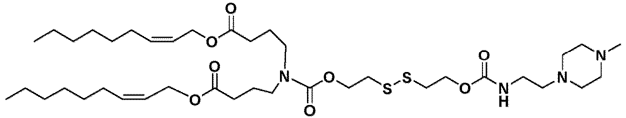

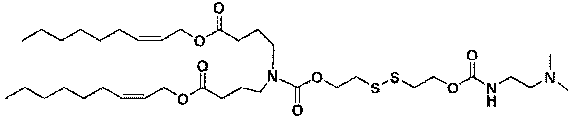

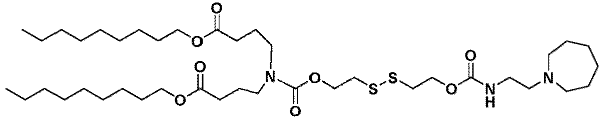

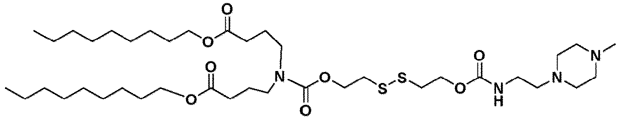

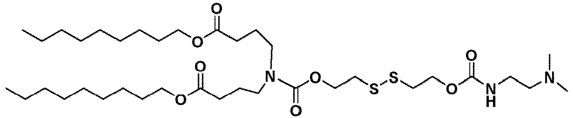

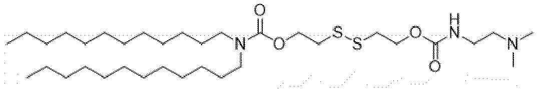

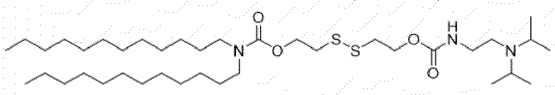

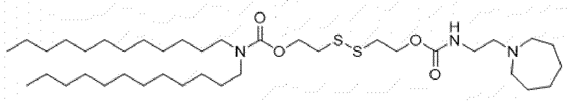

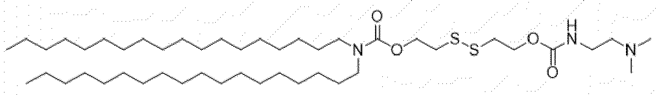

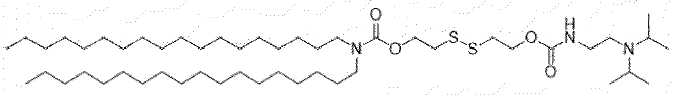

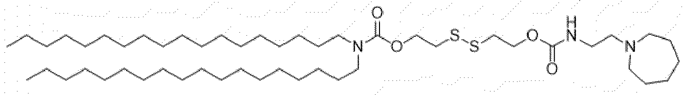

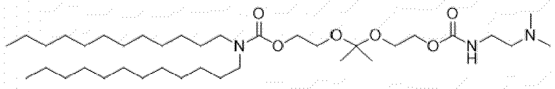

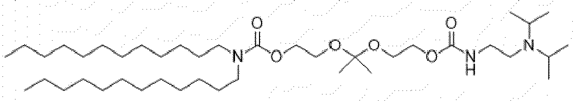

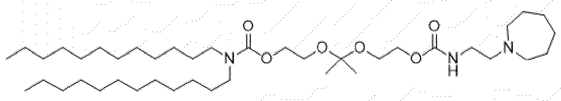

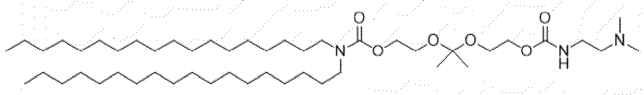

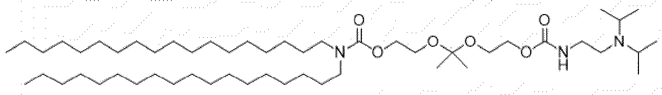

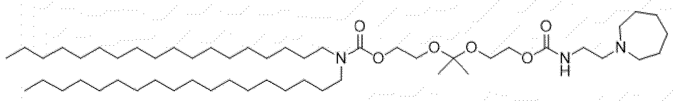

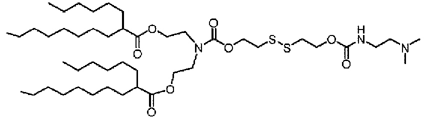

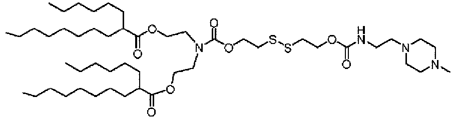

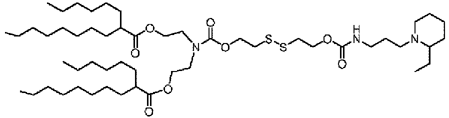

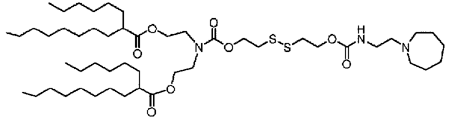

7. Lipide ionisable tel que défini dans l'une quelconque des revendications 1 à 3 ou 5 ; dans lequel le nombre total d'atomes de C dans R₁ et R₂ ensemble est d'au moins 14.

8. Lipide ionisable tel que défini dans l'une quelconque des revendications 1 à 3 ou 5 ; dans lequel m et n sont identiques, étant un entier choisi parmi 1, 2, 3 et 4 ; de préférence 2.

9. Lipide ionisable tel que défini dans l'une quelconque des revendications 1 à 3 ou 5 ; dans lequel Y est -NH-.

10. Nanoparticule lipidique ou composition de nanoparticules lipidiques comprenant un lipide ionisable tel que défini dans l'une quelconque des revendications 1 à 9.

11. Nanoparticule lipidique ou composition de nanoparticules lipidiques selon la revendication 10, comprenant en outre un phospholipide, un stérol et/ou un lipide PEG.

12. Nanoparticule lipidique ou composition de nanoparticules lipidiques selon l'une quelconque des revendications 10 à 11, comprenant en outre un agent actif, en particulier un acide nucléique, de préférence un ARNm.

13. Utilisation d'un lipide ionisable tel que défini dans l'une quelconque des revendications 1 à 9 dans la fabrication d'une nanoparticule lipidique ou d'une composition de nanoparticules lipidiques.

14. Composition pharmaceutique comprenant une nanoparticule lipidique ou une composition de nanoparticules lipidiques telles que définies dans l'une quelconque des revendications 10 à 12, et un agent pharmaceutiquement acceptable.

15. Composition pharmaceutique telle que définie dans la revendication 14 pour une utilisation en médecine.
